# EUROPEAN PATENT SPECIFICATION

(11) **EP 0 871 495 B1**
(45) Date of publication and mention of the grant of the patent: **15.06.2005**
(21) Application number: 96904498.1
(22) Date of filing: 25.01.1996
(51) Int. Cl.: C12N 15/12, C12N 5/10, C12N 5/00, C07K 14/705, A61K 48/00

(54) **REDIRECTION OF CELLULAR IMMUNITY BY RECEPTOR CHIMERAS**
NEUORIENTIERUNG DER ZELLULÄREN IMMUNITÄT DURCH REZEPTORCHIMÄREN
REORIENTATION DE L'IMMUNITE CELLULAIRE AU MOYEN DE RECEPTEURS CHIMERIQUES

(30) Priority: 24.02.1995 US 394176
(43) Date of publication of application: 21.10.1998
(73) Proprietor: THE GENERAL HOSPITAL CORPORATION, Boston, MA 02114 (US)
(72) Inventor: SEED, Brian, Boston, MA 02114 (US); ROMEO, Charles, Belmont, MA 02178 (US); KOLANUS, Waldemar, D-81245 München (DE)
(74) Representative: Jacobi, Markus Alexander
(86) International application number: PCT/US1996/001056
(87) International publication number: WO 1996/025953

(56) References cited:
- WO-A-92/15322
- WO-A-96/26265
- US-A- 5 359 046
- US-A- 5 504 000
- ROMEO C ET AL: "ACTIVATION OF IMMUNE SYSTEM EFFECTOR FUNCTION BY T-CELL OR FC RECEPTOR INTRACELLULAR DOMAINS" COLD SPRING HARBOR SYMPOSIA ON QUANTITATIVE BIOLOGY,US,NEW YORK, NY, vol. 57, 1992, pages 117-125, XP000647674
- PROC. NATL. ACAD. SCI. U.S.A., Volume 90, issued April 1993, R. MAGGIO et al., "Co-Expression Studies With Mutant Muscarinic/Adrenergic Receptors Provide Evidence for Intermolecular 'Cross-Talk' Between G-Protein-Linked Receptors", pages 3103-3107.

## Description

### Field of the Invention

The invention concerns functional T cell receptor, Fc receptor, or B cell receptor chimeras which are capable of redirection immune system function. More particularly, it concerns the regulation of lymphocytes, macrophages, natural killer cells or granulocytes by the expression in said cells of chimeras which cause the cells to respond to targets recognized by the chimeras. The invention also concerns functional T cell receptor, Fc receptor, or b cell receptor chimeras which are capable of directing therapeutic cells to specifically recognize and destroy either cells infected with a specific infective agent, the infective agent itself, a tumor cell, or an autoimmune-generated cell. More particularly, the invention relates to the production of T cell receptor, Fc receptor, or B cell receptor chimeras capable of directing cytotoxic T lymphocytes to specifically recognize and lyse cells expressing HIV envelope proteins. The invention therefore provides a method for manufacturing a medicament for the therapy for diseases such a AIDS (Acquired Immunodeficiency Syndrome) which are caused by the HIV virus.

### Background of the Invention

T cell recognition of antigen through the T cell receptor is the basis of a range of immunological phenomena. The T cells direct what is called cell-mediated immunity. This involves the destruction by cells of the immune system of foreign tissues or infected cells. A variety of T cells exist, including "helper" and "suppressor" cells, which modulate the immune response, and cytotoxic (or "killer") cells, which can kill abnormal cells directly.

A T cell that recognizes and binds a unique antigen displayed on the surface of another cell becomes activated; it can then multiply, and, if it is a cytotoxic cell, it can kill the bound cell.

Autoimmune disease is characterized by production of either antibodies that react with host tissue or immune effector T cells that are autoreactive. In some instances, autoantibodies may arise by a normal T- and B-cell response activated by foreign substances or organisms that contain antigens that cross react with similar compounds in body tissues. Examples of clinically relevant autoantibodies are antibodies against acetylcholine receptors in myasthenia gravis; and anti-DNA, anti-erythrocyte, and anti-platelet antibodies in systemic lupus erythematosus.

### HIV and Immunopathogenesis

In 1984 HIV was shown to be the etiologic agent of AIDS. Since that time the definition of AIDS has been revised a number of times with regard to what criteria should be included in the diagnosis. However, despite the fluctuation in diagnostic parameters, the simple common denominator of AIDS is the infection with HIV and subsequent development of persistent constitutional symptoms and AIDS-defining diseases such as a secondary infections, neoplasms, and neurologic disease. Harrison's Principles of Internal Medicine, 12th ed., McGraw Hill (1991).

HIV is a human retrovirus of the lentivirus group. The four recognized human retroviruses belong to two distinct groups: the human T lymphotropic (or leukemia) retroviruses, HTLV-1 and HTLV-2, and the human immunodeficiency viruses, HIV-1 and HIV-2. The former are transforming viruses whereas the latter are cytopathic viruses.

HIV-1 has been identified as the most common cause of AIDS throughout the world. Sequence homology between HIV-2 and HIV-1 is about 40% with HIV-2 being more closely related to some members of a group of simian immunodeficiency viruses (SIV). See Curran et al., Science 329:1357-1359 (1985); Weiss et al., Nature 324:572-575 (1986).

HIV has the usual retroviral genes (env, gag, and pol) as well as six extra genes involved in the replication and other biologic activities of the virus. As stated previously, the common denominator of AIDS is a profound immunosuppression, predominantly of cell-mediated immunity. This immune suppression leads to a variety of opportunistic diseases, particularly certain infections and neoplasms.

The main cause of the immune defect in AIDS has been identified as a quantitative and qualitative deficiency in the subset of thymus-derived (T) lymphocytes, the T4 population. This subset of cells is defined phenotypically by the presence of the CD4 surface molecule, which has been demonstrated to be the cellular receptor for HIV. Dalgleish et al., Nature 312:763 (1984). Although the T4 cell is the major cell type infected with HIV, essentially any human cell that expresses the CD4 molecule on its surface is capable of binding to and being infected with HIV.

Traditionally, CD4⁺ T cells have been assigned the role of helper/inducer, indicating their function in providing an activating signal to B cells, or inducing T lymphocytes bearing the reciprocal CD8 marker to become cytotoxic/suppressor cells. Reinherz and Schlossman, Cell 19:821-827 (1980); Goldstein et al., Immunol. Rev. 68:5-42 (1982).

HIV binds specifically and with high affinity, via a stretch of amino acids in the viral envelope (gp120), to a portion of the V1 region of the CD4 molecule located near its N-terminus. Following binding, the virus fuses with the target cell membrane and is internalized. Once internalized it uses the enzyme reverse transcriptase to transcribe its genomic RNA to DNA, which is integrated into the cellular DNA where it exists for the life of the cell as a "provirus."

The provirus may remain latent or be activated to transcribe mRNA and genomic RNA, leading to protein synthesis, assembly, new virion formation, and budding of virus from the cell surface. Although the precise mechanism by which the virus induces cell death has not been established, it is believed that the major mechanism is massive viral budding from the cell surface, leading to disruption of the plasma membrane and resulting osmotic disequilibrium.

During the course of the infection, the host organism develops antibodies against viral proteins, including the major envelope glycoproteins gp120 and gp41. Despite this humoral immunity, the disease progresses, resulting in a lethal immunosuppression characterized by multiple opportunistic infections, parasitemia, dementia, and death. The failure of the host anti-viral antibodies to arrest the progression of the disease represents one of the most vexing and alarming aspects of the infection, and augurs poorly for vaccination efforts based upon conventional approaches.

Two factors may play a role in the efficacy of the humoral response to immunodeficiency viruses. First, like other RNA viruses (and like retroviruses in particular), the immunodeficiency viruses show a high mutation rate in response to host immune surveillance. Second, the envelope glycoproteins themselves are heavily glycosylated molecules presenting few epitopes suitable for high affinity antibody binding. The poorly antigenic target which the viral envelope presents allows the host little opportunity for restricting viral infection by specific antibody production.

Cells infected by the HIV virus express the gp120 glycoprotein on their surface. Gp120 mediates fusion events among CD4⁺ cells via a reaction similar to that by which the virus enters the uninfected cells, leading to the formation of short-lived multinucleated giant cells. Syncytium formation is dependent on a direct interaction of the gp120 envelope glycoprotein with the CD4 protein. Dalgleish et al., supra; Klatzman et al., Nature 312:763 (1984); McDougal et al., Science 231:382 (1986); Sodroski et al., Nature 322:470 (1986); Lifson et al., Nature 323:725 (1986); Sodroski et al., Nature 321:412 (1986).

Evidence that the CD4-gp120 binding is responsible for viral infection of cells bearing the CD4 antigen includes the finding that a specific complex is formed between gp120 and CD₄ (McDougal et al., supra). Other investigators have shown that the cell lines, which were non-infective for HIV, were converted to infectable cell lines following transfection and expression of the human CD4 cDNA gene. Maddon et al., Cell 46:333-348 (1986).

Therapeutic programs based on soluble CD4 as a passive agent to interfere with viral adsorption and syncytium-mediated cellular transmission have been proposed and successfully demonstrated in vitro by a number of groups (Deen et al., Nature 331:82-84 (1988); Fisher et al., Nature 331:76-78 (1988); Hussey et al., Nature 331:78-81 (1988); Smith et al., Science 238:1704-1707 (1987); Traunecker et al., Nature 331:84-86 (1988)); and CD4 immunoglobulin fusion proteins with extended half-lives and modest biological activity have subsequently been developed (Capon et al., Nature 337:525-531 (1989); Traunecker et al. Nature 339, 68-70 (1989); Byrn et al., Nature 344:667-670 (1990); Zettlmeissl et al., DNA Cell Biol. 9:347-353 (1990)). Although CD4 immunotoxin conjugates or fusion proteins show potent cytotoxicity for infected cells in vitro (Chaudhary et al., Nature 335:369-372 (1988); Till et al., Science 242:1166-1168 (1988)), the latency of the immunodeficiency syndrome makes it unlikely that any single-treatment therapy will be effective in eliminating viral burden, and the antigenicity of foreign fusion proteins is likely to limit their acceptability in treatments requiring repetitive dosing. Trials with monkeys affected with SIV have shown that soluble CD4, if administered to animals without marked CD4 cytopenia, can reduce SIV titer and improve in vitro measures of myeloid potential (Watanabe et al., Nature 337:267-270 (1989)). However a prompt viral reemergence was observed after treatment was discontinued, suggesting that lifelong administration might be necessary to prevent progressive immune system debilitation.

### T Cell and Fc Receptors

Cell surface expression of the most abundant form of the T cell antigen receptor (TCR) requires the coexpression of at least 6 distinct polypeptide chains (Weiss et al., J. Exp. Med. 160:1284-1299 (1984); Orloffhashi et al., Nature 316:606-609 (1985); Berkhout et al., J. Biol. Chem. 263:8528-8536 (1988); Sussman et al., Cell 52:85-95 (1988)), the α/β antigen binding chains, the three polypeptides of the CD3 complex, and ζ. If any of the chains are absent, stable expression of the remaining members of the complex does not ensue. ζ is the limiting polypeptide for surface expression of the complete complex (Sussman et al., Cell 52:85-95 (1988)) and is thought to mediate at least a fraction of the cellular activation programs triggered by receptor recognition of ligand (Weissman et al., EMBO J. 8:3651-3656 (1989); Frank et al., Science 249:174-177 (1990)). A 32kDa type I integral membrane homodimer, ζ (zeta) has a 9 residue extracellular domain with no sites for N-linked glycan addition, and a 112 residue (mouse) or 113 residue (human) intracellular domain (Weissman et al., Science 238:1018-1020 (1988); Weissman et al., Proc. Natl. Acad. Sci. USA 85:9709-9713 (1988)). An isoform of ζ called η (eta) (Baniyash et al., J. Biol. Chem. 263:9874-9878 (1988); Orloff et al., J. Biol. Chem. 264:14812-14817 (1989)), which arises from an alternate mRNA splicing pathway (Jin et al., Proc. Natl. Acad. Sci. USA 87:3319-3233 (1990)), is present in reduced amounts in cells expressing the antigen receptor. ζ-η heterodimers are thought to mediate the formation of inositol phosphates, as well as the receptor-initiated programmed cell death called apoptosis (Mercep et al., Science 242:571-574 (1988); Mercep et al., Science 246:1162-1165 (1989)).

Like ζ and η, the Fc receptor-associated γ (gamma) chain is expressed in cell surface complexes with additional polypeptides, some of which mediate ligand recognition, and others of which have undefined function. γ bears a homodimeric structure and overall organization very similar to that of ζ and is a component of both the mast cell/basophil high affinity IgE receptor, FcεRI, which consists of at least three distinct polypeptide chains (Blank et al., Nature 337:187-189 (1989); Ra et al., Nature 241:752-754 (1989)), and one of the low affinity receptors for IgG, represented in mice by FcγRIIα (Ra et al., J. Biol. Chem. 264:15323-15327 (1989)), and in humans by the CD16 subtype expression by macrophages and natural killer cells, CD16_{TM} (CD16 transmembrane) (Lanier et al., Nature 342:803-805 (1989); Anderson et al., Proc. Natl. Acad. Sci. USA 87:2274-2278 (1990)) and with a polypeptide of unidentified function (Anderson et al., Proc. Natl. Acad. Sci. USA 87:2274-2278 (1990)). Recently it has been reported that γ is expressed by a mouse T cell line, CTL, in which it forms homodimers as well as γ-ζ and γ-η heterodimers (Orloff et al., Nature 347:189-191 (1990)).

The Fc receptors mediate phagocytosis of immune complexes, transcytosis, and antibody dependent cellular cytotoxicity (ADCC) (Ravetch and Kinet, Annu. Rev. Immunol. 9:457-492 (1991); Unkeless et al., Annu. Rev. Immunol 6:251-281 (1988); and Mellman, Curr. Opin. Immunol. 1:16-25 (1988)). Recently it has been shown that one of the murine low affinity Fc receptor isoforms, FcRγIIIB1, mediates internalization of Ig-coated targets into clathrin coated pits, and that another low affinity receptor, FcRγIIIA mediates ADCC through its association with one or more members of a small family of 'trigger molecules' (Miettinen et al., Cell 58:317-327 (1989); and Hunziker and Mellman, J. Cell Biol. 109:3291-3302 (1989)). These trigger molecules, T cell receptor (TCR) ζ chain, TCR η chain, and Fc receptor γ chain, interact with ligand recognition domains of different immune system receptors and can autonomously initiate cellular effector programs, including cytolysis, following aggregation (Samelson et al., Cell 43:223-231 (1985); Weissman et al., Science 239:1018-1020 (1988); Jin et al., Proc. Natl. Acad. Sci. USA 87:3319-3323 (1990); Blank et al., Nature 337:187-189 (1989); Lanier et al., Nature 342:803-805 (1989); Kurosaki and Ravetch, Nature 342:805-807 (1989); Hibbs et al., Science 246:1608-1611 (1989); Anderson et al., Proc. Natl. Acad. Sci USA 87:2274-2278 (1990); and Irving and Weiss, Cell 64: 891-901 (1991)).

In drawing parallels between the murine and human low affinity Fc receptor families, however, it has become clear that the human FcRγIIA and C isoforms have no murine counterpart. In part because of this, their function has yet to be defined.

Because humoral agents based on CD4 alone may have limited utility in vivo, previous work explored the possibility of augmenting cellular immunity to HIV. Preparations of protein chimeras in which the extracellular domain of CD4 is fused to the transmembrane and/or intracellular domains of T cell receptor, IgG Fc receptor, or B cell receptor signal transducing elements have been identified (U.S.S.N. 07/847,566 and 07/665,961, hereby incorporated by reference). Cytolytic T cells expressing chimeras which include an extracellular CD4 domain show potent MHC-independent destruction of cellular targets expressing HIV envelope proteins. An extremely important and novel component of this approach has been the identification of single T cell receptor, Fc receptor, and B cell receptor chains whose aggregation suffices to initiate the cellular response.

One particularly useful application of this approach has been the invention of chimeras between CD4 and ζ, η, or γ that direct cytolytic T lymphocytes to recognize and kill cells expressing HIV gp120 (U.S.S.N. 07/847,566 and 07/665,961, hereby incorporated by reference).

### Summary of the Invention

Although native T cell, B cell, and Fc receptors are or can be highly complicated multineric structures not lending themselves to convenient manipulation, the present invention demonstrates the feasibility of creating chimeras between the intracellular domain of any of a variety of molecules which are capable of fulfilling the task of target recognition. In particular, the formation of chimeras consisting of the intracellular portion of T cell/Fc receptor zeta, eta, or gamma chains joined to the extracellular portion of a suitably engineered antibody molecule allows the target recognition potential of an immune system cell to be specifically redirected to the antigen recognized by the extracellular antibody portion. Thus with an antibody portion capable of recognizing some determinant on the surface of a pathogen, immune system cells armed with the chimera would respond to the presence of the pathogen with the effector program appropriate to their lineage, e.g., helper T lymphocytes would respond by cytotoxic activity against the target, and B lymphocytes would be activated to synthesize antibody. Macrophages and granulocytes would carry out their effector programs, including cytokine release, phagocytosis, and reactive oxygen generation. Similarly, with an antibody portion capable of recognizing tumor cells, the immune system response to the tumor would be beneficially elevated. With an antibody capable of recognizing immune cells having an inappropriate reactivity with self determinants, the autoreactive cells could be selectively targeted for destruction.

Although these examples draw on the use of antibody chimeras as a convenient expository tool, the invention is not limited in scope to antibody chimeras, and indeed, the use of specific nonantibody extracellular domains may have important advantages. For example with an extracellular portion that is the receptor for a virus, bacterium, or parasite, cells armed with the chimeras would specifically target cells expressing the viral, bacterial, or parasitic determinants. The advantage of this approach over the use of antibodies is that the native receptor for pathogen may have uniquely high selectivity or affinity for the pathogen, allowing a greater degree of precision in the resulting immune response. Similarly, to delete immune system cells which inappropriately react with a self antigen, it may suffice to join the antigen (either as an intact protein, in the case of B cell depletion therapies, or as MHC complex, in the case of T cell depletion therapies) to intracellular zeta, eta, or gamma chains, and thereby affect the specific targeting of the cells inappropriately responding to self determinants.

Another use of the chimeras is the control of cell populations in vivo subsequent to other forms of genetic engineering. For example, the use of tumor infiltrating lymphocytes or natural killer cells to carry cytotoxic principles to the site of tumors has been proposed. The present invention provides a convenient means to regulate the numbers and activity of such lymphocytes and cells without removing them from the body of the patient for amplification in vitro. Thus, because the intracellular domains of the chimeric receptors mediate the proliferative responses of the cells, the coordination of the extracellular domains by a variety of aggregating stimuli specific for the extracellular domains (e.g., an antibody specific for the extracellular domain) will result in proliferation of the cells bearing the chimeras.

Although the specific embodiments of the present invention comprise chimeras between zeta, eta, or gamma chains, or active fragments thereof (e.g., those discussed below), any receptor chain having a similar function to these molecules, e.g., in granulocytes or B lymphocytes, could be used for the purposes disclosed here. The distinguishing features of desirable immune cell trigger molecules comprise the ability to be expressed autonomously (i.e., as a single chain), the ability to be fused to an extracellular domain such that the resultant chimera is present on the surface of a therapeutic cell, and the ability to initiate cellular effector programs upon aggregation secondary to encounter with a target ligand.

At present the most convenient method for delivery of the chimeras to immune system cells is through some form of genetic therapy. However, reconstituting immune system cells with chimeric receptors by mixture of the cells with suitably solubilized purified chimeric protein would also result in the formation of an engineered cell population capable of responding to the targets recognized by the extracellular domain of the chimeras. Similar approaches have been used, for example, to introduce the intact HIV receptor, CD4, into erythrocytes for therapeutic purposes. In this case the engineered cell population would not be capable of self renewal.

The invention concerns a method for manufacturing a medicament for the therapeutic application in an infection, a tumor disease or an autoimmune disease, characterized in that therapeutic cells are generated expressing at least two membrane-bound, proteinaceous chimeric receptors
one of said receptors comprising (a) an extracellular portion which is capable of specifically recognizing and binding a target cell or a target infective agent and (b) an intracellular or transmembrane portion which is capable of signaling said therapeutic cell to destroy a receptor-bound target cell or a receptor-bound target infective agent; wherein said intracellular of transmembrane portion is derived from a T cell receptor ξ, η, CD3 delta, or T3 gamma protein; an Fc receptor r protein; or a B cell receptor mb1 or B29 protein, or a functional derivative thereof;
and
the second of said receptors comprising (a) an extracellular portion which is capable of specifically recognizing and binding said target cell or said target infective agent and (b) an intracellular portion which is derived from CD28.

The invention concerns a cell which expresses at least two proteinaceous membrane-bound chimeric receptors, one of said receptors comprising (a) an extracellular portion wherein said intracellular or transmembrane portion is the cell signal-transducing portion of a T cell receptor protein, a B cell receptor protein, or an Fc receptor protein, or a functional derivative thereof; and the second of said receptors comprising (a) an extracellular portion, wherein said extracellular portion of the first and the second receptor comprises an HIV envelope-binding portion of CD4, or a functional HIV envelope-binding derivative thereof; and (b) an intracellular portion which is derived from CD28.

The invention further concerns a cell which expresses at least two proteinaceous membrane-bound chimeric receptors, one of said receptors comprising (a) an extracellular portion, and (b) an intracellular or transmembrane portion derived form a T cell receptor CD3, zeta, or eta polypeptide, a B cell receptor, or an Fc receptor; and
the second of said receptors comprising (a) an extracellular portion, wherein said extracellular portion of the first and the second receptor comprise an HIV envelope-binding portion of CD4, or a functional HIVenvelope-binding derivative thereof, and(b) an intracellular portion which is derived from CD28.

The invention further concerns a pair or proteinaceous membrane-bound chimeric receptors, one of said receptors comprising (a) an extracellular portion and (b) an intracellular or transmembrane portion wherein said intracellular or transmembrane portion is the signal-transducing portion of a T cell receptor protein, a B cell receptor protein, or an Fc receptor protein, or a functional derivative thereof; and the second of said receptors comprising (a) and extracellular portion, wherein said extracellular portions of the first and the second receptor comprise an HIV envelope-binding portion of CD4, or a functional HIV envelope-binding derivative thereof; and (b) an intracellular portion which is derived from CD28.

The invention further concerns a pair of proteinaceous membrane-bound chimeric receptors, one of said receptors comprising (a) an extracellular portion and (b) an intracellular or transmembrane portion derived from a T cell receptor CD3, zeta or eta polypeptide, a B cell receptor, or an Fc receptor; and
the second of said receptors comprising (a) an extracellular portion, wherein said extracellular portions of the first and the second receptor comprise HIV envelope-binding portion of CD4, or a functional HIV envelope-binding derivative thereof and (b) an intracellular portion which is derived from CD28.

In another embodiment, the method of directing cellular response to an infective agent comprises administering therapeutic cells capable of recognizing and destroying said agent, wherein the agent is a specific virus, bacteria, protozoa, or fungi. Even more specifically, the method is directed against agents such as HIV and Pneumocystis carinii.

Specifically the invention provides for a method of directing cellular response to an HIV-infected cell. The method comprises administering to a patient an effective amount of cytotoxic T lymphocytes, said lymphocytes being capable of specifically recognizing and lysing cells infected with HIV as well as circulating virus.

Thus, in one embodiment, there is provided according to the invention a method for directing cellular response to HIV-infected cells, comprising administering to a patient an effective amount of cytotoxic T lymphocytes which are capable of specifically recognizing and lysing cells infected with HIV.

In yet another embodiment is provided the chimeric receptor proteins which direct the cytotoxic T lymphocytes to recognize and lyse the HIV-infected cell. Yet another embodiment of the invention comprises host cells transformed with a vector comprising the chimeric receptors.

In yet another embodiment, the present invention provides for an antibody against the chimeric receptors of the invention.

In order to obtain cytotoxic T lymphocytes which specifically bind and lyse cells infected with HIV, the present inventors therefore attempted, and herein provide receptor chimeras. These chimeric receptors are functionally active and possess the extraordinary ability of being able to specifically bind and lyse cells expressing gp120.

It is an object of the present invention, then, to provide for a method for manufacturing a medicament for treatment for individuals infected with HIV. The present invention thus provides a number of important advances in the therapy of AIDS.

These and other non-limiting embodiments of the present invention will be apparent to those of skill from the following detailed description of the invention.

In the following detailed description, reference will be made to various methodologies known to those of skill in the art of molecular biology and immunology.

Standard references works setting forth the general principles or recombinant DNA technology include Watson et al., Molecular Biology of the Gene, volumes I and II, the Benjamin/Cummings Publishing Company, Inc., publisher, Menlo Park, CA (1987); Darnell et al., Molecular Cell Biology, Scientific American Books, Inc., publisher, New York, N.,Y. (1986); Lewin, Genes II, John Wiley & Sons, publishers, New York, N.Y., (1985); Old et al., Principles of Gene Manipulation: An Introduction to Genetic Engineering, 2^{nd} edition, University of California Press, publisher, Berkeley, CA (1981); Maniatis et al., Molecular Cloning: A Laboratory Manual, 2^{nd} ed., Cold Spring Harbor Laboratory, publisher, Cold Spring Harbor, NY (1989); and Current Protocols in Molecular biology, Ausubel et al., Wiley Press, New York, NY (1989).

### DEFINITIONS

By "cloning" is meant the use of in vitro recombination techniques to insert a particular gene or other DNA sequence into a vector molecule. In order to successfully clone a desired gene, it is necessary to employ methods for generating DNA fragments for joining the fragments to vector molecules, for introducing the composite DNA molecule into a host cell in which it can replicate, and for selecting the clone having the target gene from amongst the recipient host cells.

By "cDNA" is meant complementary or copy DNA produced from an RNA template by the action of RNA-dependent DNA polymerase (reverse transcriptase). Thus a "cDNA clone" means a duplex DNA sequence complementary to an RNA molecule of interest, carried in a cloning vector.

By "cDNA library" is meant a collection of recombinant DNA molecules containing cDNA inserts which comprise DNA copies of mRNA being expressed by the cell at the time the cDNA library was made. Such a cDNA library may be prepared by methods known to those of skill, and described, for example, in Maniatis et al., Molecular Cloning: A Laboratory Manual, supra. Generally, RNA is first isolated from the cells of an organism from whose genome it is desired to clone a particular gene. Preferred for the purpose of the present invention are mammalian, and particularly human, lymphocytic cell lines. A presently preferred vector for this purpose is the vaccinia virus WR strain.

By "vector" is meant a DNA molecule derived, e.g., from a plasmid, bacteriophage, or mammalian or insect virus, into which fragments of DNA may be inserted or cloned. A vector will contain one or more unique restriction sites and may be capable of autonomous replication in a defined host or vehicle organism such that the cloned sequence is reproducible. Thus, by "DNA expression vector" is meant any autonomous element capable of directing the synthesis of a recombinant peptide. Such DNA expression vectors include bacterial plasmids and phages and mammalian and insect plasmids and viruses.

By "substantially pure" is meant a compound, e.g., a protein, a polypeptide, or an antibody, that is substantially free of the components that naturally accompany it. Generally, a compound is substantially pure when at least 60%, more preferably at least 75%, and most preferably at least 90% of the total material in a sample is the compound of interest. Purity can be measured by any appropriate method, e.g., column chromatography, polyacrylamide gel electrophoresis, or HPLC analysis. In the context of a nucleic acid, "substantially pure" means a nucleic acid sequence, segment, or fragment that is not immediately contiguous with (i.e., covalently linked to) both of the coding sequences with which it is immediately contiguous (i.e., one at the 5' end and one at the 3' end) in the naturally occurring genome of the organism from which the DNA of the invention is derived.

By "functional derivative" is meant the "fragments," "variants," "analogues," or "chemical derivatives" of a molecule. A "fragment" of a molecule, such as any of the cDNA sequences of the present invention, is meant to refer to any nucleotide subset of the molecule. A "variant" of such molecule is meant to refer to a naturally occurring molecule substantially similar to either the entire molecule, or a fragment thereof. An "analog" of a molecule is meant to refer to a non-natural molecule substantially similar to either the entire molecule or a fragment thereof. A molecule is said to be "substantially similar" to another molecule if the sequence of amino acids in both molecules is substantially the same. In particular, a "substantially similar" amino acid sequence is one that exhibits at least 50%, preferably 85%, and most preferably 95% amino acid sequence identity to the natural or reference sequence and/or one that differs from the natural or reference amino acid sequence only by conservative amino acid substitutions. Substantially similar amino acid molecules possess similar biological activity. Thus, provided that two molecules possess a similar activity, they are considered variants as that term is used herein even if one of the molecules contains additional or fewer amino acid residues not found in the other, or if the sequence of amino acid residues is not identical. As used herein, a molecule is said to be a "chemical derivative" of another molecule when it contains additional chemical moieties not normally a part of the molecule. Such moieties may improve the molecule's solubility, absorption, biological half life, etc. The moieties may alternatively decrease the toxicity of the molecule, eliminate or attenuate any undesirable side effect of the molecule, etc. Moieties capable of mediating such effects are disclosed, for example, in Remington's Pharmaceutical Sciences, 16th ed., Mack Publishing Co., Easton, PA (1980).

Similarly, a "functional derivative" of a receptor chimera gene of the present invention is meant to include "fragments," "variants," or "analogues" of the gene, which may be "substantially similar" in nucleotide sequence, and which encode a molecule possessing similar activity to, for example, a T cell, B cell, or Fc receptor chimera. "Substantially similar" nucleic acids encode substantially similar amino acid sequences and also may include any nucleic acid sequence capable of hybridizing to the wild-type nucleic acid sequence under appropriate hybridization conditions (see, for example, Ausubel et al., Current Protocols in Molecular Biology, Wiley Press, New York, NY (1989) for appropriate hybridization stringency conditions).

Thus, as used herein, a T cell, B cell, or Fc receptor chimera protein is also meant to include any functional derivative, fragments, variants, analogues, or chemical derivatives which may be substantially similar to the "wild-type" chimera and which possess similar activity (i.e., most preferably, 90%, more preferably, 70%, preferably 40%, or at least 10% of the wild-type receptor chimera's activity). The activity of a functional chimeric receptor derivative includes specific binding (with its extracellular portion) to a targeted agent or cell and resultant destruction (directed by its intracellular or transmembrane portion) of that agent or cell; such activity may be tested, e.g., using any of the assays described herein.

A DNA sequence encoding the T cell, B cell, or Fc receptor chimera of the present invention, or its functional derivatives, may be recombined with vector DNA in accordance with conventional techniques, including blunt-ended or staggered-ended termini for ligation, restriction enzyme digestion to provide appropriate termini, filling in of cohesive ends as appropriate, alkaline phosphatase treatment to avoid undesirable joining, and ligation with appropriate ligases. Techniques for such manipulations are disclosed by Maniatis et al., supra, and are well known in the art.

A nucleic acid molecule, such as DNA, is said to be "capable of expressing" a polypeptide if it contains nucleotide sequences which contain transcriptional and translational regulatory information, and such sequences are "operably linked" to nucleotide sequences which encode the polypeptide. An operable linkage is a linkage in which the regulatory DNA sequences and the DNA sequence sought to be expressed are connected in such a way as to permit gene expression. The precise nature of the regulatory regions needed for gene expression may vary from organism to organism, but shall in general include a promoter region which, in prokaryotes, contains both the promoter (which directs the initiation of RNA transcription) as well as the DNA sequences which, when transcribed into RNA, will signal the initiation of protein synthesis. Such regions will normally include those 5'-non-coding sequences involved with initiation of transcription and translation, such as the TATA box, capping sequence, CAAT sequence, and the like.

If desired, the non-coding region 3' to the gene sequence coding for the protein may be obtained by the above-described methods. This region may be retained for its transcriptional termination regulatory sequences, such as termination and polyadenylation. Thus, by retaining the 3'-region naturally contiguous to the DNA sequence coding for the protein, the transcriptional termination signals may be provided. Where the transcriptional termination signals are not satisfactorily functional in the expression host cell, then a 3' region functional in the host cell may be substituted.

Two DNA sequences (such as a promoter region sequence and a T cell receptor, B cell receptor, or Fc receptor chimera encoding sequence) are said to be operably linked if the nature of the linkage between the two DNA sequences does not (1) result in the introduction of a frame-shift mutation, (2) interfere with the ability of the promoter region sequence to direct the transcription of the receptor chimera gene sequence, or (3) interfere with the ability of the receptor chimera gene sequence to be transcribed by the promoter region sequence. A promoter region would be operably linked to a DNA sequence if the promoter were capable of effecting transcription of that DNA sequence. Thus, to express the protein, transcriptional and translational signals recognized by an appropriate host are necessary.

The present invention encompasses the expression of a T cell receptor, B cell receptor, or Fc receptor chimera protein (or a functional derivative thereof) in either prokaryotic or eukaryotic cells, although eukaryotic (and, particularly, human lymphocyte) expression is preferred.

Antibodies according to the present invention may be prepared by any of a variety of methods. For example, cells expressing the receptor chimera protein, or a functional derivative thereof, can be administered to an animal in order to induce the production of sera containing polyclonal antibodies that are capable of binding the chimera.

In a preferred method, antibodies according to the present invention are monoclonal antibodies. Such monoclonal antibodies can be prepared using hybridoma technology (Kohler et al., Nature 256:495 (1975); Kohler et al., Eur. J. Immunol. 6:511 (1976); Kohler et al., Eur. J. Immunol. 6:292 (1976); Hammerling et al., In: Monoclonal Antibodies and T-Cell Hybridomas, Elsevier, N.Y., pp. 563-684 (1981)). In general, such procedures involve immunizing an animal with the T cell receptor, B cell receptor, or Fc receptor chimera antigen. The splenocytes of such animals are extracted and fused with a suitable myeloma cell line. Any suitable myeloma cell line may be employed in accordance with the present invention. After fusion, the resulting hybridoma cells are selectively maintained in HAT medium, and then cloned by limiting dilution as described by Wands et al. (Gastroenterology 80:225-232 (1981)). The hybridoma cells obtained through such a selection are then assayed to identify clones which secrete antibodies capable of binding the chimera.

Antibodies according to the present invention also may be polyclonal, or, preferably, region specific polyclonal antibodies.

Antibodies against the T cell receptor, B cell receptor, or Fc receptor chimera according to the present invention may be used to monitor the amount of chimeric receptor (or chimeric receptor-bearing cells) in a patient. Such antibodies are well suited for use in standard immunodiagnostic assays known in the art, including such immunometric or "sandwich" assays as the forward sandwich, reverse sandwich, and simultaneous sandwich assays. The antibodies may be used in any number of combinations as may be determined by those of skill without undue experimentation to effect immunoassays of acceptable specificity, sensitivity, and accuracy.

Standard reference works setting forth general principles of immunology include Roitt, Essential Immunology, 6th ed., Blackwell Scientific Publications, publisher, Oxford (1988); Kimball, Introduction to Immunology, 2nd ed., Macmillan Publishing Co., publisher, New York (1986); Roitt et al., Immunology, Gower Medical Publishing Ltd., publisher, London, (1985); Campbell, "Monoclonal Antibody Technology," in Burdon et al., eds., Laboratory Techniques in Biochemistry and Molecular Biology, volume 13, Elsevier, publisher, Amsterdam (1984); Klein, Immunology: The Science of Self-Nonself Discrimination, John Wiley & Sons, publisher, New York (1982); and Kennett et al., eds., Monoclonal Antibodies, Hybridoma: A New Dimension In Biological Analyses, Plenum Press, publisher, New York (1980).

By "detecting" it is intended to include determining the presence or absence of a substance or quantifying the amount of a substance. The term thus refers to the use of the materials, compositions, and methods of the present invention for qualitative and quantitative determinations.

The isolation of other hybridomas secreting monoclonal antibodies of the same specificity as those described herein can be accomplished by the technique of anti-idiotypic screening (Potocmjak et al., Science 215:1637 (1982)). Briefly, an anti-idiotypic antibody is an antibody which recognizes unique determinants present on the antibody produced by the clone of interest. The anti-idiotypic antibody is prepared by immunizing an animal of the same strain used as the source of the monoclonal antibody with the monoclonal antibody of interest. The immunized animal will recognize and respond to the idiotypic determinants of the immunizing antibody by producing antibody to these idiotypic determinants (anti-idiotypic antibody).

For replication, the hybrid cells may be cultivated both in vitro and in vivo. High in vivo production makes this the presently preferred method of culture. Briefly, cells from the individual hybrid strains are injected intraperitoneally into pristane-primed BALB/c mice to produce ascites fluid containing high concentrations of the desired monoclonal antibodies. Monoclonal antibodies of isotype IgM or IgG may be purified from cultured supernatants using column chromatography methods well known to those of skill in the art.

Antibodies according to the present invention are particularly suited for use in immunoassays wherein they may be utilized in liquid phase or bound to a solid phase carrier. In addition, the antibodies in these immunoassays can be detectably labeled in various ways.

There are many different labels and methods of labeling known in the art. Examples of the types of labels which can be used in the present invention include, but are not limited to, enzymes, radioisotopes, fluorescent compounds, chemiluminescent compounds, bioluminescent compounds, and metal chelates. Those of ordinary skill in the art will know of other suitable labels for binding to antibodies, or will be able to ascertain the same by the use of routine experimentation. Furthermore, the binding of these labels to antibodies can be accomplished using standard techniques commonly known to those of ordinary skill in the art.

One of the ways in which antibodies according to the present invention can be detectably labeled is by linking the antibody to an enzyme. This enzyme, in turn, when later exposed to its substrate, will react with the substrate in such a manner as to produce a chemical moiety which can be detected as, for example, by spectrophotometric or fluorometric means. Examples of enzymes which can be used to detectably label antibodies include malate dehydrogenase, staphylococcal nuclease, delta-V-steroid isomerase, yeast alcohol dehydrogenase, alpha-glycerophosphate dehydrogenase, triose phosphate isomerase, biotinavidin peroxidase, horseradish peroxidase, alkaline phosphatase, asparaginase, glucose oxidase, β-galactosidase, ribonuclease, urease, catalase, glucose-VI-phosphate dehydrogenase, glucoamylase, and acetylcholine esterase.

The presence of detectably labeled antibodies also can be detected by labeling the antibodies with a radioactive isotope which then can be determined by such means as the use of a gamma counter or a scintillation counter. Isotopes which are particularly useful for the purpose of the present invention are ³H, ¹²⁵I, ³²P, ³⁵S, ¹⁴C, ⁵¹Cr, ³⁶Cl, ⁵⁷Co, ⁵⁸Co, ⁵⁹Fe, and ⁷⁵Se.

It is also possible to detect the binding of detectably labeled antibodies by labeling the antibodies with a fluorescent compound. When a fluorescently labeled antibody is exposed to light of the proper wavelength, its presence then can be detected due to the fluorescence of the dye. Among the most commonly used fluorescent labeling compounds are fluorescein, isothiocyanate, rhodamine, phycoerythrin, phycocyanin, allophycocyanin, o-phthaldehyde, and fluorescamine.

The antibodies of the invention also can be detectably labeled using fluorescent emitting metals such as ¹⁵²Eu, or others of the lanthanide series. These metals can be attached to the antibody molecule using such metal chelating groups as diethyl-enteriaminepentaacetic acid (DTPA) or ethylenediaminetetraacetic acid (EDTA).

Antibodies also can be detectably labeled by coupling them to a chemiluminescent compound. The presence of the chemiluminescent-tagged antibody is then determined by detecting the presence of luminescence that arises during the course of the chemical reaction. Examples of particularly useful chemiluminescent labeling compounds are luminal, isoluminol, theromatic acridinium ester, imidazole, acridinium salts, oxalate ester, and dioxetane.

Likewise, a bioluminescent compound may be used to label the antibodies according to the present invention. Bioluminescence is a type of chemiluminescence found in biological systems in which a catalytic protein increases the efficiency of the chemiluminescent reaction. The presence of a bioluminescent antibody is determined by detecting the presence of luminescence. Important bioluminescent compounds for purposes of labeling include luciferin and luciferase aequorin.

The antibodies and substantially purified antigen of the present invention are ideally suited for the preparation of a kit. Such a kit may comprise a carrier means being compartmentalized to receive in close confinement therewith one or more container means such as vials, tubes, and the like, each of said container means comprising the separate elements of the assay to be used.

The types of assays which can be incorporated in kit form are many and include, for example, competitive and non-competitive assays. Typical examples of assays which can utilize the antibodies of the invention are radioimmunoassays (RIA), enzyme immunoassays (EIA), enzyme-linked immunosorbent assays (ELISA), and immunometric, or sandwich immunoassays.

By the term "immunometric assay" or "sandwich immunoassay," it is meant to include simultaneous sandwich, forward sandwich, and reverse sandwich immunoassays. These terms are well understood by those skilled in the art. Those of skill will also appreciate that antibodies according to the present invention will be useful in other variations and forms of assays which are presently known or which may be developed in the future. These are intended to be included within the scope of the present invention.

In the preferred mode for performing the assays it is important that certain "blockers" be present in the incubation medium (usually added with the labeled soluble antibody). The "blockers" are added to assure that nonspecific proteins, protease, or human antibodies to mouse immunoglobulins present in the experimental sample do not cross-link or destroy the antibodies on the solid phase support, or the radiolabeled indicator antibody, to yield false positive or false negative results. The selection of "blockers" therefore adds substantially to the specificity of the assays described in the present invention.

It has been found that a number of nonrelevant (i.e., nonspecific) antibodies of the same class or subclass (isotype) as those used in the assays (e.g., IgG₁, IgG₂ₐ, IgM, etc.) can be used as "blockers." The concentration of the "blockers" (normally 1-100 µg/µl) is important, in order to maintain the proper sensitivity yet inhibit any unwanted interference by mutually occurring cross-reactive proteins in human serum. In addition, the buffer system containing the "blockers" needs to be optimized. Preferred buffers are those based on weak organic acids, such as imidazole, HEPPS, MOPS, TES, ADA, ACES, HEPES, PIPES, TRIS, and the like, at physiological pH ranges. Somewhat less preferred buffers are inorganic buffers such as phosphate, borate, or carbonate. Finally, known protease inhibitors are preferably added (normally at 0.01-10 µg/ml) to the buffer which contains the "blockers."

There are many solid phase immunoadsorbents which have been employed and which can be used in the present invention. Well known immunoadsorbents include glass, polystyrene, polypropylene, dextran, nylon, and other materials, in the form of tubes, beads, and microtiter plates formed from or coated with such materials, and the like. The immobilized antibodies can be either covalently or physically bound to the solid phase immunoadsorbent, by techniques such as covalent bonding via an amide or ester linkage, or by absorption. Those skilled in the art will know many other suitable solid phase immunoadsorbents and methods for immobilizing antibodies thereon, or will be able to ascertain such, using no more than routine experimentation.

For in vivo, in vitro, or in situ diagnosis, labels such as radionuclides may be bound to antibodies according to the present invention either directly or by using an intermediary functional group. An intermediary group which is often used to bind radioisotopes which exist as metallic cations to antibodies is diethylenetriaminepentaacetic acid (DTPA). Typical examples of metallic cations which are bound in this manner are: ^{99m}Tc, ¹²³I, ¹¹¹In, ¹³¹I, ⁹⁷Ru, ⁶⁷Cu, ⁶⁷Ga, and ⁶⁸Ga. The antibodies of the invention can also be labeled with non-radioactive isotopes for purposes of diagnosis. Elements which are particularly useful in this manner are ¹⁵⁷Gd, ⁵⁵Mn, ¹⁶²Dy, ⁵²Cr, and ⁵⁶Fe.

The antigen of the invention may be isolated in substantially pure form employing antibodies according to the present invention. Thus, an embodiment of the present invention provides for substantially pure T cell receptor, B cell receptor, or Fc receptor chimera, said antigen characterized in that it is recognized by and binds to antibodies according to the present invention. In another embodiment, the present invention provides a method of isolating or purifying the receptor chimeric antigen, by forming a complex of said antigen with one or more antibodies directed against the receptor chimera.

The substantially pure T cell receptor, B cell receptor, or Fc receptor chimera antigens of the present invention may in turn be used to detect or measure antibody to the chimera in a sample, such as serum or urine. Thus, one embodiment of the present invention comprises a method of detecting the presence or amount of antibody to T cell receptor, B cell receptor, or Fc receptor chimera antigen in a sample, comprising contacting a sample containing an antibody to the chimeric antigen with detectably labeled receptor chimera, and detecting said label. It will be appreciated that immunoreactive fractions and immunoreactive analogues of the chimera also may be used. By the term "immunoreactive fraction" is intended any portion of the chimeric antigen which demonstrates an equivalent immune response to an antibody directed against the receptor chimera. By the term "immunoreactive analogue" is intended a protein which differs from the receptor chimera protein by one or more amino acids, but which demonstrates an equivalent immunoresponse to an antibody of the invention.

By "specifically recognizes and binds" is meant that the antibody recognizes and binds the chimeric receptor polypeptide but does not substantially recognize and bind other unrelated moleucles in a sample, e.g., a biological sample.

By "autoinmune-generated cell" is meant cells producing antibodies that react with host tissue or immune effector T cells that are autoreactive; such cells include antibodies against acetylcholine receptors (leading, e.g., to myasthenia gravis) or anti-DNA, anti-erythrocyte, and anti-placelet autoantibodies (leading, e.g., to lupus erythematosus).

By "therapeutic cell" is meant a cell which has been transformed by a chimera of the invention so that it is capable of recognizing and destroying a specific infective agent, a cell infected by a specific agent, a tumor or cancerous cell, or an autoimmune-generated cell; preferably such therapeutic cells are cells of the hematopoietic system.

By a "target infective agent" is meant any infective agent (e.g., a virus, bacterium, protozoan, or fungus) which can be recognized by a chimeric receptor-bearing therapeutic cell. By a "target cell" is meant any host cell which can be recognized by a chimeric receptor-bearing therapeutic cell; target cells include, without limitation, host cells which are infected with a virus, bacterium, protozoan, or fungus as well as tumor or cancerous cells and autoimmune-generated cells.

By "extracellular" is meant having at least a portion of the molecule exposed at the cell surface. By "intracellular" is meant having at least a portion of the molecule exposed to the therapeutic cell's cytoplasm. By "transmembrane" is meant having at least a portion of the molecule spanning the plasma membrane. An "extracellular portion," an "intracellular portion," and a "transmembrane portion," as used herein, may include flanking amino acid sequences which extend into adjoining cellular compartments.

By "oligomerize" is meant to complex with other proteins to form dimers, trimers, tetramers, or other higher order oligomers. Such oligomers may be homo-oligomers or hetero-oligomers. An "oligomerizing portion" is that region of a molecule which directs complex (i.e., oligomer) formation.

By "cytolytic" is meant to be capable of destroying a cell (e.g., a cell infected with a pathogen, a tumor or cancerous cell, or an autoimmune-generated) cell or to be capable of destroying an infective agent (e.g., a virus).

By "immunodeficiency virus" is meant a retrovirus that, in wild-type form, is capable of infecting T4 cells of a primate host and possesses a viral morphogenesis and morphology characteristic of the lentivirus subfamily. The term includes, without limitation, all variants of HIV and SIV, including HIV-1, HIV-2, SIVmac, SIVagm, SIVmnd, SIVsmm, SIVman, SIVmand, and SIVcpz.

By "MHC-independent" is meant that the cellular cytolytic response does not require the presence of an MHC class II antigen on the surface of the targeted cell.

By a "functional cytolytic signal-transducing derivative" is meant a functional derivative (as defined above) which is capable of directing at least 10%, preferably 40%, more preferably 70%, or most preferably at least 90% of the biological activity of the wild type molecule. As used herein, a "functional cytolytic signal-transducing derivative" may act by directly signaling the therapeutic cell to destroy a receptor-bound agent or cell (e.g., in the case of an intracellular chimeric receptor portion) or may act indirectly by promoting oligomerization with cytolytic signal transducing proteins of the therapeutic cell (e.g., in the case of a transmembrane domain). Such derivatives may be tested for efficacy, e.g., using the in vitro assays described herein.

By a "functional HIV envelope-binding derivative" is meant a functional derivative (as defined above) which is capable of binding any HIV envelope protein. Functional derivatives may be identified using, e.g., the in vitro assays described herein.

### THERAPEUTIC ADMINISTRATION

The transformed cells of the present invention may be used for the therapy of a number of diseases. Current methods of administering such transformed cells involve adoptive immunotherapy or cell-transfer therapy. These methods allow the return of the transformed immune-system cells to the bloodstream. Rosenberg, Sci. Am. 62 (May 1990); Rosenberg et al., New Engl. J. Med. 323:570 (1990).

The pharmaceutical compositions of the invention may be administered to any animal which may experience the beneficial effects of the compounds of the invention. Foremost among such animals are humans, although the invention is not intended to be so limited.

### Detailed Description

The drawings will first be described.

### Brief Description of the Drawings

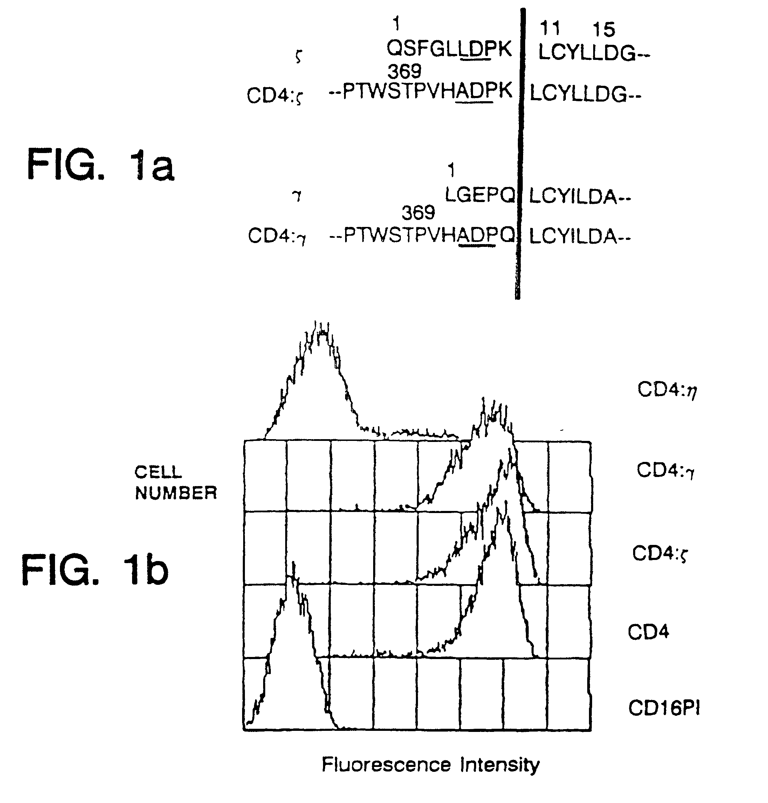

**FIG. 1A** presents the amino acid sequence about the site of fusion between CD4 (residues 1-369) and different receptor chains (SEQ ID NOS: 28-31). The underlined sequence shows the position of the amino acids encoded within the BamHI site used for fusion construction. The beginning of the transmembrane domain is marked with a vertical bar. The η sequence is identical to the ζ sequence at the amino terminus, but diverges at the carboxyl terminus (Jin et al., Proc. Natl. Acad. Sci. USA 87:3319-3323 (1990)). **FIG. 1B** presents flow cytometric analysis of surface expression of CD4, CD4:ζ, CD4:γ and CD4:η in CV1 cells. Cells were infected with virus expressing CD4 chimeras or CD16_{PI}, incubated for 9 hours at 37°C, and stained with phycoerythrin-conjugated anti-CD4 MAb Leu3A.

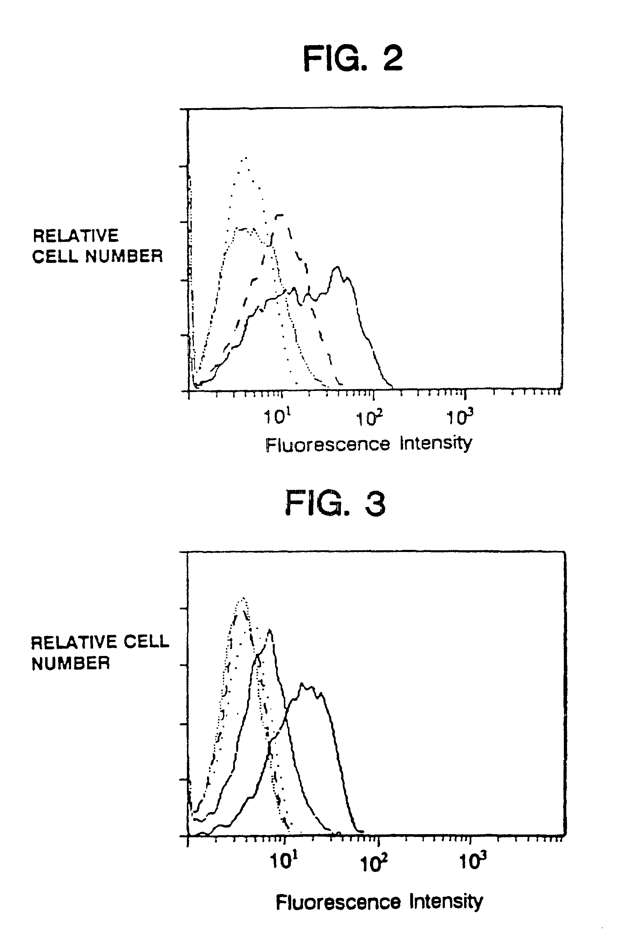

**FIG. 2** shows surface expression of CD16_{TM} following coinfection of CD16_{TM} alone (dense dots), or coinfected with virus expressing CD4:γ (dashes) or CD4:ζ (solid line). Sparse dots, cells infected with CD4:ζ alone, stained with 3G8 (Fleit et al., Proc. Natl. Acad. Sci. USA 79:3275-3279 (1982)) (anti-CD16 MAb).
**FIG. 3** shows surface expression of CD16_{TM} following coinfection by viruses expressing CD16_{TM} and the following ζ chimeras: CD4:ζ (thick line), CD4:ζ C11G (solid line); CD4:ζ (dashed line); CD4:ζ C11G/D15G (dense dots); no coinfection (CD16_{TM} alone, sparse dots). Cells were incubated with anti-CD16 MAb 3G8 and phycoerythrin-conjugated Fab'₂ goat antibodies to mouse IgG. The level of expression of the ζ chimeras was essentially identical for the different mutants analyzed, and coinfection of cells with viruses expressing CD16_{TM} and ζ chimeras did not appreciably alter surface expression of the chimeras.

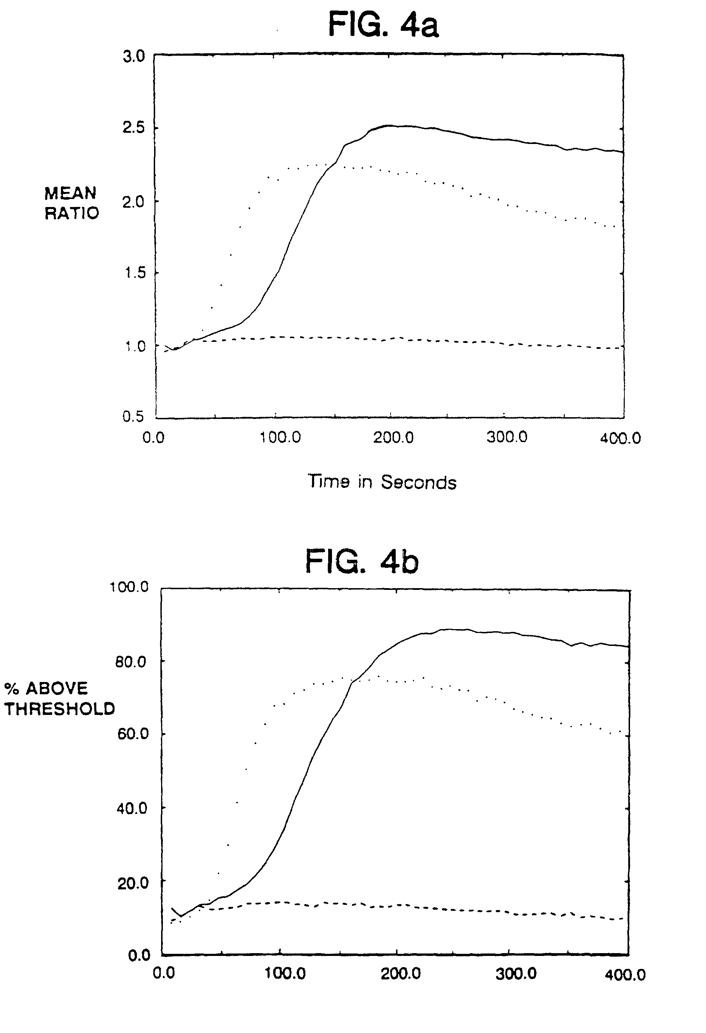

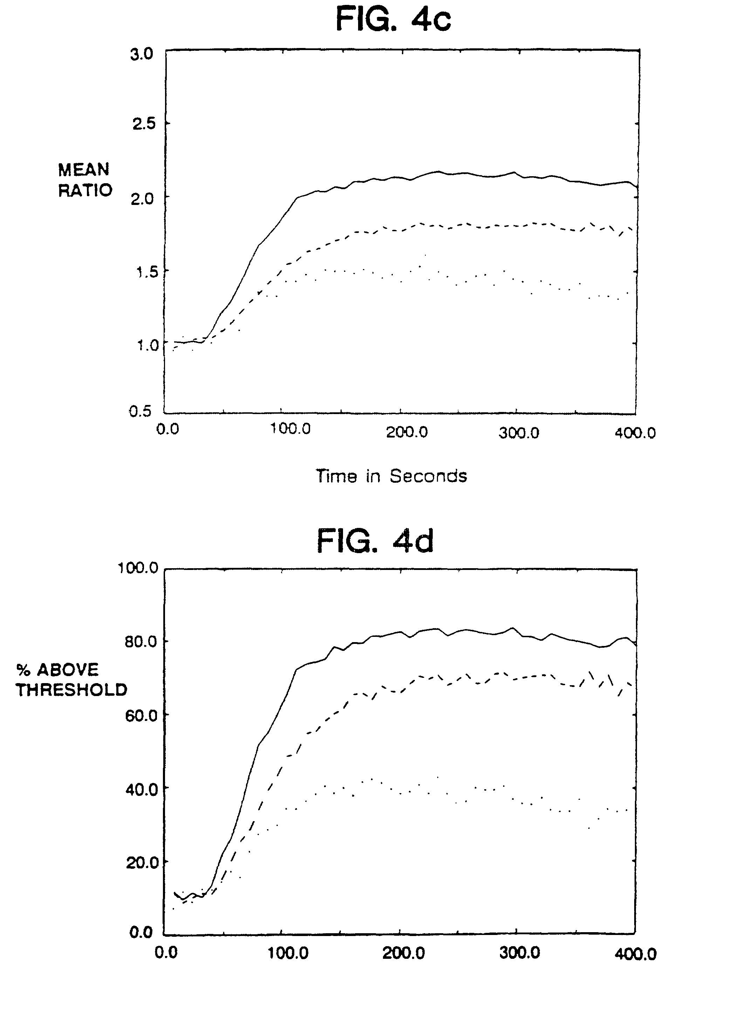

**FIG. 4A-D** shows increased intracellular free calcium ion follows crosslinking of mutant ζ chimeras in a T cell line. Jurkat E6 cells (Weiss et al., J. Immunol. 133:123-128 (1984)) were infected with recombinant vaccinia viruses and analyzed by flow cytometry. The results shown are for the gated CD₄⁺ population, so that only cells expressing the relevant chimeric protein are analyzed. The mean ratio of violet to blue Indo-1 fluorescence reflects the intracellular free calcium concentration in the population as a whole and the percentage of responding cells reflects the fraction of cells which exceed a predetermined threshold ratio (set so that 10% of untreated cells are positive). **FIG. 4A** and **FIG. 4B** show Jurkat cells expressing CD4:ζ (solid line) or CD16:ζ (dashed line) which were exposed to anti-CD4 MAb Leu3a (phycoerythrin conjugate), followed by crosslinking with goat antibody to mouse IgG. The dotted line shows the response of uninfected cells to anti-CD3 MAb OKT3. **FIGS. 4C** and **4D** show Jurkat cells expressing CD4:ζD15G (solid line); CD4:ζC11G/D15G (dashes); or CD4;ζC11G (dots) which were treated and analyzed as in FIGS. 4A and 4B.

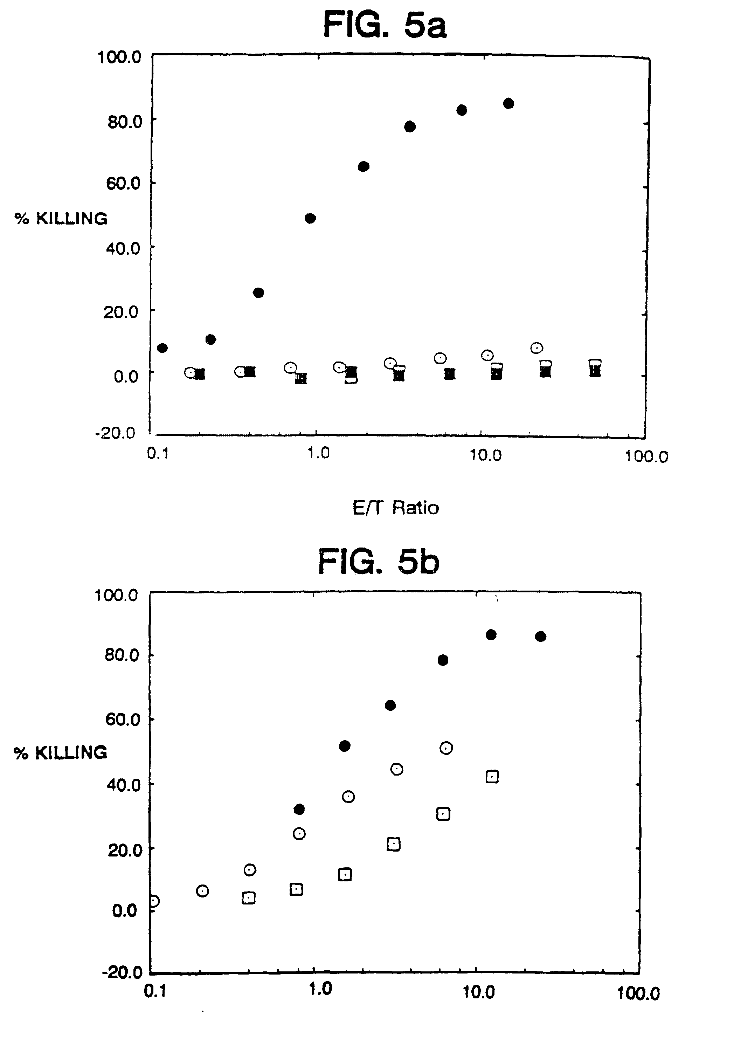

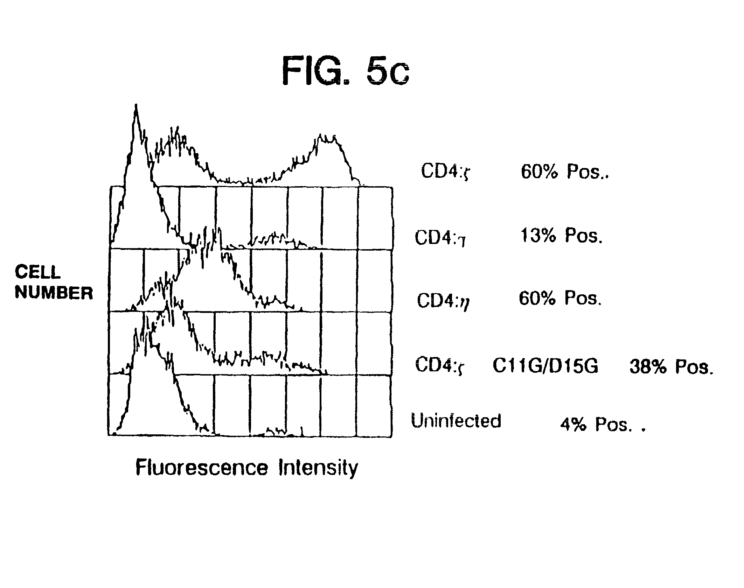

**FIG. 5A-C** shows that CD4:ζ, CD4:η, and CD4:γ receptors allow cytolytic T lymphocytes (CTL) to kill targets expressing HIV-1 gp120/41. **FIG. 5A:** solid circles, CTL expressing CD4:ζ incubated with HeLa cells expressing gp120/41; open circles, CTL expressing CD4:ζ incubated with uninfected HeLa cells; solid squares, uninfected CTL incubated with HeLa cells expressing gp120/41; open squares, uninfected CTL incubated with uninfected HeLa cells. **FIG. 5B:** solid circles, CTL expressing CD4:η incubated with HeLa cells expressing gp120/41; open circles, CTL expressing CD4:γ incubated with HeLa cells expressing gp120/41; open squares, CTL expressing the C11G/D15G double mutant CD4:ζ chimera incubated with HeLa cells expressing gp120/41. **FIG. 5C:** Flow cytometric analysis of CD4 expression by the CTL used in Fig. 5B. To correct the target to effector ratios the percent of cells expressing CD4 chimera was determined by subtracting the scaled negative (uninfected) population by histogram superposition; for comparative purposes in this figure the uninfected cells were assigned an arbitrary threshold which gives roughly the same fraction positive for the other cell populations as would histogram subtraction.

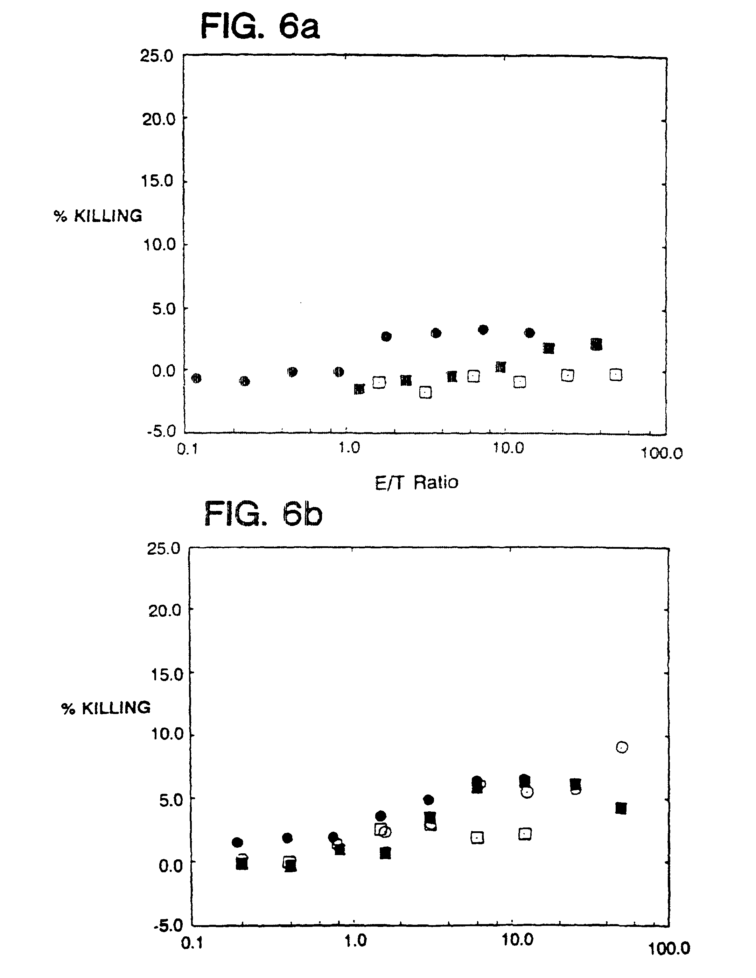

**FIG. 6A-B** shows specificity of CD4-directed cytolysis. **FIG. 6A:** solid circles, CTL expressing CD4:ζ incubated with HeLa cells expressing CD16_{P1}; open circles, CTL expressing CD4 incubated with HeLa cells expressing gp120; solid squares, CTL expressing CD16:ζ incubated with HeLa cells expressing gp120/41; open squares, CTL expressing CD16_{P1} incubated with HeLa cells expressing gp120/41. **FIG. 6B**: solid circles, CTL expressing CD4:ζ incubated with Raji (MHC class II⁺) cells; open circles, uninfected CTL cells incubated with RJ2.2.5 (MHC class II⁻ Raji mutant) cells; solid squares, uninfected CTL incubated with Raji (MHC class II⁺) cells; open squares, CTL expressing CD4:ζ incubated with RJ2.2.5 (MHC class II⁻) cells. The ordinate scale is expanded.

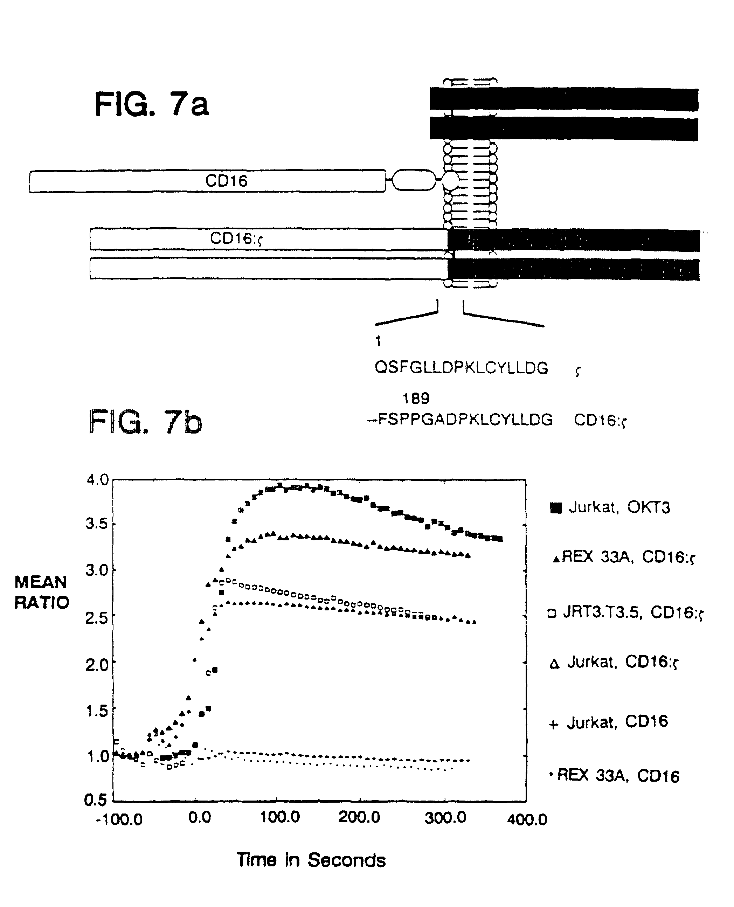

**FIG. 7A-B** shows characterization of the CD16:ζ chimeric receptor. **FIG. 7A** is a schematic diagram of the CD16:ζ fusion protein. The extracellular portion of the phosphatidylinositol-linked form of monomeric CD16 was joined to dimeric ζ just external to the transmembrane domain. The protein sequence at the fusion junction is shown at the bottom (SEQ ID NOS: 32, 33). **FIG. 7B** shows a flow cytometric analysis of calcium mobilization following crosslinking of the CD16:ζ chimera in either a TCR positive or TCR negative cell line. The mean ratio of violet to blue fluorescence (a measure of relative calcium ion concentration) among cell populations treated with antibodies at time 0 is shown. Solid squares, the response of Jurkat cells to anti-CD3 MAb OKT3; solid triangles, the response of CD16:ζ to anti-CD16 MAb 3G8 crosslinking in the REX33A TCR⁻ mutant; open squares, the response to CD16:ζ crosslinking in the Jurkat TCR⁻ mutant line JRT3.T3.5; open triangles, the response to CD16:ζ crosslinking in Jurkat cells; crosses, the response to nonchimeric CD16 in Jurkat cells; and dots, the response to nonchimeric CD16 in the REX33A TCR⁻ cell line.

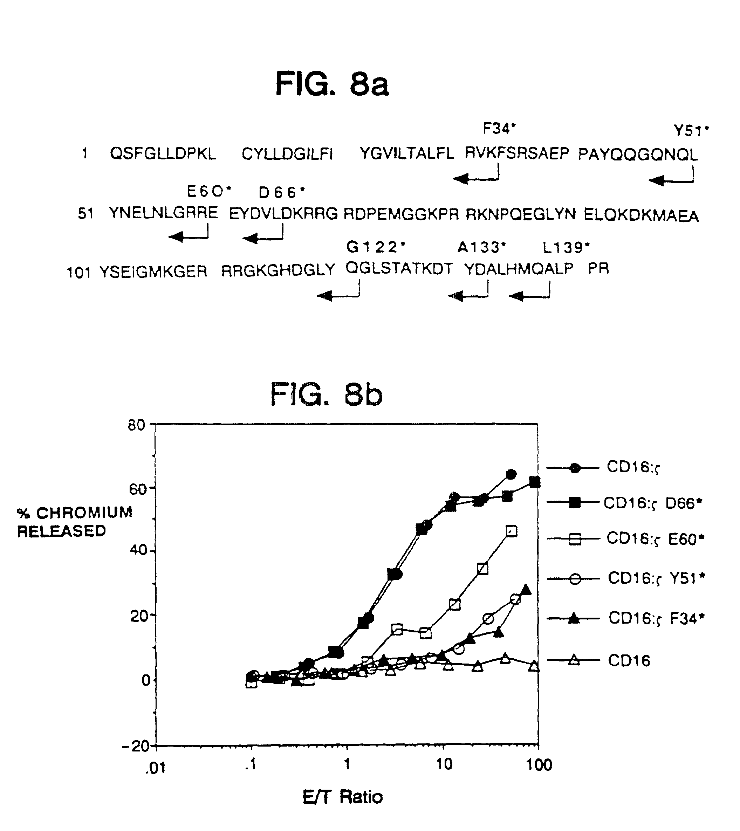

**FIG. 8A-B** shows deletion analysis of cytolytic potential. **FIG. 8A** shows the locations of the ζ deletion endpoints. Here as elsewhere mutations in ζ are represented by the original residue-location-mutant residue convention, so that D66*, for example, denotes replacement of Asp-66 by a termination codon. **FIG. 8B** shows cytolysis assay results of undeleted CD16:ζ and salient ζ deletions. Hybridoma cells expressing surface antibody to CD16 were loaded with ⁵¹Cr and incubated with increasing numbers of human cytolytic lymphocytes (CTL) infected with vaccinia recombinants expressing CD16:ζ chimeras. The percent of ⁵¹Cr released is plotted as a function of the effector (CTL) to target (hybridoma) cell ratio (e/t). Solid circles, cytolysis mediated by cells expressing CD16:ζ (mfi 18.7); solid squares, cytolysis mediated by cells expressing CD16:ζ Asp66* (mfi 940.2); open squares, cytolysis mediated by cells expressing CD16:ζGlu60* (mfi 16.0); open circles, cytolysis mediated by cells expressing CD16:ζTyr51* (mfi 17.4); solid triangles, cytolysis mediated by cells expressing CD16:ζPhe34* (mfi 17.8); and open triangles, cytolysis mediated by cells expressing nonchimeric CD16 (mfi 591). Although in this experiment the expression of CD16:ζAsp66* was not matched to that of the other fusion proteins, cytolysis by cells expressing CD16:ζ at equivalent levels in the same experiment gave results essentially identical to those shown by cells expressing CD16:ζAsp66.

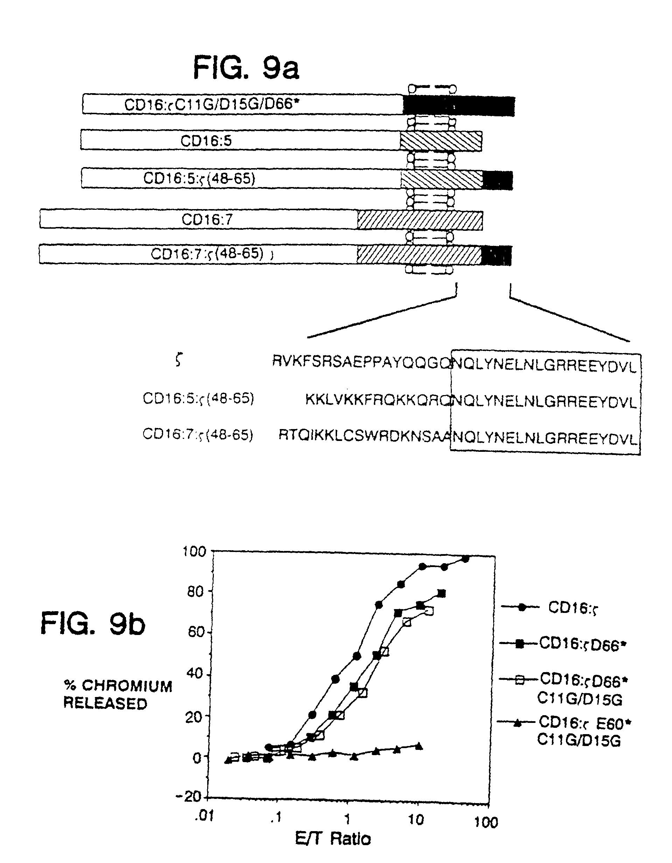

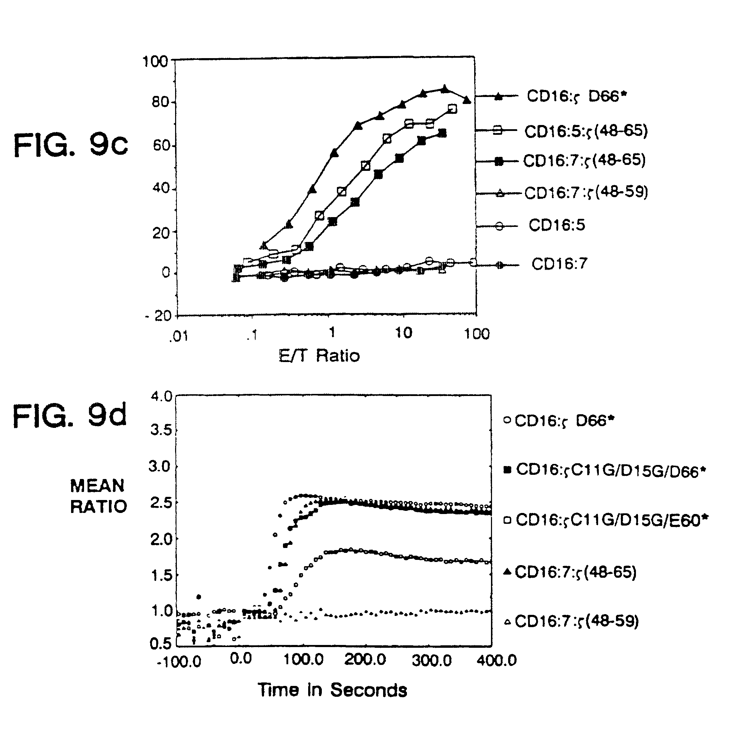

**FIG. 9A-D** shows that elimination of the potential for transmembrane interactions reveals a short ζ segment capable of mediating cytolysis. **FIG. 9A** is a schematic diagram of the monomeric bipartite and tripartite chimeras. At the top is the CD16:ζ construct truncated at residue 65 and lacking transmembrane Cys and Asp residues. Below are the CD16:CD5:ζ and CD16:CD7:ζ constructs and related controls. The peptide sequences of the intracellular domains are shown below (SEQ ID NOS: 35-37). **FIG. 9B** shows the cytolytic activity of monomeric chimera deletion mutants. The cytolytic activity of cells expressing CD16:ζ (solid circles; mfi 495) was compared to that of cells expressing CD16:ζAsp66* (solid squares; mfi 527) or the mutants CD16:ζCys11Gly/Asp15Gly/Asp66*, (open squares; mfi 338) and CD16:ζCys11Gly/Asp15Gly/Glu60* (filled triangles; mfi 259). **FIG. 9C** shows the cytolytic activity mediated by tripartite fusion proteins. Solid triangles, CD16:ζAsp66*; open squares, CD16:5:ζ(48-65); solid squares CD16:7:ζ(48-65); open triangles, CD16:7:ζ(48-59); open circles, CD16:5; solid circles, CD16:7. **FIG. 9D** shows calcium mobilization by mutant and tripartite chimeras in the TCR negative Jurkat JRT3.T3.5 mutant cell line. Open circles, response of cells expressing dimeric CD16:ζAsp66*; solid squares, response of cells expressing CD16:ζCys11Gly/Asp15Gly/Asp66*; open squares, response of cells expressing CD16:ζCys11Gly/Asp15Gly/Glu60*; solid triangles, response of cells expressing CD16:7:ζ(48-65); and open triangles, response of cells expressing CD16:ζ(48-59).

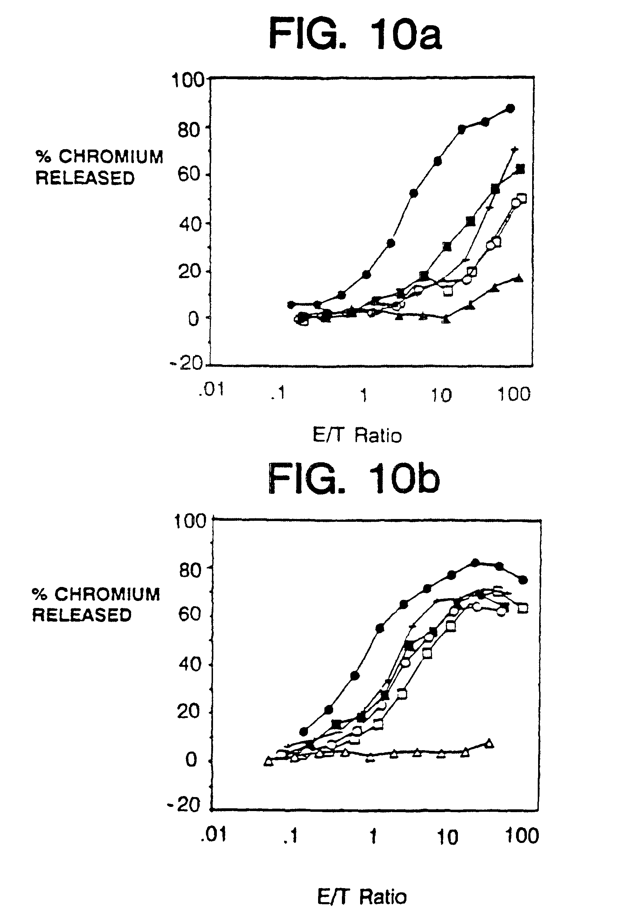

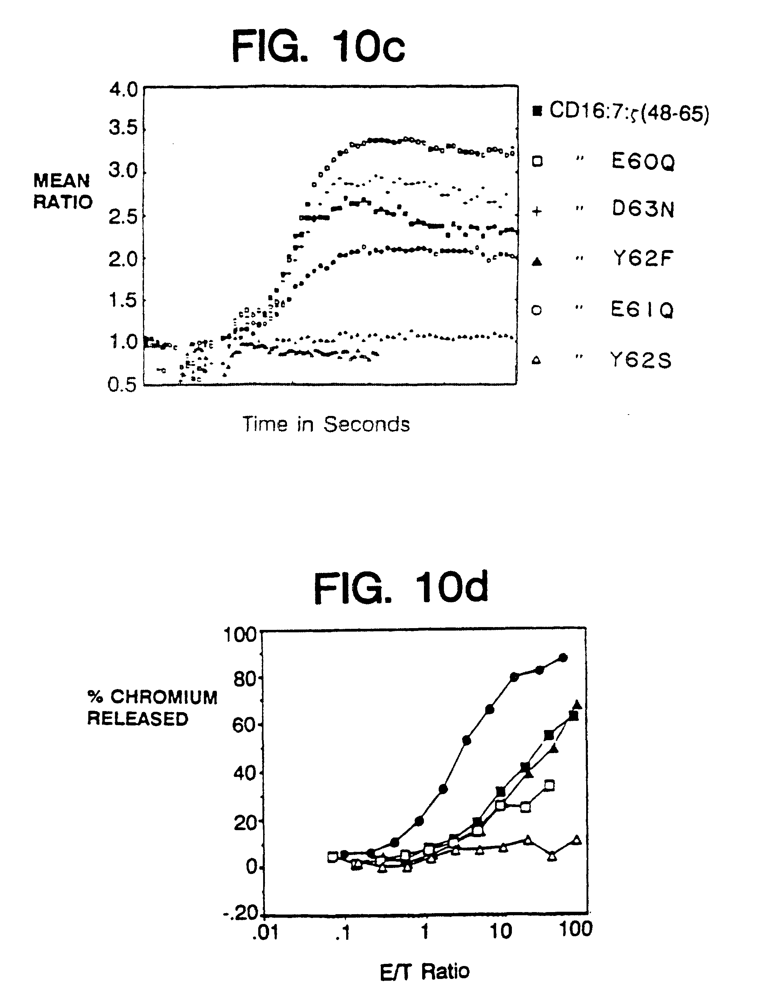

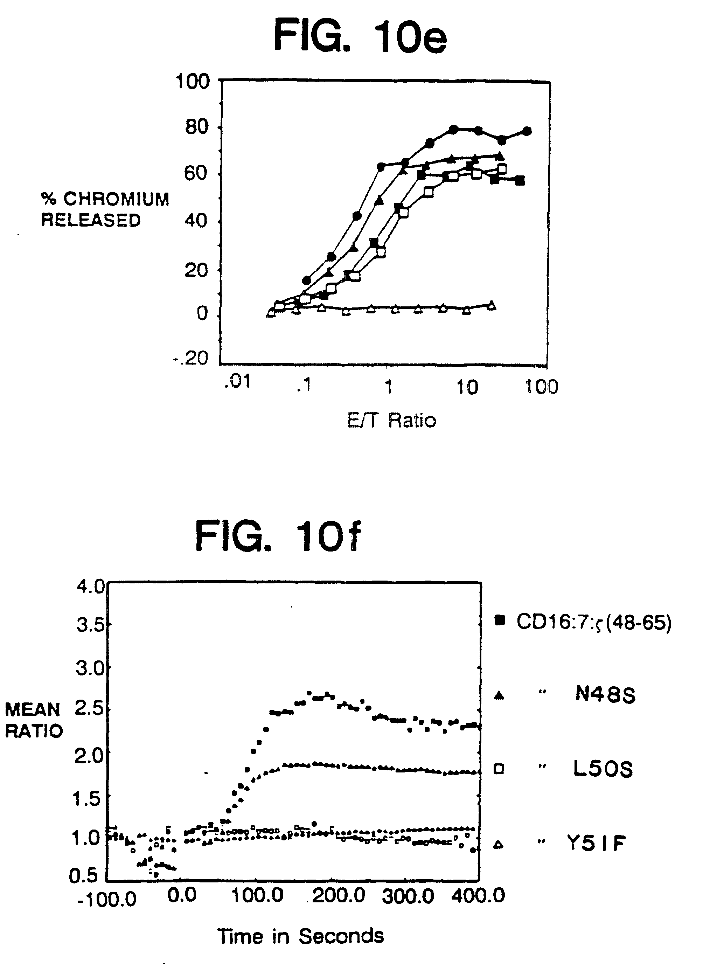

**FIG. 10A-F** shows the contribution of individual amino acids to the activity of the 18 residue cytolytic signal-transducing motif. **FIGS. 10A** and **10B** show cytolytic activity and **FIG. 10C** shows calcium ion mobilization mediated by chimeras bearing point mutations near the carboxyl terminal tyrosine (Y62). **FIGS. 10A** and **10B** represent data collected on cells expressing low and high amounts, respectively, of the CD16:ζ fusion proteins. Identical symbols are used for the calcium mobilization and cytolysis assays, and are shown in one letter code at right. Solid circles, cells expressing CD16:ζ (mfi in A, 21; B, 376); solid squares, cells expressing CD16:7:ζ(48-65) (mfi A, 31; B, 82); open squares, CD16:7:ζ(48-65)Glu60Gln (mfi A, 33; B, 92), crosses, CD16:7:ζ(48-65)Asp63Asn (mfi A, 30; B, 74); solid triangles, CD16:7:ζ(48-65)Tyr62Phe (mfi A, 24; B, 88); open circles, CD16:7:ζ(43-65)Glu61Gln (mfi A, 20; B, 62); and open triangles, CD16:7:ζ(48-65)Tyr62Ser (mfi B, 64). **FIGS. 10D** and **10E** show cytolytic activity and **FIG. 10F** shows calcium ion mobilization by chimeras bearing point mutations near the amino terminal tyrosine (Y51). Identical symbols are used for the calcium mobilization and cytolysis assays and are shown at right. Solid circles, cells expressing CD16:ζ (mfi in D, 21.2; in E, 672); solid squares, cells expressing CD16:7:ζ(48-65) (mfi D, 31.3; E, 179); solid triangles, CD16:7:ζ(48-65)Asn48Ser (mfi D, 22.4; E, 209); open squares, CD16:7:ζ(48-65)Leu50Ser (mfi D, 25.0; E, 142); and open triangles, CD16:7:ζ(48-65)Tyr51Phe (mfi D, 32.3; E, 294).

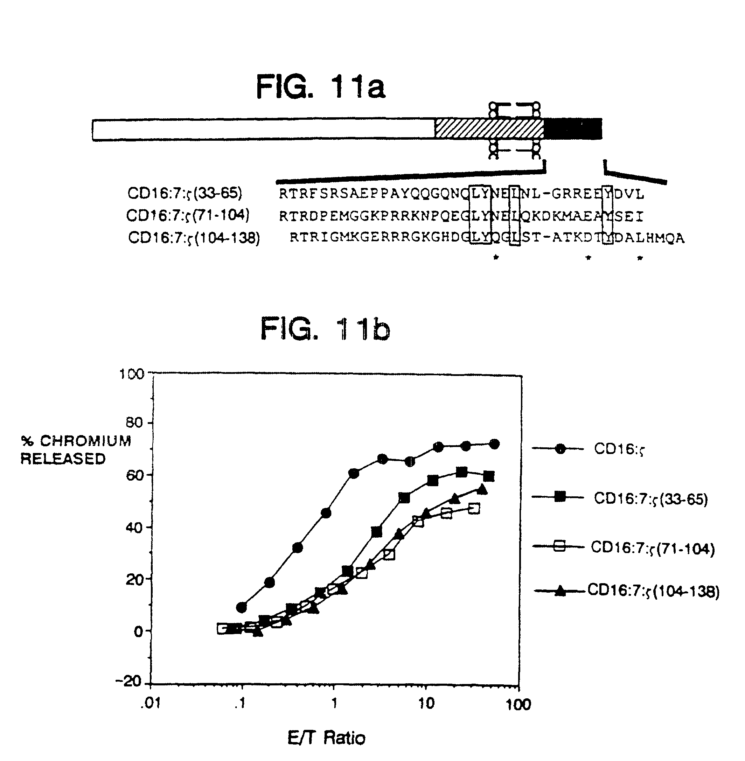

**FIG. 11A-B** shows alignment of internal repeats of ζ and comparison of their ability to support cytolysis. **FIG. 11A** is a schematic diagram of chimeras formed by dividing the ζ intracellular domain into thirds and appending them to the transmembrane domain of a CD16:7 chimera. The sequences of the intracellular domains are shown below (SEQ ID NOS: 38-40), with shared residues boxed, and related residues denoted by asterisks. **FIG. 11B** shows the cytolytic potency of the three ζ subdomains. Solid circles, cells expressing CD16:ζ (mfi 476); solid squares, CD16:7:ζ(33-65) (mfi 68); open squares, CD16:7:ζ(71-104) (mfi 114); and solid triangles, CD16:7:ζ(104-138) (mfi 104).

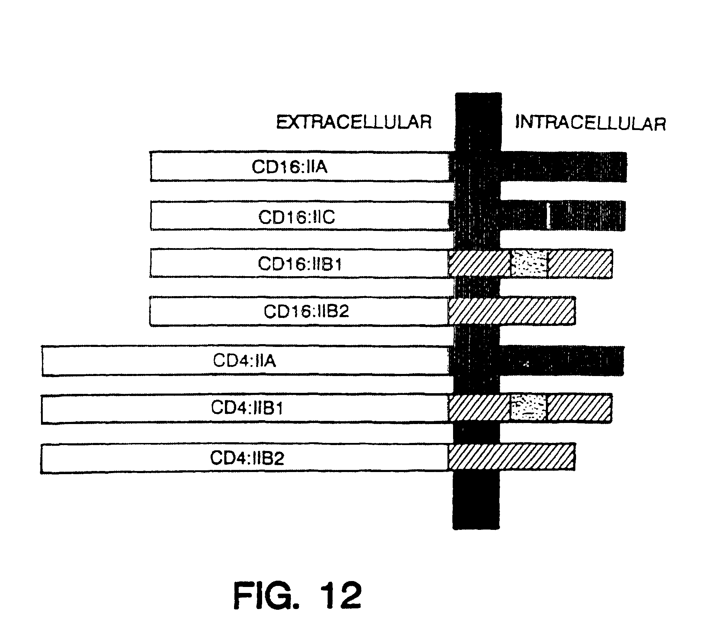

**FIG. 12** is a schematic diagram of the CD16:FcRγII chimeras.

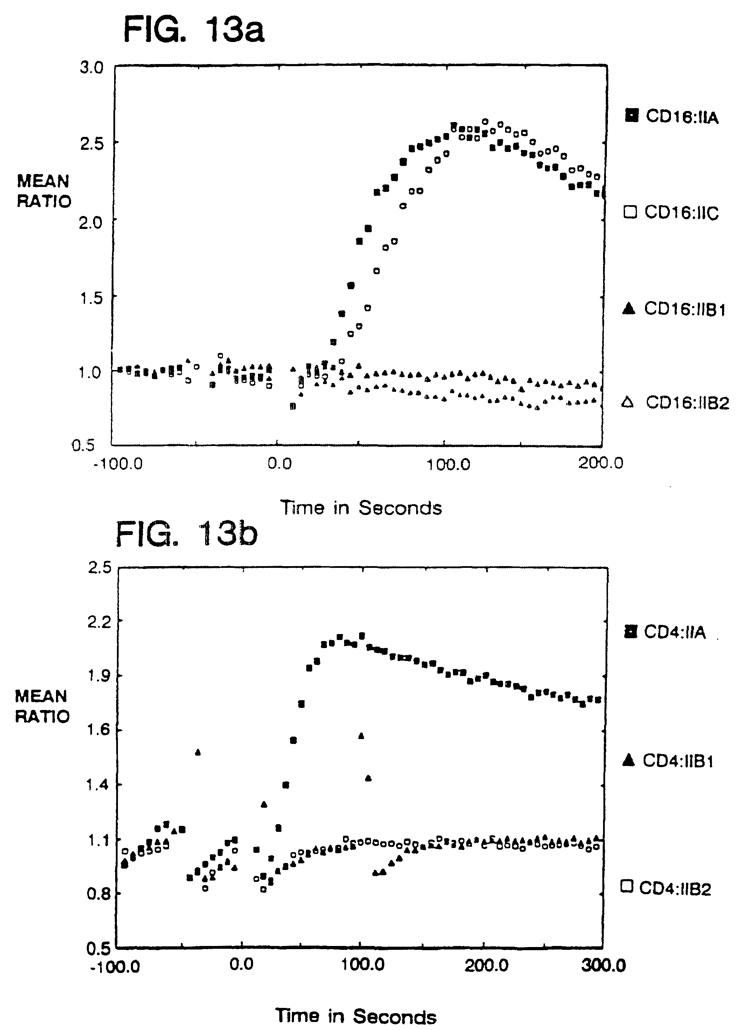

**FIG. 13A-B** shows calcium mobilization following crosslinking of CD4:FcRγII and CD16:FcRγII chimeras. **FIG. 13A** shows the ratio of violet to blue fluorescence emitted by cells loaded with the calcium sensitive fluorophore Indo-1 shown as a function of time following crosslinking of the CD16 extracellular domain with antibodies. **FIG. 13B** shows a similar analysis of the increase in ratio of violet to blue fluorescence of cells bearing CD4:FcRγII chimeras, following crosslinking with antibodies.

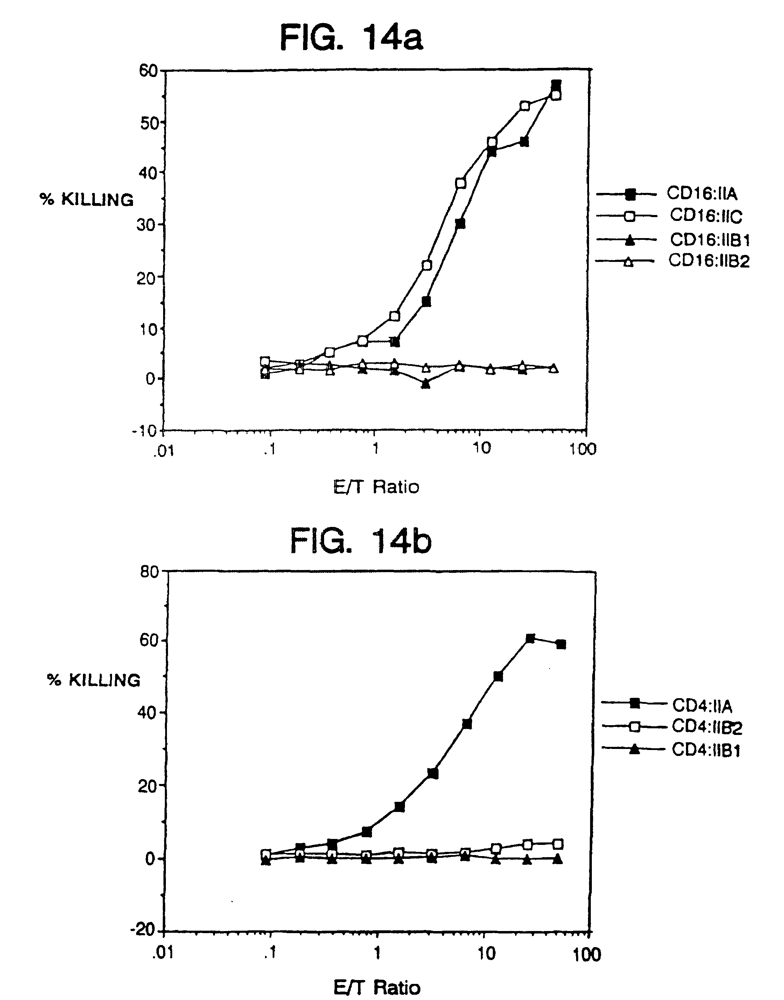

**FIG. 14A-B** shows cytolysis assays of CD4:FcRγII and CD16:FcRγII chimeras. **FIG. 14A** shows the percent of ⁵¹Cr released from anti-CD16 hybridoma (target) cells when the cells are exposed to increasing numbers of cytotoxic T lymphocytes expressing CD16:FcRγII chimeras (effector cells). **FIG. 14B** shows a similar analysis of cytotoxicity mediated by CD4:FcRγII chimeras against target cells expressing HIV envelope glycoproteins.

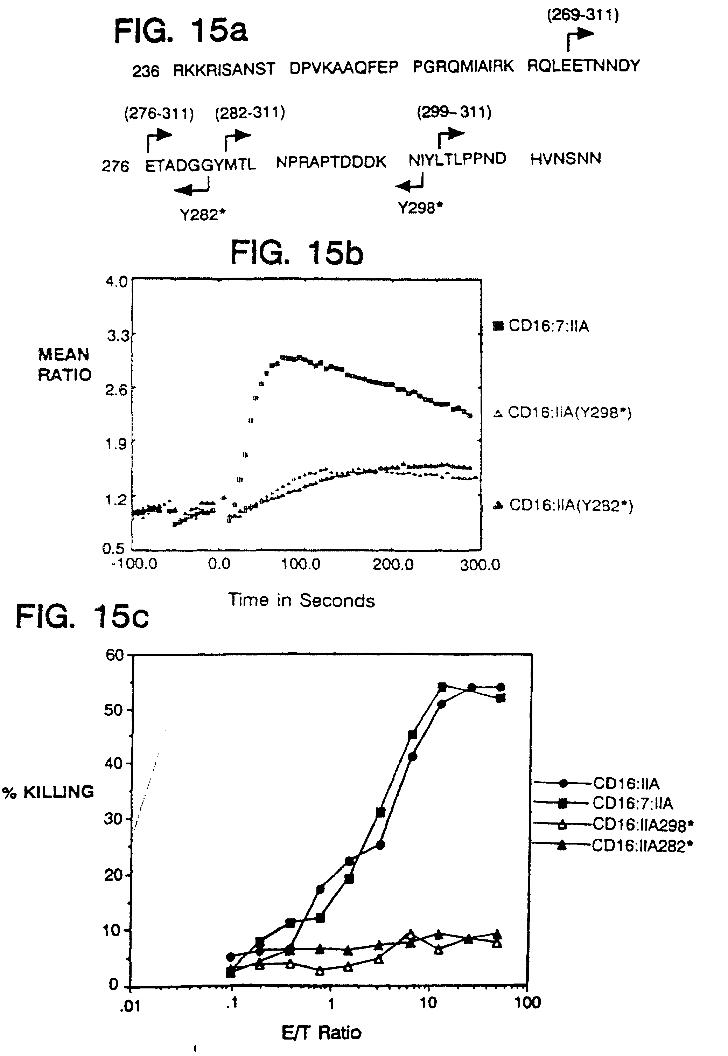

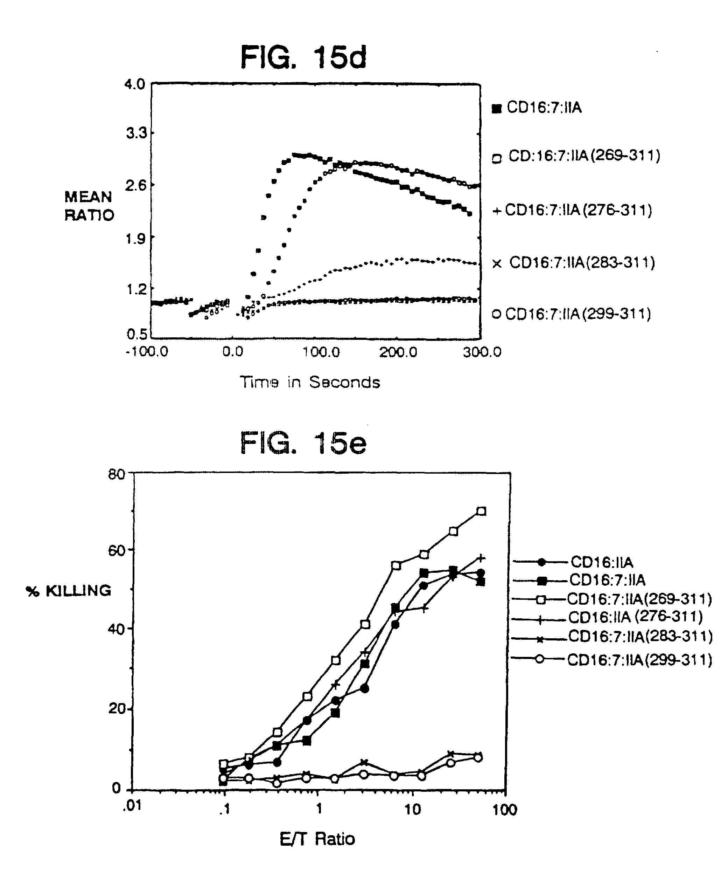

**FIG. 15A-E** shows identification of residues in the FcRγII A tail which are important for cytolysis. **FIG. 15A** is a schematic diagram of the deletion constructs. **FIGS. 15B** and **15C** shows calcium mobilization and cytolysis by carboxyl-terminal deletion variants of CD16:FcRγII A. **FIGS. 15D** and **15E** show calcium mobilization and cytolysis by tripartite chimeras bearing progressively less of the amino terminus of the intracellular tail of CD16:FcRγII A.

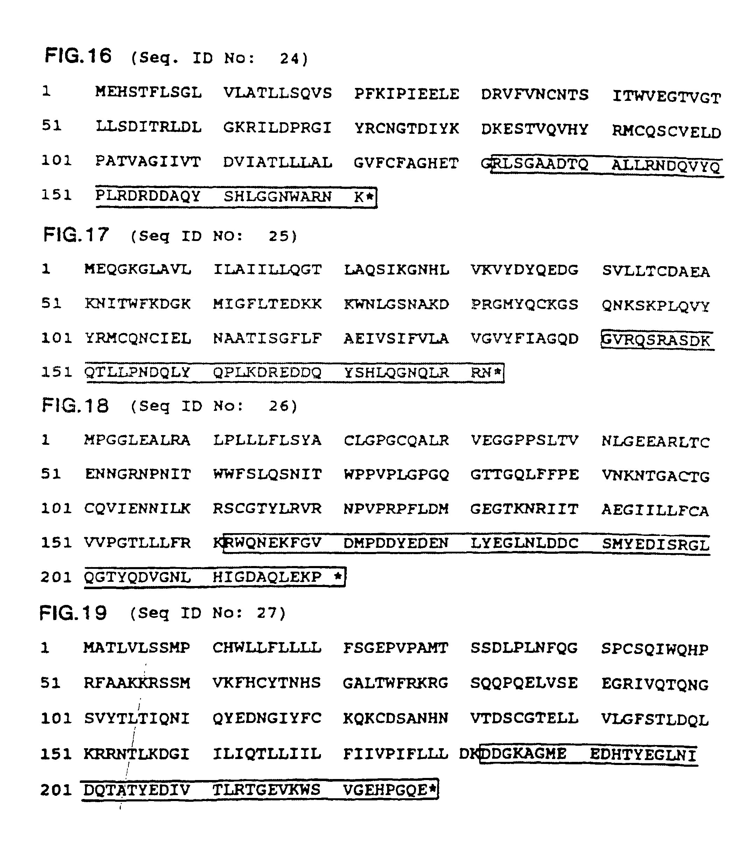

**FIG. 16** (SEQ ID NO: 24) shows the amino acid sequence of the CD3 delta receptor protein; the boxed sequence represents a preferred cytolytic signal transducing portion.
**FIG. 17** (SEQ ID NO: 25) shows the amino acid sequence of the T3 gamma receptor protein; the boxed sequence represents a preferred cytolytic signal transducing portion.
**FIG. 18** (SEQ ID NO: 26) shows the amino acid sequence of the mbl receptor protein; the boxed sequence represents a preferred cytolytic signal transducing portion.
**FIG. 19** (SEQ ID NO: 27) shows the amino acid sequence of the B29 receptor protein; the boxed sequence represents a preferred cytolytic signal transducing portion.

### EXAMPLE I

### Construction of Human IgG1:Receptor Chimeras

Human IgG1 heavy chain sequences were prepared by joining sequences in the C_{H}3 domain to a cDNA fragment derived from the 3' end of the transmembrane form of the antibody mRNA. The 3' end fragment was obtained by polymerase chain reaction using a tonsil cDNA library as substrate, and oligonucleotides having the sequences: corresponding to the 5' and 3' ends of the desired DNA fragments respectively. The 5' oligo is complementary to a site in the C_{H}1 domain of human IgG1, and the 3' oligo is complementary to a site just 5' of the sequences encoding the membrane spanning domain. The PCR product was digested with BstXI and BamHI and ligated between BstXI and BamHI sites of a semisynthetic IgG1 antibody gene bearing variable and constant regions. Following the insertion of the BstXI to BamHI fragment, the amplified portions of the construct were replaced up to the SmaI site in C_{H}3 by restriction fragment interchange, so that only the portion between the SmaI site and the 3' oligo was derived from the PCR reaction.

To create a human IgG1:ζ chimeric receptor, the heavy chain gene ending in a BamHI site was joined to the BamHI site of the ζ chimera described below, so that the antibody sequences formed the extracellular portion. Flow cytometry of COS cells transfected with a plasmid encoding the chimera showed high level expression of antibody determinants when an expression plasmid encoding a light chain cDNA was cotransfected, and modest expression of antibody determinants when the light chain expression plasmid was absent.

Similar chimeras including human IgG1 fused to η or γ (see below), or any signal-transducing portion of a T cell receptor or Fc receptor protein may be constructed generally as described above using standard techniques of molecular biology.

To create a single transcription unit which would allow both heavy and light chains to be expressed from a single promoter, a plasmid encoding a bicistronic mRNA was created from heavy and light chain coding sequences, and the 5' untranslated portion of the mRNA encoding the 78kD glucose regulated protein, otherwise known as grp78, or BiP. grp78 sequences were obtained by PCR of human genomic DNA using primers having the sequences: and at the 5' and 3' ends respectively. Polymerase chain reactions with these oligos were performed in the presence of 10% dimethyl sulfoxide. The fragment obtained by PCR was digested with NotI and HincII and inserted between NotI and HpaI sites downstream from human IgG1 coding sequences. Sequences encoding a human IgG kappa light chain cDNA were then inserted downstream from the grp78 leader, using the HincII site and another site in the vector. The expression plasmid resulting from these manipulations consisted of the semisynthetic heavy chain gene, followed by the grp78 leader sequences, followed by the kappa light chain cDNA sequences, followed by polyadenylation signals derived from an SV40 DNA fragment. Transfection of COS cells with the expression plasmid gave markedly improved expression of heavy chain determinants, compared to transfection of plasmid encoding heavy chain determinants alone.

To create a bicistronic gene comprising a heavy chain/receptor chimera and a light chain, the upstream heavy chain sequences can be replaced by any chimeric heavy chain/ receptor gene described herein.

### EXAMPLE II

### Construction of CD4 Receptor Chimeras

Human ζ (Weissman et al., Proc. Natl. Acad. Sci. USA 85:9709-9713 (1988b)) and γ (Küster et al., J. Biol. Chem. 265:6448-6452 (1990)) cDNAs were isolated by polymerase chain reaction from libraries prepared from the HPB-ALL tumor cell line (Aruffo et al., Proc. Natl. Acad. Sci. USA 84:8573-8577 (1987b)) and from human natural killer cells, while η cDNA (Jin et al., Proc. Natl. Acad. Sci. USA 87:3319-3323 (1990)) was isolated from a murine thymocyte library. ζ, η and γ cDNAs were joined to the extracellular domain of an engineered form of CD4 possessing a BamHI site just upstream of the membrane spanning domain (Aruffo et al., Proc. Natl. Acad. Sci. USA 84:8573-8577 (1987b); Zettlmeissl et al., DNA Cell Biol. 9347-353 (1990)) which was joined to the BamHI site naturally present in the ζ and η cDNAs at a similar location a few residues upstream of the membrane spanning domain (SEQ ID NOS: 1, 3, 4 and 6). To form the fusion protein with γ a BamHI site was engineered into the sequence at the same approximate location (Fig. 1; SEQ ID NO: 2 and 5). The gene fusions were introduced into a vaccinia virus expression plasmid bearing the E. coli gpt gene as a selectable marker, and inserted into the genome of the vaccinia WR strain by homologous recombination and selection for growth in mycophenolic acid (Falkner et al., J. Virol. 62:1849-1854 (1988); Boyle et al., Gene 65:123-128 (1988)). Flow cytometric analysis showed that the vaccinia recombinants direct the abundant production of CD4:ζ and CD4:γ fusion proteins at the cell surface, whereas the expression of CD4:η is substantially weaker (Fig. 1B). The latter finding is consistent with a recent report that transfection of an η cDNA expression plasmid into a murine hybridoma cell line gave substantially less expression than transfection of a comparable ζ expression plasmid (Clayton et al., J. Exp. Med. 172:1243-1253 (1990)). Immunoprecipitation of cells infected with the vaccinia recombinants revealed that the fusion proteins form covalent dimers, unlike the naturally occurring CD4 antigen. The molecular masses of the monomeric CD4:ζ and CD4:γ fusion proteins and native CD4 were found to be 63, 55 and 53 kD respectively. The larger masses of the fusion proteins are approximately consistent with the greater length of the intracellular portion, which exceeds that of native CD4 by 75 (CD4:ζ) or 5 (CD4:γ) residues.

### EXAMPLE III

### CD4 Chimeras Can Associate With Other Receptor Chains

Cell surface expression of the macrophage/natural killer cell form of human FcγRIII (CD16_{TM}) on transfectants is facilitated by cotransfection with murine (Kurosaki et al., Nature 342:805-807 (1989)) or human (Hibbs et al., Science 246:1608-1611 (1989)) γ, as well as by human ζ (Lanier et al., Nature 342:803-805 (1989)).

Consistent with these reports, expression of the chimeras also allowed surface expression of CD16_{TM} when delivered to the target cell either by cotransfection or by coinfection with recombinant vaccinia viruses (Fig. 2). The promotion of CD16_{TM} surface expression by ζ was more pronounced than promotion by γ (Fig. 2) in the cell lines examined, whereas native CD4 did not enhance CD16_{TM} surface expression.

### EXAMPLE IV

### Asp ζ Mutants Do Not Coassociate with Fc Receptor

To create chimeras which would not associate with existing antigen or Fc receptors, mutant ζ fusion proteins which lacked either the intramembranous Asp or intramembranous Cys residue or both were prepared. Flow cytometry showed that the intensity of cell surface expression by the different mutant chimeras was not appreciably different from the unmutated precursor, and immunoprecipitation experiments showed that total expression by the chimeras was similar. As expected, the mutant chimeras lacking the transmembrane cysteine residue were found not to form disulfide linked dimers. The two mutant chimeras lacking Asp were incapable of supporting the surface expression of CD16, whereas the monomeric chimeras lacking Cys but bearing Asp allowed CD16_{TM} to be coexpressed, but at lower efficiency than the parental dimer (Fig. 3).

### EXAMPLE V

### Mutant Receptors Retain the Ability to Initiate a Calcium Response

To determine whether crosslinking of the fusion proteins would allow the accumulation of free intracellular calcium in a manner similar to that known to occur with the T cell antigen receptor, cells of the human T cell leukemia line, Jurkat E6 (ATCC Accessior Number TIB 152, American Type Culture Collection, Rockville, MD), were infected with the vaccinia recombinants and the relative cytoplasmic calcium concentration following crosslinking of the extracellular domain with antibodies was measured. Flow_cytometric measurements were performed with cells loaded with the calcium sensitive dye Indo-1 (Grynkiewicz et al., J. Biol. Chem. 260:3340-3450 (1985); Rabinovitch et al., J. Immunol. 137:952-961 (1986)). Figure 4A-D shows the results of calcium flux experiments with cells infected with CD4:ζ and the Asp⁻ and Cys⁻ mutants of ζ. Crosslinking of the chimeras, reproducibly increased intracellular calcium. CD4:η and CD4:γ similarly allowed accumulation of intracellular calcium in infected cells. Jurkat cells express low levels of CD4 on the cell surface, however, crosslinking of the native CD4 in the presence or absence of CD16:ζ does not alter intracellular calcium levels (Fig. 4A-B).

### EXAMPLE VI

### CD4:ζ, η, and γ Chimeras Mediate Cytolysis of Targets Expressing HIV gp120/41

To determine whether the chimeric receptors would trigger cytolytic effector programs, a model target:effector system based on CD4 recognition of the HIV envelope gp120/gp41 complex was created. HeLa cells were infected with recombinant vaccinia viruses expressing gp120/gp41 (Chakrabarti et al., Nature 320:535-537 (1986); Earl et al., J. Virol. 64:2448-2451 (1990)) and labeled with ⁵¹Cr. The labeled cells were incubated with cells from a human allospecific (CD8⁺, CD4⁻) cytotoxic T lymphocyte line which had been infected with vaccinia recombinants expressing the CD4:ζ, CD4:η, or CD4:γ chimeras, or the CD4:ζCys11Gly:Asp15Gly double mutant chimera. Fig. 5A-C shows that HeLa cells expressing gp120/41 were specifically lysed by cytotoxic T lymphocytes (CTL) expressing CD4 chimeras. Uninfected HeLa cells were not targeted by CTL armed with CD4:ζ chimeras, and HeLa cells expressing gp120/41 were not recognized by uninfected CTL. To compare the efficacy of the various chimeras, the effector to target ratios were corrected for the fraction of CTL expressing CD4 chimeras, and for the fraction of HeLa cells expressing gp120/41, as measured by flow cytometry. Fig. 5C shows a cytometric analysis of CD4 expression by the CTL used in the cytolysis experiment shown in Figs. 5A and 58. Although the mean density of surface CD4:ζ greatly exceeded the mean density of CD4:η, the cytolytic efficiencies of cells expressing either form were similar. Correcting for the fraction of targets expressing gp120, the efficiency of cytolysis mediated by CD4:ζ and CD4:η proteins are comparable to the best efficiencies reported for specific T cell receptor target:effector pairs (the mean effector to target ratio for 50% release by T cells expressing CD4:ζ was 1.9 ± 0.99, n=10. The CD4:γ fusion was less active, as was the CD4:ζ fusion lacking the transmembrane Asp and Cys residues. However in both cases significant cytolysis was observed (Fig. 5B-C).

To control for the possibility that vaccinia infection might promote artefactual recognition by CTL, similar cytolysis experiments were performed with target cells infected with vaccinia recombinants expressing the phosphatidylinositol linked form of CD16 (CD16_{PI}) and labeled with ⁵¹Cr, and with CTL infected with control recombinants expressing either CD16_{PI} or CD16:ζ. Fig. 6A shows that T cells expressing non-CD4 chimeras do not recognize native HeLa cells or HeLa cells expressing gp120/41, and similarly that T cells expressing CD4 chimeras do not recognize HeLa cells expressing other vaccinia-encoded surface proteins. In addition, CTLs expressing non-chimeric CD4 do not significantly lyse HeLa cells expressing gp120/41 (Fig. 6A).

### EXAMPLE VII

### MHC Class II-Bearing Cells Are Not Targeted by the Chimeras

CD4 is thought to interact with a nonpolynorphic sequence expressed by MHC class II antigen (Gay et al., Nature 328:626-629 (1987); Sleckman et al., Nature 328:351-353 (1987)). Although a specific interaction between CD4 and class II antigen has never been documented with purified proteins, under certain conditions adhesion between cells expressing CD4 and cells expressing class II molecules can be demonstrated (Doyle et al., Nature 330:256-259 (1987); Clayton et al., J. Exp. Med. 172:1243-1253 (1990); Lamarre et al., Science 245:743-746 (1989)). Next examined was whether killing could be detected against cells bearing class II antigen. Fig. 68 shows that there is no specific cytolysis directed by CD4:ζ against the Raji B cell line, which expresses abundant class II antigen. Although a modest (≈5%) cytolysis is observed, a class II-negative mutant of Raji, RJ2.2.5, (Accolla, J. Exp. Med. 157:1053-1058 (1983)) shows a similar susceptibility, as do Raji cells incubated with uninfected T cells.

### EXAMPLE VIII

### Sequence Requirements for Induction of Cytolysis by the T Cell Antigen/Fc Receptor Zeta Chain

Although chimeras between CD4 and ζ can arm cytotoxic T lymphocytes (CTL) to kill target cells expressing HIV gp120, an alternative to CD4 was sought in order to unambiguously compare the properties of zeta chimeras introduced into human T cell lines. Such lines can express CD4, making it difficult to specifically define the relationship between the type or degree of calcium mobilization and the cytotoxic potential of the different chimeras. To circumvent this, chimeras were created between ζ and CD16 in which the extracellular domain of CD16 is attached to the transmembrane and intracellular sequences of ζ (Fig. 7A). The gene fusions were introduced into a vaccinia virus expression plasmid bearing the E. coli gpt gene as a selectable marker and inserted into the genome of the vaccinia WR strain by homologous recombination and selection for growth in mycophenolic acid (Falkner and Moss, J. Virol. 62:1849 (1988); Boyle and Coupar, Gene 65:123 (1988)).

T cell lines were infected with the vaccinia recombinants and the relative cytoplasmic free calcium ion concentration was measured following crosslinking of the extracellular domains with antibodies. Both spectrofluorimetric (bulk population) and flow cytometric (single cell) measurements were performed with cells loaded with the dye Indo-1 (Grynkiewicz et al., J. Biol. Chem. 260:3440 (1985); Rabinovitch et al., J. Immunol. 137:952 (1986)). Figure 7B shows an analysis of data collected from cells of the Jurkat human T cell leukemia line infected with vaccinia recombinants expressing CD16:ζ fusion protein. Crosslinking of the chimeras reproducibly increased intracellular calcium, while similar treatment of cells expressing nonchimeric CD16 had little or no effect. When the chimera was expressed in mutant cell lines lacking antigen receptor, either REX33A (Breitmeyer et al., J. Immunol. 138:726 (1987); Sancho et al., J. Biol. Chem 264:20760 (1989)) or Jurkat mutant JRT3.T3.5 (Weiss et al., J. Immunol. 135:123 (1984)), a strong response to CD16 antibody crosslinking was seen. Similar data have been collected on the REX20A (Breitmeyer et al., supra, 1987; Blumberg et al., J. Biol. Chem. 265:14036 (1990)) mutant cell line, and a CD3/Ti negative mutant of the Jurkat cell line established in this laboratory. Infection with recombinants expressing CD16:ζ did not restore the response to anti-CD3 antibody, showing that the fusion protein did not act by rescuing intracellular CD3 complex chains.

To evaluate the ability of the chimeras to redirect cell-mediated immunity, CTLs were infected with vaccinia recombinants expressing CD16 chimeras and used to specifically lyse hybridoma cells expressing membrane-bound anti-CD16 antibodies. This assay is an extension of a hybridoma cytotoxicity assay originally developed to analyze effector mechanisms of cells bearing Fc receptors (Graziano and Fanger, J. Immunol. 138:945, 1987; Graziano and Fanger, J. Immunol. 139:35-36, 1987; Shen et al., Mol. Immunol. 26:959, 1989; Fanger et al., Immunol. Today 10: 92, 1989). Fig. 8B shows that expression of CD16:ζ in cytotoxic T lymphocytes allows the armed CTL to kill 3G8 (anti-CD16; Fleit et al., Proc. Natl. Acad. Sci. USA 79:3275, 1982) hybridoma cells, whereas CTL expressing the phosphatidylinositol-linked form of CD16 are inactive. CTL armed with CD16:ζ also do not kill hybridoma cells expressing an irrelevant antibody.

To identify the minimal ζ sequences necessary for cytolysis, a series of deletion mutants were prepared in which successively more of the ζ intracellular domain was removed from the carboxyl terminus (Fig. 8A). Most of the intracellular domain of zeta could be removed with little consequence for cytolytic potential; the full length chimera CD16:ζ was essentially equal in efficacy to the chimera deleted to residue 65, CD16:ζAsp66* (Fig. 8B). A substantial decrease in cytotoxicity was observed on deletion to ζ residue 59 (chimera CD16:ζGlu60*), and further deletion to residue 50 resulted in slightly less activity. However complete loss of activity was not observed even when the intracellular domain was reduced to a three residue transmembrane anchor (Fig. 8B).

Because ζ is a disulfide linked dimer, one explanation for the retention of cytolytic activity was that endogenous ζ was forming heterodimers with the chimeric ζ deletion, thereby reconstituting activity. To test this idea, ζ residues 11 and 15 were changed from Asp and Cys respectively to Gly (Cys11Gly/Asp15Gly), and immunoprecipitations were carried out as follows. Approximately 2 x 10⁶ CV1 cells were infected for one hour in serum free DME medium with recombinant vaccinia at a multiplicity of infection (moi) of at least ten. Six to eight hours post-infection, the cells were detached from the plates with PBS/1mM EDTA and surface labeled with 0.2 mCi ¹²⁵I per 2 x 10⁶ cells using lactoperoxidase and H₂O₂ by the method of Clark and Einfeld (*Leukocyte Typing II*, pp 155-167, Springer-Verlag, NY, 1986). The labeled cells were collected by centrifugation and lysed in 1% NP-40, 0.1% SDS, 0.15M NaCl, 0.05M Tris, pH 8.0, 5mM MgCl₂, 5mM KCl, 0.2M iodoacetamide and 1mM PMSF. Nuclei were removed by centrifugation, and CD16 proteins were immunoprecipitated with antibody 3G8 (Fleit et al., supra, 1982; Medarex) and anti-mouse IgG agarose (Cappel, Durham, NC). Samples were electrophoresed through an 8% polyacrylamide/SDS gel under non-reducing conditions or through a 10% gel under reducing conditions. These immunoprecipitations confirmed that the CD16:ζCys11Gly/Asp15Gly chimera did not associate in disulfide-linked dimer structures.

The cytolytic activity of the mutant receptors was also tested. The mutated chimera deleted to residue 65 (CD16:ζCys11Gly/Asp15Gly/Asp66*) was, depending on the conditions of assay, two to eight fold less active in the cytolysis assay than the comparable unmutated chimera (CD16:ζAsp66*), which was usually within a factor of two of, or indistinguishable in activity from, CD16:ζ (Fig. 9B). The reduction in activity of the mutant chimeras is comparable to the reduction seen with CD4 chimeras of similar structure (see above) and is most likely attributable to the lower efficiency of ζ monomers compared to dimers. In contrast, the Asp⁻, Cys⁻ mutated chimera deleted to residue 59 had no cytolytic activity (Fig. 9B), supporting the hypothesis that association with other chains mediated by the transmembrane Cys and/or Asp residues was responsible for the weak persistence of cytolytic activity in deletions more amino terminal than residue 65.

Flow cytometric studies showed that the deletion mutants lacking transmembrane Asp and Cys residues could still promote an increase in free intracellular calcium ion in response to antibody crosslinking in a TCR⁻ mutant Jurkat cell line (Fig. 9D). Similar results were obtained for chimeras expressed in the parental Jurkat line. In the case of CD16:ζCys11Gly/Asp15Gly/Glu60*, these data demonstrate that the ability to mediate calcium responsiveness can be mutationally separated from the ability to support cytolysis.

To definitively eliminate the possible contribution of ζ transmembrane residues, the transmembrane and first 17 cytoplasmic residues of ζ were replaced by sequences encoding the membrane spanning and first 14 or first 17 cytoplasmic residues of the CD5 or CD7 antigens, respectively (Fig. 9A). The resulting tripartite fusion proteins CD16:5:ζ(48-65) and CD16:7:ζ(48-65) did not form disulfide-linked dimers as do the simpler CD16:ζ chimeras, because they lacked the cysteine residue in the ζ transmembrane domain. Both tripartite chimeras were able to mobilize calcium in Jurkat and TCR negative cell lines (Fig. 9D) and to mount a cytolytic response in CTL (Fig. 9C and data not shown). However truncation of the ζ portion to residue 59 in chimera CD16:7:ζ(48-59) abrogates the ability of tripartite fusion to direct calcium responsiveness in TCR positive or negative Jurkat cells or cytolysis in mature CTL (Fig. 9C and 9D and data not shown).

To examine the contributions of individual residues within the 18-residue motif, we prepared a number of mutant variants by site-directed mutagenesis, and evaluated their ability to mediate receptor-directed killing under conditions of low (Figs. 10A and 10D) or high (Figs. 10B and 10E) expression of chimeric receptor. Fig. 10A-F shows that while a number of relatively conservative substitutions (i.e., replacing acidic residues with their cognate amides, or tyrosine with phenylalanine) which spanned residues 59 to 63 yielded moderate compromise of cytolytic efficacy, in general the variants retained the ability to mobilize calcium. However collectively these residues comprise an important submotif inasmuch as their deletion eliminates cytolytic activity. Conversion of Tyr 62 to either Phe or Ser eliminated both the cytotoxic and calcium responses. At the amino terminus of the 18 residue segment, replacement of Tyr 51 with Phe abolished both calcium mobilization and cytolytic activity, while substitution of Leu with Ser at position 50 eliminated the calcium response while only partially impairing cytolysis. Without being bound to a particular hypothesis, it is suspected that the inability of the Leu50Ser mutant to mobilize calcium in short term flow cytometric assays does not fully reflect its ability to mediate a substantial increase in free intracellular calcium ion over the longer time span of the cytolysis assay. However, calcium-insensitive cytolytic activity has been reported for some cytolytic T cell lines, and the possibility that a similar phenomenon underlies the results described herein has not been ruled out. Replacement of Asn48 with Ser partially impaired cytotoxicity in some experiments while having little effect in others.

To investigate the potential role of redundant sequence elements, the intracellular domain of ζ was divided into three segments, spanning residues 33 to 65, 71 to 104, and 104 to 138. Each of these segments was attached to a CD16:CD7 chimera by means of a MluI site introduced just distal to the basic membrane anchoring sequences of the intracellular domain of CD7 (see below; Fig. 11A). Comparison of the cytolytic efficacy of the three elements showed they were essentially equipotent (Fig. 11B). Sequence comparison (Fig. 11A) shows that the second motif bears eleven residues between tyrosines, whereas the first and third motifs bear ten.

Although a precise accounting of the process of T cell activation has not been made, it is clear that aggregation of the antigen receptor, or of receptor chimeras which bear ζ intracellular sequences, triggers calcium mobilization, cytokine and granule release, and the appearance of cell surface markers of activation. The active site of ζ, a short linear peptide sequence probably too small to have inherent enzymatic activity, likely interacts with one or at most a few proteins to mediate cellular activation. It is also clear that mobilization of free calcium is not by itself sufficient for cellular activation, as the ability to mediate cytolysis can be mutationally separated from the ability to mediate calcium accumulation.

As shown herein, addition of 18 residues from the intracellular domain of ζ to the transmembrane and intracellular domain of two unrelated proteins allows the resulting chimeras to redirect cytolytic activity against target cells which bind to the extracellular portion of the fusion proteins. Although chimeras bearing the 18 residue motif are approximately eight-fold less active than chimeras based on full length ζ, the reduced activity can be attributed to the loss of transmembrane interactions which normally allow wild-type ζ to form disulfide linked dimers. That is, ζ deletion constructs which have the same carboxyl terminus as the motif and lack transmembrane Cys and Asp residues typically show slightly less activity than chimeras bearing only the 18 residue motif.

The cytolytic competency element on which we have focused has two tyrosines and no serines or threonines, restricting the possible contributions of phosphorylation to activity. Mutation of either tyrosine destroys activity, however, and although preliminary experiments do not point to a substantial tyrosine phosphorylation following crosslinking of chimeric surface antigens bearing the 18 reside motif, the possible participation of such phosphorylation at a low level cannot be excluded. In addition to the effects noted at the two tyrosine residues, a number of amino acid replacements at the amino and carboxyl terminus of the motif weaken activity under conditions of low receptor density.

Sequences similar to the ζ active motif can be found in the cytoplasmic domains of several other transmembrane proteins, including the CD3 δ and γ molecules, the surface IgM associated proteins mb1 and B29, and the β and γ chains of the high affinity IgE receptor, FcεRI (Reth, Nature 338:383, 1989). Although the function of these sequences is uncertain, if efficiently expressed, each may be capable of autonomous T cell activation, and such activity may explain the residual TCR responsiveness seen in a zeta-negative mutant cell line (Sussman et al., Cell 52:85, 1988). ζ itself bears three such sequences, approximately equally spaced, and a rough trisection of the intracellular domain shows that each is capable of initiating a cytolytic response. η, a splice isoform of ζ (Jin et al., supra, 1990; Clayton et al., Proc. Natl. saveAcad. Sci. USA 88:5202, 1991), lacks the carboxyl half of the third motif. Because removal of the carboxyl half of the first motif abolishes activity, it appears likely that the majority of the biological effectiveness of η can be attributed to the first two motifs. Although by different measures η is equally as active as ζ in promoting antigen-mediated cytokine release (Bauer et al., Proc. Natl. Acad. Sci. USA 88:3842, 1991) or redirected cytolysis (see above), η is not phosphorylated in response to receptor stimulation (Bauer et al., supra, 1991). Thus either the presence of all three motifs is required for phosphorylation, or the third motif represents a favored substrate for an unidentified tyrosine kinase.

### EXAMPLE IX

### Cytolytic Signal Transduction by Human Fc Receptor

To evaluate the actions of different human Fc receptor subtypes, chimeric molecules were created in which the extracellular domain of the human CD4, CD5 or CD16 antigens were joined to the transmembrane and intracellular domains of the FcRIIγA, B1, B2, and C subtypes (nomenclature of Ravetch and Kinet, Ann. Rev. Immunol. 9:457, 1991). Specifically, cDNA sequences corresponding to the transmembrane and cytoplasmic domains of the previously described FcRIIA, B1, and B2 isoforms were amplified from the preexisting clone PC23 or from a human tonsil cDNA library (constructed by standard techniques) using the following synthetic oligonucleotides primers: FcRII A reverse); FcRII B1 and FcRII B2 forward); These primers contained cleavage sites for the enzymes BamHI and NotI, respectively, indented 6 residues from the 5' end. The NotI site was immediately followed by an antisense stop codon, either CTA or TTA. All primers contained 18 or more residues complementary to the 5' and 3' ends of the desired fragments. The cDNA fragment corresponding to the FcRIIγC cytoplasmic domain, which differs from the IIA isoform in only one amino acid residue (L for P at residue 268) was generated by site directed mutagenesis by overlap PCR using primers of sequence: and

The PCR fragments were inserted into vaccinia virus expression vectors which contained the CD16 or CD4 extracellular domains respectively and subsequently inserted into wild type vaccinia by recombination at the thymidine kinase locus, using selection for cointegration of E coli gpt to facilitate identification of the desired recombinants. The identities of all isoforms (shown in Fig. 12) were confirmed by dideoxy sequencing.

Production of the chimeric receptor proteins was further confirmed by immunoprecipitation studies. Approximately 10⁷ JRT3.T3.5 cells were infected for one hour in serum free IMDM medium with recombinant vaccinia at a multiplicity of infection of at least ten. Twelve hours post-infection, the cells were harvested and surface labeled with 0.5mCi ¹²⁵I per 10⁷ cells using the lactoperoxidase/glucose oxidase method (Clark and Einfeld, supra). The labeled cells were collected by centrifugation and lysed 1% NP-40, 0.1mM MgCl₂, 5mM KCl, 0.2M iodoacetamide and 1mM PMSF. Nuclei were removed by centrifugation, and CD16 fusion proteins immunoprecipitated with antibody 4G8 and anti-mouse IgG agarose. Samples were electrophoresed under reducing conditions. All immunoprecipitated chimeric receptor molecules were of the expected molecular masses.

To test the ability of the chimeric receptors to mediate an increase in cytoplasmic free calcium ion, the recombinant viruses were used to infect the TCR⁻ mutant Jurkat cell line JRT3.T3.5 (as described herein) and cytoplasmic free calcium was measured in the cells (as described herein) following crosslinking of the receptor extracellular domains with monoclonal antibody 3G8 or Leu-3A (as described herein). These experiments revealed that the intracellular domains of FcRγII A and C were capable of mediating an increase in cytoplasmic free calcium ion after crosslinking of the extracellular domains, whereas the intracellular domains of FcRγII B1 and B2 were inactive under comparable conditions (Fig. 13 A and 13B). The CD4, CD5 and CD16 hybrids of FcRγII A shared essentially equal capacity to promote the calcium response (Fig. 13A-B). Other cell lines, from both monocytic and lymphocytic lineages, were capable of responding to the signal initiated by crosslinking of the extracellular domains.

To explore the involvement of the different FcRγII intracellular domains in cytolysis, human cytotoxic T lymphocytes (CTL) were infected with vaccinia recombinants expressing CD16:FcRγII A, B1, B2 and C chimeras. The infected cells were then cocultured with ⁵¹Cr-loaded hybridoma cells (i.e., 3G8 10-2 cells) which expressed cell surface antibody to CD16. In this assay CTLs bearing the CD16 chimera killed the hybridoma target cells (allowing release of free ⁵¹Cr) if the CD16 extracellular domain of the chimera has been joined to an intracellular segment capable of activating the lymphocyte effector program; this cytolysis assay is described in detail below. Fig. 14A shows that CTL armed with CD16:FcRγIIA and C, but not FcRγII B1 or B2, are capable of lysing target cells expressing cell surface anti-CD16 antibody.

To eliminate the possibility that the specific cytolysis was in some way attributable to interaction with the CD16 moiety, cytolysis experiments were conducted in which the FcRII intracellular domains were attached to a CD4 extracellular domain. In this case the target cells were HeLa cells expressing HIV envelope gp120/41 proteins (specifically, HeLa cells infected with the vaccinia vector vPE16 (available from the National Institute of Allergy and Infections Disease AIDS Depository, Bethesda, MD). As in the CD16 system, target cells expressing HIV envelope were susceptible to lysis by T cells expressing the CD4:FcRγII A chimera, but not FcRγII B1 or B2 (Fig. 14B).

The intracellular domains of FcRγII A and C share no appreciable sequence homology with any other protein, including the members of the extended FcRγ/TCRζ family. To define the sequence elements responsible for induction of cytolysis, 5' and 3' deletions of the intracellular domain coding sequences (described below and shown in Fig. 15A) were prepared and were evaluated for efficacy in calcium mobilization and cytolysis assays (as described herein). In the experiments in which the amino terminal portion of the intracellular domain was removed, the transmembrane domain of FcRγII was replaced with the transmembrane domain of the unrelated CD7 antigen to eliminate the possible contribution of interactions mediated by the membrane-spanning domain.

Figs. 15B and 15C show that removal of the 14 carboxyl-terminal residues, including tyrosine 298, resulted in a complete loss of cytolytic capacity and a substantial reduction in calcium mobilization potential. Further deletion to just before tyrosine 282 gave an identical phenotype (Figs. 15B and 15C). Deletion from the N-terminus of the intracellular domain to residue 268 had no substantial effect on either calcium profile or cytolytic potency, whereas deletion to residue 275 markedly impaired free calcium release but had little effect on cytolysis (Figs. 15D and 15E). Further deletion, to residue 282, gave FcRγII tails which lacked the ability to either mobilize calcium or trigger cytolysis (Figs. 15D and 15E). The 'active element' defined by these crude measures is relatively large (36 amino acids) and contains two tyrosines separated by 16 residues.

### EXAMPLE X

### Additional T Cell Receptor and B Cell Receptor Trigger Proteins

Other intracellular and transmembrane signal transducing domains according to the invention may be derived from the T cell receptor proteins, CD3 delta and T3 gamma, and the B cell receptor proteins, mb1 and 829. The amino acid sequences of these proteins are shown in Fig. 16 (CD3 delta; SEQ ID NO: 24), Fig. 17 (T3 gamma; SEQ ID NO: 25), Fig. 18 (mbl; SEQ ID NO: 26), and Fig. 19 (B29; SEQ ID NO: 27). The portions of the sequences sufficient for cytolytic signal transduction (and therefore preferably included in a chimeric receptor of the invention) are shown in brackets. Chimeric receptors which include these protein domains are constructed and used in the therapeutic methods of the invention generally as described above.

### EXAMPLE XI

### CD28 Chimeric Receptors

Because T cell activation has been shown to be augmented by engagement of CD28, the invention also includes therapeutic cells expressing pairs of chimeric receptors: the first chimera includes the intracellular domain of CD28, and the second chimera includes any intracellular or transmembrane signal-transducing domain described herein. In a given pair of chimeric receptors, the extracellular domains may be identical (for example, both may be derived from the CD4 protein and hence both recognize HIV or an HIV-infected cell), or each may be designed to recognize a different target molecule on the surface of a cell or pathogen.

In one particular example, a pair of chimeras may include two different extracellular domains, each recognizing a distinct antigen characteristic of a targeted tumor. Examples of tumor antigens include, without limitation, any of a number of carbohydrates (e.g., Le^{Y}, sialyl-Le^{Y}, Le^{X}, and sialyl-Le^{X}), carcinoembryonic antigen, CD40, modified CD44, α-fetoprotein, the T and Tn antigens, tenascin, and growth factor receptors (e.g., HER2/neu). By increasing the number of tumor surface markers that must be recognized to elicit a potent destructive response, this approach increases therapeutic specificity by decreasing the likelihood and frequency of destruction of non-cancerous cells.

This method of combinatorial control can be extended to any number of cooperating chimeric receptors and may be used to regulate any therapeutic method of the invention.

CD28 chimeras are constructed and expressed according to the methods described herein. The CD28 sequence is provided in Aruffo and Seed (Proc. Natl. Acad. Sci. USA 84:8573-8577 (1987)). Also included in this reference are descriptions of the CD28 intracellular and transmembrane domains. An example of a chimera bearing an intracellular CD28 domain is disclosed in Romeo et al. (Cold Spring Harbor Symp. on Quant. Biol. LVII:117-125 (1992)).

### EXAMPLE XII

### Experimental Methods

### Vaccinia Infection and Radioimmunoprecipitation

Approximately 5 x 10⁶ CV1 cells were infected for one hour in serum free DME medium with recombinant vaccinia at a multiplicity of infection (moi) of at least ten (titer measured on CV1 cells). The cells were placed in fresh medium after infection and labelled metabolically with 200µCi/ml ³⁵S-methionine plus cysteine (Tran³⁵S-label, ICN; Costo Mesa, CA) in methionine and cysteine free DMEM (Gibco; Grand Island, NY) for six hours. The labelled cells were detached with PBS containing 1mM EDTA, collected by centrifugation, and lysed in 1% NP-40, 0.1% SDS, 0.15 M NaCl, 0.05M Tris pH 8.0, 5mM EDTA, and 1mM PMSF. Nuclei were removed by centrifugation, and CD4 proteins immunoprecipitated with OKT4 antibody and anti-mouse IgG agarose (Cappel, Durham, NC). Samples were electrophoresed through 8% polyacrylamide/SDS gels under non-reducing (NR) and reducing (R) conditions. Gels containing ³⁵S-labelled samples were impregnated with En³Hance (New England Nuclear, Boston, MA) prior to autoradiography. Facilitated expression of the transmembrane form of CD16, CD16_{TM}, was measured by comparing its expression in CV1 cells singly infected with CD16_{TM} with expression in cells coinfected with viruses encoding CD16_{TM} and ζ or γ chimeras. After infection and incubation for six hours or more, cells were detached from plates with PBS, 1mM EDTA and the expression of CD16TM or the chimeras was measured by indirect immunofluorescence and flow cytometry.

### Calcium Flux Assay

Jurkat subline E6 (Weiss et al., J. Immunol. 133:123-128 (1984)) cells were infected with recombinant vaccinia viruses for one hour in serum free IMDM at an moi of 10 and incubated for three to nine hours in IMDM, 10% FBS. Cells were collected by centrifugation and resuspended at 3 x 10⁶ cells/ml in complete medium containing 1mM Indo-1 acetomethoxyester (Grynkiewicz et al., J. Biol. Chem. 260:3340-3450 (1985)) (Molecular Probes) and incubated at 37°C for 45 minutes. The Indo-1 loaded cells were pelleted and resuspended at 1 x 10⁶/ml in serum free IMDM and stored at room temperature in the dark. Cells were analyzed for free calcium ion by simultaneous measurement of the violet and blue fluorescence emission by flow cytometry (Rabinovitch et al., J. Immunol. 137:952-961 (1986)). To initiate calcium flux, either phycoerythrin (PE)-conjugated Leu-3A (anti-CD4) (Becton Dickinson, Lincoln Park, NJ) at 1 µg/ml was added to the cell suspension followed by 10µg/ml of unconjugated goat anti-mouse IgG at time 0 or unconjugated 3G8 (anti-CD16) monoclonal antibody was added to the cell suspension at 1 µg/ml followed by 10 µg/ml of PE-conjugated Fab₂' goat anti-monse IgG at time 0. Histograms of the violet/blue emission ratio were collected from the PE positive (infected) cell population, which typically represented 40-80% of all cells. The T cell antigen receptor response in uninfected cells was triggered by antibody OKT3, without crosslinking. For experiments involving CD16 chimeric receptors, samples showing baseline drift toward lower intracellular calcium (without antibody) were excluded from the analysis. Histogram data were subsequently analyzed by conversion of the binary data to ASCII using Write Hand Man (Cooper City, FL) software, followed by analysis with a collection of FORTRAN programs. The violet/blue emission ratio prior to the addition of the second antibody reagents was used to establish the normalized initial ratio, set equal to unity, and the resting threshold ratio, set so that 10% of the resting population would exceed threshold.

### Cytolysis Assay

Human T cell line WH3, a CD8⁺ CD4⁻ HLA B44 restricted cytolytic line was maintained in IMDM, 10% human serum with 100 U/ml of IL-2 and was periodically stimulated either nonspecifically with irradiated (3000 rad) HLA-unmatched peripheral blood lymphocytes and 1µg/ml of phytohemagglutinin, or specifically, with irradiated B44-bearing mononuclear cells. After one day of nonspecific stimulation, the PHA was diluted to 0.5 µg/ml by addition of fresh medium, and after three days the medium was changed. Cells were grown for at least 10 days following stimulation before use in cytotoxicity assays. The cells were infected with recombinant vaccinia at a multiplicity of infection of at least 10 for one hour in serum free medium, followed by incubation in complete medium for three hours. Cells were harvested by centrifugation and resuspended at a density of 1 x 10⁷ cells/ml. 100µl were added to each well of a U-bottom microtiter plate containing 100 µl/well of complete medium. Cells were diluted in two-fold serial steps. Two wells for each sample did not contain lymphocytes, to allow spontaneous chromium release and total chromium uptake to be measured. The target cells, from HeLa subline S3, were infected in 6.0 or 10.0 cm plates at an approximate moi of 10 for one hour in serum free medium, followed by incubation in complete medium for three hours. They were then detached from the dishes with PBS, 1mM EDTA and counted. An aliquot of 10⁶ target cells (HeLa, Raji, or RJ2.2.5 cells for the CD4 chimeric receptor experiments and 3G8 10-2 cells; Shen et al., Mol. Immunol. 26:959 (1989) for the CD16 chimeric receptor experiments) was centrifuged and resuspended in 50 µl of sterile ⁵¹Cr-sodium chromate (1mCi/ml, Dupont Wilmington, DE) for one hour at 37°C with intermittent mixing, then washed three times with PBS. 100 µl of labelled cells resuspended in medium at 10⁵ cells/ml were added to each well. Raji and RJ2.2.5 target cells were labelled in the same manner as HeLa cells. The microtiter plate was spun at 750 x g for 1 minute and incubated for 4 hours at 37°C. At the end of the incubation period, the cells in each well were resuspended by gentle pipetting, a sample removed to determine the total counts incorporated, and the microtiter plate spun at 750 x g for 1 minute. 100µl aliquots of supernatant were removed and counted in a gamma ray scintillation counter. The percent killing was corrected for the fraction of infected target cells (usually 50-90%) measured by flow cytometry. For infected effector cells the effector:target ratio was corrected for the percent of cells infected (usually 20-50% for the CD4 chimeric receptor experiments and >70% for the CD16 chimeric receptor experiments).

### In Vitro Mutagenesis of the ζ Sequence

To create point mutations in amino acid residues 11 and or 15 of the ζ sequence, synthetic oligonucleotide primers extending from the BamHI site upstream of the ζ transmembrane domain, and converting native ζ residue 11 from Cys to Gly (C11G) or residue 15 from Asp to Gly (D15G) or both (C11G/D15G) were prepared and used in PCR reactions to generate mutated fragments which were reinserted into the wild type CD4:ζ constructs.

To create ζ deletions, ζ cDNA sequences were amplified by PCR using synthetic oligonucleotide primers designed to create a stop codon (UAG) after residues 50, 59, or 65. The primers contained the cleavage site for the enzyme NotI indented five or six residues from the 5' end, usually in a sequence of the form CGC GGG CGG CCG CTA (SEQ ID NO: 11), where the last three residues correspond to the stop anticodon. The NotI and stop anticodon sequences were followed by 18 or more residues complementary to the desired 3' end of the fragment. The resulting chimeras were designated CD16:ζY51*, CD16:ζE60* and CD16:ζD66* respectively. The BamHI site upstream of the transmembrane domain and the NotI site were used to generate fragments that were reintroduced into the wild type CD16:ζ construct. Monomeric ζ chimeras were created by liberating the ζ transmembrane and membrane proximal intracellular sequences by BamHI and SacI digestion of the Asp⁻ and Cys⁻ CD4:ζ construct described above and inserting the fragment into the CD16:ζE60* and CD16:ζD66* construct respectively.

### CD16:7:ζ(48-65) and CD16:7ζ(48-59) tripartite chimera construction.

To prepare the construct CD16:ζD66*, the ζ cDNA sequence corresponding to the transmembrane domain and the 17 following residues of the cytoplasmic domain was replaced by corresponding transmembrane and cytoplasmic domain obtained from the CD5 and CD7 cDNA. The CD5 and CD7 fragments were generated by a PCR reaction using forward oligonucleotides including a BamHI restriction cleavage site and corresponding to the region just upstream of the transmembrane domain of CD5 and CD7 respectively and the following reverse oligonucleotides overlapping the CD5 and CD7 sequences respectively and the ζ sequence which contained the SacI restriction cleavage site.

The CD5 and CD7 PCR products were digested with BamHI and SacI and ligated to BamHI and SacI digested CD16:ζE60* and replacing the ζ sequence from BamHI to SacI by the CD7 fragment. To make the constructs CD16:CD5 and CD16:CD7, CD5 and CD7 fragments were obtained by PCR using an oligonucleotide containing a NotI restriction cleavage site and encoding a stop codon (UAA) after the residue Gln416 and Ala193 of CD5 and CD7 respectively. The CD5 and CD7 PCR fragment were digested with BamHI and NotI and inserted in the CD16:ζAsp66* construct.

### In Vitro Mutagenesis of the N-terminal Residues within the ζ Cytolytic Signal-Transducing Motif

Synthetic oligonucleotide primers extending from the SacI site inside the ζ motif and converting native residue 48 from Asn to Ser (N48S), residue 50 from Leu to Ser (L50S) and residue 51 from Tyr to Phe (Y51F) were synthesized and used in a PCR reaction to generate fragments that were reintroduced into the wild type CD16:7:ζ(48-65) construct.

### In Vitro Mutagenesis of C-terminal Residues within the ζ Cytolytic Signal-Transducing Motif

Synthetic oligonucleotide primers extending from the NotI site 3' to the stop codon and converting native residue 60 from Glu to Gln (E60Q), residue 61 from Glu to Gln (E61Q), residue 62 from Tyr to Phe or Ser (Y62F or Y62S) and residue 63 from Asp to Asn (D63N) were synthesized and used in PCR to generate fragments that were subcloned into the wild type CD16:ζD66* construct from the BamHI site to the NotI site.

### CD16:7:ζ(33-65), CD16:7:ζ(71-104), CD16:7:ζ(104-137)chimera constructions

A CD7 transmembrane fragment bearing MluI and NotI sites at the junction between the transmembrane and intracellular domains was obtained by PCR using an oligonucleotide with the following sequence: CGC GGG GCG GCC ACG CGT CCT CGC CAG CAC ACA (SEQ ID NO:14). The resulting PCR fragment was digested with BamHI and NotI and reinserted into the CD16:7:ζ(48-65) construct. ζ fragments encoding residues 33 to 65, 71 to 104, and 104 to 137 were obtained by PCR reaction using pairs of primers containing MluI sites at the 5' end of the forward primers and stop codons followed by NotI sites at the 5' end of the reverse primers. In each case the restriction sites were indented six residues from the 5' terminus of the primer to insure restriction enzyme cleavage. and

### Construction of FcRγIIA Deletion Mutants

Carboxyl terminal FcRIIA deletion mutants were constructed by PCR in the same fashion as for the full length constructs, converting the sequences encoding tyrosine at positions 282 and 298 into stop codons (TAA). The N-terminal deletions were generated by amplifying fragments encoding successively less of the intracellular domain by PCR, using oligonucleotides which allowed the resulting fragments to be inserted between MluI and NotI restriction sites into a previously constructed expression plasmid encoding the CD16 extracellular domain fused to the CD7 transmembrane domain, the latter terminating in a MluI site and the junction between the transmembrane and the intracellular domain.

### OTHER EMBODIMENTS

The examples described above demonstrate that aggregation of ζ, η, or γ chimeras suffices to initiate the cytolytic effector cell response in T cells. The known range or expression of ζ, η, and γ, which includes T lymphocytes, natural killer cells, basophilic granulocytes, macrophages, and mast cells, suggests that conserved sequence motifs may interact with a sensory apparatus common to cells of hematopoietic origin and that an important component of host defense in the immune system may be mediated by receptor aggregation events.

The potency of the cytolytic response and the absence of a response to target cells bearing MHC class II receptors demonstrates that chimeras based on ζ, η, or γ form the basis for a genetic intervention for AIDS through adoptive immunotherapy. The broad distribution of endogenous ζ and γ and evidence that Fc receptors associated with γ mediate cytotoxicity in different cells types (Fanger et al., Immunol. Today 10:92-99 (1989)) allows a variety of cells to be considered for this purpose. For example, neutrophilic granulocytes, which have a very short lifespan (≈ 4h) in circulation and are intensely cytolytic, are attractive target cells for expression of the chimeras. Infection of neutrophils with HIV is not likely to result in virus release, and the abundance of these cells (the most prevalent of the leukocytes) should facilitate host defense. Another attractive possibility for host cells are mature T cells, a population presently accessible to retroviral engineering (Rosenberg, Sci. Am. 262:62-69 (1990)). With the aid of recombinant IL-2, T cell populations can be expanded in culture with relative ease, and the expanded populations typically have a limited lifespan when reinfused (Rosenberg et al., N. Engl. J. Med. 323:570-578 (1990)).

Under the appropriate conditions, HIV recognition by cells expressing CD4 chimeras should also provide mitogenic stimuli, allowing the possibility that the armed cell population could respond dynamically to the viral burden. Although we have focused here on the behavior of the fusion proteins in cytolytic T lymphocytes, expression of the chimeras in helper lymphocytes might provide an HIV-mobilized source of cytokines which could counteract the collapse of the helper cell subset in AIDS. Recent description of several schemes for engineering resistance to infection at steps other than virus penetration (Friedman et al., Nature 335:452-454 (1988); Green et al., Cell 58:215-223 (1989); Malim et al., Cell 58:205-214 (1989); Trono et al., Cell 59:113-120 (1989); Buonocore et al., Nature 345:625-628 (1990)) suggests that cells bearing CD4 chimeras could be designed to thwart virus production by expression of appropriate agents having an intracellular site of action.

The ability to transmit signals to T lymphocytes through autonomous chimeras also provides the ability for the regulation of retrovirally engineered lymphocytes in vivo. Crosslinking stimuli, mediated for example by specific IgM antibodies engineered to remove complement-binding domains, may allow such lymphocytes to increase in number in situ, while treatment with similar specific IgG antibodies (for example recognizing an amino acid variation engineered into the chimeric chain) could selectively deplete the engineered population. Additionally, anti-CD4 IgM antibodies do not require additional crosslinking to mobilize calcium in Jurkat cells expressing CD4:ζ chimeras. The ability to regulate cell populations without recourse to repeated extracorporeal amplification may substantially extend the range and efficacy of current uses proposed for genetically engineered T cells.

To create other chimeras consisting of ζ, η or γ intracellular sequences, cDNA or genomic sequences encoding an extracellular domain of the receptor can be endowed with a restriction site introduced at a location just preceding the transmembrane domain of choice. The extracellular domain fragment terminating in the restriction site can then be joined to ζ, η, or γ sequences. Typical extracellular domains may be derived from receptors which recognize complement, carbohydrates, viral proteins, bacteria, protozoan or metazoan parasites, or proteins induced by them. Similarly, ligands or receptors expressed by pathogens or tumor cells can be attached to ζ, η, or γ sequences, to direct immune responses against cells bearing receptors recognizing those ligands.

While the invention has been described in connection with specific embodiments thereof, it will be understood that it is capable of further modifications and this application is intended to cover variations, uses, or adaptations of the invention and including such departures from the present disclosure as come within the art to which the invention pertains and as may be applied to the essential features hereinbefore set forth as follows in the scope of the appended claims.

## Claims

1. A method for manufacturing a medicament for the therapeutic application in an infection, a tumor disease or an autoimmune disease, **characterized in that** therapeutic cells are generated expressing at least two membrane-bound, proeinaceous chimeric receptors
one of said receptors comprising (a) an extracellular portion which is capable of specifically recognizing and binding a target cell or a target infective agent and (b) an intracellular or transmembrane portion which is capable of signalling said therapeutic cell to destroy a receptor-bound target cell or a receptor-bound target infective agent; wherein said intracellular or transmembrane portion is derived from a T cell receptor ξ, η, CD3 delta, or T3 gamma protein; an Fc receptor γ protein; or a B cell receptor mb1 or B29 protein, or a functional derivative thereof;
and
the second of said receptors comprising (a) an extracellular portion which is capable of specifically recognizing and binding said target cell or said target infective agent and (b) an intracellular portion which is derived from CD28.

2. The method of any of claim 1, wherein said target cell is a host cell infected with an infective agent, a tumor or cancerous cell, or an autoimmune-generated cell.

3. The method of any of claims 1 or 2, wherein said cellular response is MHC-independent.

4. The method of any of claims 1-3, wherein, following binding of said extracellular portion to said agent or said cell, said transmembrane portion oligomerizes with a cytolytic signal-transducing protein of said therapeutic cell resulting in destruction of said receptor-bound cell or agent.

5. The method of claim 4, wherein said chimeric receptor comprises either
(a) amino acids 421-532 of SEQ ID NO: 6 or a functional cytolytic signal-transducing derivative thereof;
(b) amino acids (a) 423-455; (b) 438-455; (c) 461-494; or (d) 494-528 of SEQ ID NO: 6;
(c) amino acids 400-420 of SEQ ID NO: 6;
(d) amino acids 421-575 of SEQ ID NO: 4 or a functional cytolytic signal-transducing derivative thereof;
(e) amino acids (a) 423-455; (b) 438-455; (c) 461-494; or (d) 494-528 of SEQ ID NO:4;
(f) amino acids 400-420 of SEQ ID NO: 4 or a
(g) amino acids 421-462 of SEQ ID NO: 5 or a functional cytolytic signal-transducing derivatice thereof;
(h) amino acids 402-419 of SEQ ID NO: 5;
(i) amino acids Tyr282-Tyr298 inclusive of Fig. 15 A;
(j) amino acids 132-171 of Fig. 16 (SEQ ID NO: 24);
(k) amino acids 140-182 of Fig. 17 (SEQ ID NO: 25);
(l) amino acids 162-220 of Fig. 18 (SEQ ID NO: 26; or
(m) amino acids 183-228 of Fig. 19 (SEQ ID NO: 27).

6. The method of claims 1 to 3, wherein said Fc receptor protein is human FcγRIII, human FcRIIγA, or human FcRIIγC.

7. The method of claims 1 to 3, wherein said therapeutic cells are selected from the group consisting of:
(a) T lymphocytes;
(b) cytotoxic T lymphocytes;
(c) natural killer cells;
(d) neuthrophils;
(e) granulocytes;
(f) macrophages;
(g) mast cells and
(h) HeLa cells

8. The method of claims 1 to 3, wherein said target infective agent is an immunodeficiency virus.

9. The method of claims 1 to 3 , wherein said extracellular portion comprises an HIV envelope-binding portion of CD4, or a functional HIV envelope-binding derivative thereof.

10. The method of claims 1 to 3, wherein said HIV-envelope binding portion of CD4 comprises the peptide encoded by nucleotides 1-369 of SEQ ID NO: 1.

11. The method of claims 1 to 3, wherein said therapeutic cells destroy said receptor-bound target cell or target infective agent by cytolysis.

12. A cell which expresses at least two proteinaceous membrane-bound chimeric receptors, one of said receptors comprising (a) an extracellular portion and (b) an intracellular or transmembrane portion wherein said intracellular or transmembrane portion is the cell signal-transducing portion of a T cell receptor protein, a B cell receptor protein, or an Fc receptor protein, or a functional derivative thereof; and the second of said receptors comprising (a) an extracellular portion, wherein said extracellular portion of the first and the second receptor comprises an HIV envelope-binding portion of CD4, or a functional HIV envelope-binding derivative thereof; and (b) an intracellular portion which is derived from CD28.

13. The cell of claim 12, wherein said target cell is a host cell infected with an infective agent, a tumor or cancerous cell, or an autoimmune-generated cell.

14. The cell of any of claims 12 or 13, wherein said binding is MHC-independent.

15. The cell of any of claims 12-14, wherein said intracellular or transmembrane domain is derived from either a T cell receptor ξ, η, CD3 delta, or T3 gamma protein; an Fc receptor γ protein; or a B cell receptor mbl or B29 protein.

16. The cell of claim 15, wherein said chimeric receptor comprises either
(a) amino acids 421-532 of SEQ ID NO: 6 or a functional cytolytic signal-transducing derivative thereof;
(b) amino acids (a) 423-455; (b) 438-455; (c) 461-494; or
(d) 494-528 of SEQ ID NO: 6;
(c) amino acids 400-420 of SEQ ID NO: 6;
(d) amino acids 421-575 of SEQ ID NO: 4; or a functional cytolytic signal-transducing derivative thereof;
(e) amino acids (a) 423-455; (b) 438-455; (c) 461-494; or
(d) 494-528 of SEQ ID NO: 4;
(f) amino acids 400-420 of SEQ ID NO: 4;
(g) amino acids 421-462 of SEQ ID NO: 5 or a functional cytolytic signal-transducing derivative thereof;
(h) amino acids 402-419 of SEQ ID NO: 5;
(i) amino acids Tyr 282-Tyr298 inclusive Fig. of 15A
(j) amino acids 132-171 of Fig. 16 (SEQ ID NO: 24);
(k) amino acids 140-182 of Fig. 17 (SEQ ID NO: 25);
(l) amino acids 162-220 of Fig. 18 (SEQ ID NO: 26);
(m) amino acids 183-228 of Fig. 19 (SEQ ID NO: 27);

17. The cell of any of claims 12-14, wherein said Fc receptor protein is human FcγRIII, human FcRIIIγA, or human FcRIIγC.

18. The cell of any of claims 12-14, wherein said extracellular portion comprises the ligand-binding portion of a receptor, the receptor-binding portion of a ligand, the antigen-binding portion of an antibody, or a functional derivative thereof.

19. The cell of claim 18, wherein said target infective agent is an immunodeficiency virus or said target cell is a host cell infected with an immunodeficiency virus.

20. The cell of claim 19, wherein said extracellular portion comprises an HIV envelope-binding portion of CD4, or a functional derivative thereof.

21. The cell of claim 20, wherein said HIV-envelope binding portion of CD4 comprises the peptide encoded by nucleotides 1-369 of SEQ ID NO: 1.

22. The cell of claim 12, wherein said cell destroys said receptor-bound target cell or target infective agent by cytolysis.

23. A cell which expresses at least two protinaceous membrane-bound chimeric receptors, one of said receptors comprising (a) an extracellular portion, and (b) an intracellular or transmembrane portion derived form a T cell receptor CD3, zeta, or eta polypeptide, a B cell receptor, or an Fc receptor; and
the second of said receptors comprising (a) an extracellular portion, wherein said extracellular portion of the first and the second receptor comprise an HIV envelope-binding portion of CD4, or a functional HIV envelope-binding derivative thereof, and (b) an intracellular portion which is derived from CD28.

24. The cell of claim 12 or 23, wherein said chimeric receptor includes either
(a) a CD16, CD7, or CD5 extracellular portion;
(b) a CD5 or CD7 transmembrane portion; or
(c) a CD5 intracellular portion.

25. A pair of proteinaceous membrane-bound chimeric receptors,
one of said receptors comprising (a) an extracellular portion and (b) an intracellular or transmembrane portion wherein said intracellular or transmembrane portion is the signal-transducing portion of a T cell receptor protein, a B cell receptor protein, or an Fc receptor protein, or a functional derivative thereof; and the second of said receptors comprising (a) an extracellular portion, wherein said extracellular portions of the first and the second receptor comprise an HIV envelope-binding portion of CD4, or a functional HIV envelope-binding derivative therof; and (b) an intracellular portion which is derived from CD28.

26. A pair of proteinaceaous membrane-bound chimeric receptors, one of said receptors comprising (a) an extracellular portion and (b) an intracellular or transmembrane portion derived from a T cell receptor CD3, zeta, or eta polypeptide, a B cell receptor, or an Fc receptor; and
the second of said receptors comprising (a) an extracellular portion, wherein said extracellular portions of the first and the second receptor comprise HIV envelope-binding portion of CD4, or a functional HIV envelope-binding derivative thereof and (b) an intracellular portion which is derived from CD28.

27. A pair of DNA molecules, each encoding a proteinaceous membrane-bound chimeric receptor according to any of claims 25 or 26.

## Patentansprüche

1. Verfahren zur Herstellung eines Medikaments zur therapeutischen Anwendung bei einer Infektion, einer Tumorerkrankung oder einer Autoimmunerkrankung, **dadurch gekennzeichnet, daß** therapeutische Zellen erzeugt werden, die wenigstens zwei Membran-gebundene, proteinische, chimäre Rezeptoren exprimieren,
wobei einer der Rezeptoren umfaßt: (a) einen extrazellulären Teil, der fähig ist, eine Zielzelle oder ein infektiöses Zielagens spezifisch zu erkennen und zu binden, und (b) einen intrazellulären oder Transmembran-Teil, der fähig ist, der therapeutischen Zelle das Signal zu geben, eine Rezeptor-gebundene Zielzelle oder ein Rezeptor-gebundenes, infektiöses Agens zu zerstören; wobei der intrazelluläre oder Transmembran-Teil von einem T-Zellrezeptor ζ-, η-, CD3-delta- oder T3-gamma-Protein; einem Fc-Rezeptor-γ-protein oder einem B-Zellrezeptor mbl- oder B29-Protein oder einem funktionellen Derivat davon abgeleitet ist; und
wobei der zweite der Rezeptoren umfaßt: (a) einen extrazellulären Teil, der fähig ist, die Zielzelle oder das infektiöse Zielagens spezifisch zu erkennen und zu binden und (b) einen intrazellulären Teil, der von CD28 abgeleitet ist.

2. Verfahren nach Anspruch 1, wobei die Zielzelle eine mit einem infektiösen Agens infizierte Wirtszelle, eine Tumor- oder Krebszelle oder eine autoimmun gebildete Zelle ist.

3. Verfahren nach Anspruch 1 oder Anspruch 2, wobei die zelluläre Antwort MHC-unabhängig ist.

4. Verfahren nach einem der Ansprüche 1 bis 3, wobei nach Bindung des extrazellulären Teils an das Agens oder die Zelle der Transmembran-Teil mit einem cytolytischen, signaltransduzierenden Protein der therapeutischen Zelle oligomerisiert, was zur Zerstörung der Rezeptor-gebundenen Zelle oder des Rezeptor-gebundenen Agenses führt.

5. Verfahren nach Anspruch 4, wobei der chimäre Rezeptor entweder
(a) Aminosäuren 421-532 von SEQ ID NO: 6 oder ein funktionelles, cytolytisches, signaltransduzierendes Derivat davon;
(b) Aminosäuren (a) 423-455; (b) 438-455; (c) 461-494 oder (d) 494-528 von SEQ ID NO: 6;
(c) Aminosäuren 400-420 von SEQ ID NO: 6;
(d) Aminosäuren 421-575 von SEQ ID NO: 4 oder ein funktionelles, cytolytisches, signaltransduzierendes Derivat davon;
(e) Aminosäuren (a) 423-455; (b) 438-455; (c) 461-494 oder (d) 494-528 von SEQ ID NO: 4;
(f) Aminosäuren 400-420 von SEQ ID NO: 4 oder
(g) Aminosäuren 421-462 von SEQ ID NO: 5 oder ein funktionelles, cytolytisches, signaltransduzierendes Derivat davon;
(h) Aminosäuren 402-419 von SEQ ID NO: 5;
(i) Aminosäuren Tyr282-Tyr298, einschließlich,von Figur 15A;
(j) Aminosäuren 132-171 von Figur 16 (SEQ ID NO: 24);
(k) Aminosäuren 140-182 von Figur 17 (SEQ ID NO: 25);
(l) Aminosäuren 162-220 von Figur 18 (SEQ ID NO: 26); oder
(m) Aminosäuren 183-228 von Figur 19 (SEQ ID NO: 27)
umfaßt.

6. Verfahren nach den Ansprüchen 1 bis 3, wobei das Fc-Rezeptorprotein humanes FcγRIII, humanes FcRIIγA oder humanes FcRIIγC ist.

7. Verfahren nach den Ansprüchen 1 bis 3, wobei die therapeutischen Zellen aus der Gruppe, bestehend aus
(a) T-Lymphozyten;
(b) cytotoxischen T-Lymphozyten;
(c) natürlichen Killerzellen;
(d) Neuthrophilen;
(e) Granulozyten;
(f) Makrophagen;
(g) Mastzellen und
(h) HeLa-Zellen, ausgewählt sind.

8. Verfahren nach den Ansprüchen 1 bis 3, wobei des infektiöse Zielagens ein Immundefizienzvirus ist.

9. Verfahren nach den Ansprüchen 1 bis 3, wobei der extrazelluläre Teil einen HIV-Hülle-bindenden Teil von CD4 oder ein funktionelles HIV-Hülle-bindendes Derivat davon umfaßt.

10. Verfahren nach den Ansprüchen 1 bis 3, wobei der HIV-Hülle-bindenden Teil von CD4 das Peptid umfaßt, das durch die Nukleotide 1-369 von SEQ ID NO: 1 codiert wird.

11. Verfahren nach den Ansprüchen 1 bis 3, wobei die therapeutischen Zellen die Rezeptor-gebundene Zielzelle oder das infektiöse Zielagens durch Cytolyse zerstören.

12. Zelle, die wenigstens zwei proteinische, membran-gebundene, chimäre Rezeptoren exprimiert, wobei einer der Rezeptoren umfaßt: (a) einen extrazellulären Teil und (b) einen intrazellulären oder Transmembran-Teil,
wobei der intrazelluläre Teil oder Transmembran-Teil der zellsignaltransduzierende Teil eines T-Zell-Rezeptorproteins, eines B-Zell-Rezeptorproteins oder eines Fc-Rezeptorproteins oder eines funktionellen Derivats davon ist; und der zweite der Rezeptoren umfaßt: (a) einen extrazellulären Teil, wobei der extrazelluläre Teil des ersten und des zweiten Rezeptors einen HIV-Hülle-bindenden Teil von CD4 oder ein funktionelles HIV-Hülle-bindendes Derivat davon umfaßt, und (b) einen intrazellulären Teil, der von CD28 abgeleitet ist.

13. Zelle nach Anspruch 12, wobei die Zielzelle einem mit einem infektiösen Agens infizierte Wirtszelle, eine Tumor- oder Krebszelle oder eine autoimmun gebildete Zelle ist.

14. Zelle nach Anspruch 12 oder 13, wobei die Bindung MHC-unabhängig ist.

15. Zelle nach einem der Ansprüche 12 bis 14, wobei die intrazelluläre oder Transmembran-Domäne entweder von einem T-Zellrezeptor ζ-, -η-, CD3-delta- oder T3-gamma-Protein; einem Fc-Rezeptor-γ-protein oder einem B-Zellrezeptor mbl- oder B29-Protein abgeleitet ist.

16. Zelle nach Anspruch 15, wobei der chimäre Rezeptor entweder
(a) Aminosäuren 421-532 von SEQ ID NO: 6 oder ein funktionelles, cytolytisches, signaltransduzierendes Derivat davon;
(b) Aminosäuren (a) 423-455; (b) 438-455; (c) 461-494 oder (d) 494-528 von SEQ ID NO: 6;
(c) Aminosäuren 400-420 von SEQ ID NO: 6;
(d) Aminosäuren 421-575 von SEQ ID NO: 4 oder ein funktionelles, cytolytisches, signaltransduzierendes Derivat davon;
(e) Aminosäuren (a) 423-455; (b) 438-455; (c) 461-494 oder (d) 494-528 von SEQ ID NO: 4;
(f) Aminosäuren 400-420 von SEQ ID NO: 4
(g) Aminosäuren 421-462 von SEQ ID NO: 5 oder ein funktionelles, cytolytisches, signaltransduzierendes Derivat davon;
(h) Aminosäuren 402-419 von SEQ ID NO: 5;
(i) Aminosäuren Tyr282-Tyr298, einschließlich, von Figur 15A;
(j) Aminosäuren 132-171 von Figur 16 (SEQ ID NO: 24);
(k) Aminosäuren 140-182 von Figur 17 (SEQ ID NO: 25) ;
(l) Aminosäuren 162-220 von Figur 18 (SEQ ID NO: 26);
(m) Aminosäuren 183-228 von Figur 19 (SEQ ID NO: 27)
umfaßt.

17. Zelle nach einem der Ansprüche 12 bis 14, wobei das Fc-Rezeptorprotein humanes FcγRIII, humanes FcRIIIγA oder humanes FcRIIγC ist.

18. Zelle nach einem der Ansprüche 12 bis 14, wobei der extrazelluläre Teil den Liganden-bindenden Teil eines Rezeptors, den Rezeptor-bindenden Teil eines Liganden, den Antigen-bindenden Teil eines Antikörpers oder ein funktionelles Derivat davon umfaßt.

19. Zelle nach Anspruch 18, wobei das infektiöse Zielagens ein Immundefizienzvirus ist oder die Zielzelle eine mit einem Immundefizienzvirus infizierte Wirtszelle ist.

20. Zelle nach Anspruch 19, wobei der extrazelluläre Teil einen HIV-Hülle-bindenden Teil von CD4 oder ein funktionelles Derivat davon umfaßt.

21. Zelle nach Anspruch 20, wobei der HIV-Hülle-bindende Teil von CD4 das Peptid umfaßt, das durch die Nukleotide 1-369 von SEQ ID NO: 1 codier wird.

22. Zelle nach Anspruch 12, wobei die Zelle die Rezeptor-gebundene Zielzelle oder das infektiöse Zielagens durch Cytolyse zerstört.

23. Zelle, die wenigstens zwei proteinische Membran-gebundene, chimäre Rezeptoren exprimiert, wobei einer der Rezeptoren umfaßt: (a) einen extrazellulären Teil und (b) einen intrazellulären oder Transmembran-Teil, der von einem T-Zellrezeptor CD3, zeta- oder eta-Polypeptid, einem B-Zellrezeptor oder einem Fc-Rezeptor abgeleitet ist, und
der zweite der Rezeptoren umfaßt: (a) einen extrazellulären Teil, wobei der extrazelluläre Teil des ersten und des zweiten Rezeptors einen HIV-Hüllebindenden Teil von CD4 oder ein funktionelles HIV-Hülle-bindendes Derivat davon umfaßt, und (b) einen intrazellulären Teil, der von CD28 abgeleitet ist.

24. Zelle nach Anspruch 12 oder 23, wobei der chimäre Rezeptor entweder
(a) einen extrazellulären CD16-, CD7- oder CD5-Teil;
(b) einen CD5- oder CD7-Transmembran-Teil oder
(c) einen intrazellulären CD5-Teil umfaßt.

25. Paar proteinischer Membran-gebundener, chimärer Rezeptoren, wobei einer der Rezeptoren umfaßt: (a) einen extrazellulären Teil und (b) einen intrazellulären oder Transmembran-Teil, wobei der intrazelluläre oder Transmembran-Teil der signaltransduzierende Teil eines T-Zell-Rezeptorproteins, eines B-Zell-Rezeptorproteins oder eines Fc-Rezeptorproteins oder eines funktionellen Derivats davon ist; und der zweite der Rezeptoren umfaßt: (a) einen extrazellulären Teil, wobei die extrazellulären Teile des ersten und des zweiten Rezeptors einen HIV-Hülle-bindenden Teil von CD4 oder ein funktionelles HIV-Hülle-bindendes Derivat davon umfassen, und (b) einen intrazellulären Teil, der von CD28 abgeleitet ist.

26. Paar proteinischer membrangebundener, chimärer Rezeptoren, wobei einer der Rezeptoren umfaßt: (a) einen extrazellulären Teil und (b) einen intrazellulären oder Transmembran-Teil, der von einem T-Zellrezeptor CD3, zeta- oder eta-Polypeptid, einem B-Zellrezeptor oder einem Fc-Rezeptor abgeleitet ist; und
der zweite der Rezeptoren umfaßt: (a) einen extrazellulären Teil, wobei die extrazellulären Teile des ersten und des zweiten Rezeptors einen HIV-Hüllebindenden Teil von CD4 oder ein funktionelles HIV-Hülle-bindendes Derivat davon umfassen, und (b) einen intrazellulären Teil, der von CD28 abgeleitet ist.

27. Paar DNA-Moleküle, wobei jedes für einen proteinischen, Membran-gebundenen, chimären Rezeptor nach Anspruch 25 oder 26 codiert.

## Revendications

1. Procédé de fabrication d'un médicament destiné à une application thérapeutique au cours d'une infection, d'une maladie tumorale ou d'une maladie auto-immune, **caractérisé en ce que** des cellules thérapeutiques sont générées, lesquelles expriment au moins deux récepteurs protéiques-chimères liés à une membrane :
l'un desdits récepteurs comprenant : (a) une partie extra-cellulaire, qui est capable de reconnaître spécifiquement une cellule cible ou un agent infectieux cible et de se lier à celle-ci ; et (b) une partie intra-cellulaire ou transmembranaire, qui est capable de donner le signal à ladite cellule thérapeutique de détruire une cellule cible liée à un récepteur ou un agent infectieux cible lié à un récepteur ; dans lequel ladite partie intra-cellulaire ou transmembranaire est dérivée : d'une protéine ξ, η, CD3 delta ou T3 gamma d'un récepteur de lymphocyte T ; d'une protéine γ d'un récepteur de Fc ; d'une protéine mbl ou B29 d'un récepteur de lymphocyte B ; ou d'un dérivé fonctionnel de celles-ci ;
et
le deuxième desdits récepteurs comprenant : (a) une partie extra-cellulaire, qui est capable de reconnaître spécifiquement ladite cellule cible ou ledit agent infectieux cible et de se lier à celle-ci ; et (b) une partie intra-cellulaire qui est dérivée d'un CD28.

2. Procédé selon la revendication 1, dans lequel ladite cellule cible est une cellule hôte infectée par un agent infectieux, une cellule tumorale ou cancéreuse ou une cellule générée de manière auto-immune.

3. Procédé selon l'une quelconque des revendications 1 ou 2, dans lequel ladite réponse cellulaire est indépendante du CMH.

4. Procédé selon l'une quelconque des revendications 1 à 3, dans lequel, après une liaison de ladite partie extra-cellulaire au dit agent ou à ladite cellule, ladite partie transmembranaire s'oligomérise avec une protéine cytolytique transduisant un signal de ladite cellule thérapeutique, ce qui conduit à une destruction de ladite cellule, ou dudit agent, liée au récepteur.

5. Procédé selon la revendication 4, dans lequel ledit récepteur-chimère comprend :
(a) les acides aminés 421 à 532 de la SEQ ID n° : 6 ou un dérivé fonctionnel cytolytique de celle-ci transduisant un signal ;
(b) les acides aminés : (a) 423 à 455 ; (b) 438 à 455 ; (c) 461 à 494 ; ou (d) 494 à 528 de la SEQ ID n° : 6 ;
(c) les acides aminés 400 à 420 de la SEQ ID n° : 6 ;
(d) les acides aminés 421 à 575 de la SEQ ID n° : 4 ou un dérivé fonctionnel cytolytique de celle-ci transduisant un signal ;
(e) les acides aminés : (a) 423 à 455 ; (b) 438 à 455 ; (c) 461 à 494 ; ou (d) 494 à 528 de la SEQ ID n° : 4 ;
(f) les acides aminés 400 à 420 de la SEQ ID n° : 4 ou un
(g) les acides aminés 421 à 462 de la SEQ ID n° : 5 ou un dérivé fonctionnel cytolytique de celle-ci transduisant un signal ;
(h) les acides aminés 402 à 419 de la SEQ ID n° : 5 ;
(i) les acides aminés Tyr282 à Tyr298 inclus, de la figure 15A ;
(j) les acides aminés 132 à 171 de la figure 16 (SEQ ID n° : 24) ;
(k) les acides aminés 140 à 182 de la figure 17 (SEQ ID n° : 25) ;
(l) les acides aminés 162 à 220 de la figure 18 (SEQ ID n° : 26) ; ou
(m) les acides aminés 183 à 228 de la figure 19 (SEQ ID n° : 27).

6. Procédé selon les revendications 1 à 3, dans lequel ladite protéine du récepteur de Fc est une FcγRIII humaine, une FcRIIγA humaine ou une FcRIIγC humaine.

7. Procédé selon les revendications 1 à 3, dans lequel lesdites cellules thérapeutiques sont choisies dans le groupe composé de :
(a) lymphocytes T ;
(b) lymphocytes T cytotoxiques ;
(c) cellules tueuses naturelles ;
(d) polynucléaires neutrophiles ;
(e) granulocytes ;
(f) macrophages ;
(g) mastocytes ;
(h) cellules HeLa.

8. Procédé selon les revendications 1 à 3, dans lequel ledit agent cible infectieux est un virus d'immunodéficience.

9. Procédé selon les revendications 1 à 3, dans lequel ladite partie extra-cellulaire comprend une partie d'un CD4 de liaison à l'enveloppe d'un VIH ou un dérivé fonctionnel de celle-ci de liaison à l'enveloppe d'un VIH.

10. Procédé selon les revendications 1 à 3, dans lequel ladite partie d'un CD4 de liaison à l'enveloppe d'un VIH comprend le peptide codé par les nucléotides 1 à 369 de la SEQ ID n° : 1.

11. Procédé selon les revendications 1 à 3, dans lequel lesdites cellules thérapeutiques détruisent par cytolyse ladite cellule cible, ou ledit agent infectieux cible, liée au récepteur.

12. Cellule qui exprime au moins deux récepteurs protéiques-chimères liés à une membrane, l'un desdits récepteurs comprenant (a) une partie extra-cellulaire ; et (b) une partie intra-cellulaire ou transmembranaire ; dans lequel ladite partie intra-cellulaire ou transmembranaire est la partie cellulaire transduisant le signal :
d'une protéine de récepteur de lymphocyte T ;
d'une protéine de récepteur de lymphocyte B ;
d'une protéine de récepteur de Fc ; ou d'un dérivé fonctionnel de celles-ci ; et le deuxième desdits récepteurs comprenant : (a) une partie extra-cellulaire, dans lequel ladite partie extra-cellulaire des premier et deuxième récepteurs comprend une partie d'un CD4 de liaison à l'enveloppe d'un VIH ou un dérivé fonctionnel de celle-ci de liaison à l'enveloppe d'un VIH ; et (b) une partie intra-cellulaire, qui est dérivée d'un CD28.

13. Cellule selon la revendication 12, dans laquelle ladite cellule cible est une cellule hôte infectée par un agent infectieux, une cellule tumorale ou cancéreuse ou une cellule générée de manière auto-immune.

14. Cellule selon l'une quelconque des revendications 12 ou 13, dans laquelle ladite liaison est indépendante du CMH.

15. Cellule selon l'une quelconque des revendications 12 à 14, dans laquelle ledit domaine intra-cellulaire ou transmembranaire est dérivé : d'une protéine ξ, η, CD3 delta ou T3 gamma d'un récepteur de lymphocyte T ; d'une protéine γ d'un récepteur de Fc ; ou d'une protéine mbl ou B29 d'un récepteur de lymphocyte B.

16. Cellule selon la revendication 15, dans laquelle ledit récepteur-chimère comprend :
(a) les acides aminés 421 à 532 de la SEQ ID n° : 6 ou un dérivé fonctionnel cytolytique de celle-ci transduisant un signal ;
(b) les acides aminés : (a) 423 à 455 ; (b) 438 à 455 ; (c) 461 à 494 ; ou (d) 494 à 528 de la SEQ ID n° : 6 ;
(c) les acides aminés 400 à 420 de la SEQ ID n° : 6 ;
(d) les acides aminés 421 à 575 de la SEQ ID n° : 4 ou un dérivé fonctionnel cytolytique de celle-ci transduisant un signal ;
(e) les acides aminés : (a) 423 à 455 ; (b) 438 à 455 ; (c) 461 à 494 ; ou (d) 494 à 528 de la SEQ ID n° : 4 ;
(f) les acides aminés 400 à 420 de la SEQ ID n° : 4 ;
(g) les acides aminés 421 à 462 de la SEQ ID n° : 5 ou un dérivé fonctionnel cytolytique de celle-ci transduisant un signal ;
(h) les acides aminés 402 à 419 de la SEQ ID n° : 5 ;
(i) les acides aminés Tyr282 à Tyr298 inclus, de la figure 15A ;
(j) les acides aminés 132 à 171 de la figure 16 (SEQ ID n° : 24) ;
(k) les acides aminés 140 à 182 de la figure 17 (SEQ ID n° : 25) ;
(l) les acides aminés 162 à 220 de la figure 18 (SEQ ID n° : 26) ;
(m) les acides aminés 183 à 228 de la figure 19 (SEQ ID n° : 27).

17. Cellule selon l'une quelconque des revendications 12 à 14, dans laquelle la protéine du récepteur de Fc est une FcγRIII humaine, une FcRIIIγA humaine ou une FcRIIγC humaine.

18. Cellule selon l'une quelconque des revendications 12 à 14, dans laquelle ladite partie extra-cellulaire comprend : la partie d'un récepteur de liaison à un ligand ; la partie d'un ligand de liaison à un récepteur ; la partie d'un anticorps de liaison à un antigène ; ou un dérivé fonctionnel de celles-ci.

19. Cellule selon la revendication 18, dans laquelle ledit agent infectieux cible est un virus d'immunodéficience ou ladite cellule cible est une cellule hôte infectée par un virus d'immunodéficience.

20. Cellule selon la revendication 19, dans laquelle ladite partie extra-cellulaire comprend une partie d'un CD4 de liaison à l'enveloppe d'un VIH ou un dérivé fonctionnel de celle-ci.

21. Cellule selon la revendication 20, dans laquelle ladite partie d'un CD4 de liaison à l'enveloppe d'un VIH comprend le peptide codé par les nucléotides 1 à 369 de la SEQ ID n° : 1.

22. Cellule selon la revendication 12, dans laquelle ladite cellule détruit par cytolyse ladite cellule cible, ou ledit agent infectieux, liée au récepteur.

23. Cellule qui exprime au moins deux récepteurs protéiques-chimères liés à une membrane, l'un desdits récepteurs comprenant : (a) une partie extra-cellulaire ; et (b) une partie intra-cellulaire ou transmembranaire dérivée d'un polypeptide CD3, zeta ou eta d'un récepteur de lymphocyte T ; d'un récepteur de lymphocyte B ; ou d'un récepteur de Fc ; et
le deuxième desdits récepteurs comprenant : (a) une partie extra-cellulaire, dans lequel ladite partie extra-cellulaire des premier et deuxième récepteurs comprend une partie d'un CD4 de liaison à l'enveloppe d'un VIH ou un dérivé fonctionnel de celle-ci de liaison à l'enveloppe d'un VIH ; et (b) une partie intra-cellulaire, qui est dérivée d'un CD28.

24. Cellule selon la revendication 12 ou 23, dans laquelle ledit récepteur-chimère comprend :
(a) une partie extra-cellulaire d'un CD16, CD7 ou CD5 ;
(b) une partie transmembranaire d'un CD5 ou CD7 ; ou
(c) une partie intra-cellulaire d'un CD5.

25. Paire de récepteurs protéiques-chimères liés à une membrane, l'un desdits récepteurs comprenant : (a) une partie extra-cellulaire ; et (b) une partie intra-cellulaire ou transmembranaire, dans lequel ladite partie intra-cellulaire ou transmembranaire est la partie transduisant le signal : d'une protéine d'un récepteur de lymphocyte T ; d'une protéine d'un récepteur de lymphocyte B ; d'une protéine d'un récepteur de Fc ; ou d'un dérivé fonctionnel de celles-ci ; et le deuxième desdits récepteurs comprenant : (a) une partie extra-cellulaire, dans lequel lesdites parties extra-cellulaires des premier et deuxième récepteurs comprennent une partie d'un CD4 de liaison à l'enveloppe d'un VIH ou un dérivé fonctionnel de celle-ci de liaison à l'enveloppe d'un VIH ; et (b) une partie intra-cellulaire, qui est dérivée d'un CD28.

26. Paire de récepteurs protéiques-chimères liés à une membrane, l'un desdits récepteurs comprenant : (a) une partie extra-cellulaire ; et (b) une partie intra-cellulaire ou transmembranaire dérivée d'un polypeptide CD3, zeta ou eta d'un récepteur de lymphocyte T ; d'un récepteur de lymphocyte B ; ou d'un récepteur de Fc ; et
le deuxième desdits récepteurs comprenant : (a) une partie extra-cellulaire, dans lequel lesdites parties extra-cellulaires des premier et deuxième récepteurs comprennent une partie d'un CD4 de liaison à l'enveloppe d'un VIH ou un dérivé fonctionnel de celle-ci de liaison à l'enveloppe d'un VIH ; et (b) une partie intra-cellulaire, qui est dérivée d'un CD28.

27. Paire de molécules d'ADN, dont chacune code pour un récepteur protéique-chimère lié à une membrane, selon l'une quelconque des revendications 25 ou 26.


### SEQUENCE LISTING

(1) GENERAL INFORMATION:
   (i) APPLICANTS: Seed, Brian et al.
   (ii) TITLE OF INVENTION: Redirection of Cellular Immunity by Receptor Chimeras
   (iii) NUMBER OF SEQUENCES: 40
   (iv) CORRESPONDENCE ADDRESS:
      (A) ADDRESSEE: Fish & Richardson P.C.
      (B) STREET: 225 Franklin Street
      (C) CITY: Boston
      (D) STATE: MA
      (E) COUNTRY: USA
      (F) ZIP: 02110-2804
   (v) COMPUTER READABLE FORM:
      (A) MEDIUM TYPE: 3.5" Diskette, 1.44 Mb
      (B) COMPUTER: IBM PS/2 Model 50Z or 55SX
      (C) OPERATING SYSTEM: IBM P.C. DOS (Version 3.30)
      (D) SOFTWARE: Wordperfect (Version 5.0)
   (vi) CURRENT APPLICATION DATA:
      (A) APPLICATION NUMBER: PCT/US96/-----
      (B) FILING DATE: Herewith
   (vii) PRIOR APPLICATION DATA:
      (A) APPLICATION NUMBER: 08/394,176
      (B) FILING DATE: 24 February 1995
   (viii) ATTORNEY/AGENT INFORMATION:
      (A) NAME: Karen F. Lech, Ph.D
      (B) REGISTRATION NUMBER: 35,238
      (C) REFERENCE/DOCKET NUMBER: 00786/270001
   (ix) TELECOMMUNICATION INFORMATION:
      (A) TELEPHONE: (617) 542-5070
      (B) TELEFAX: (617) 542-8906
      (C) TELEX: 200154
(2) INFORMATION FOR SEQ ID NO:1:
   (i) SEQUENCE CHARACTERISTICS:
      (A) LENGTH: 1728 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:1:
   ATGAACCGGG GAGTCCCTTT TAGGCACTTG CTTCTGGTGC TGCAACTGGC 50
   GCTCCTCCCA GCAGCCACTC AGGGAAACAA AGTGGTGCTG GGCAAAAAAG 100
   GGGATACAGT GGAACTGACC TGTACAGCTT CCCAGAAGAA GAGCATACAA 150
   TTCCACTGGA AAAACTCCAA CCAGATAAAG ATTCTGGGAA ATCAGGGCTC 200
   CTTCTTAACT AAAGGTCCAT CCAAGCTGAA TGATCGCGCT GACTCAAGAA 250
   GAAGCCTTTG GGACCAAGGA AACTTCCCCC TGATCATCAA GAATCTTAAG 300
   ATAGAAGACT CAGATACTTA CATCTGTGAA GTGGAGGACC AGAAGGAGGA 350
   GGTGCAATTG CTAGTGTTCG GATTGACTGC CAACTCTGAC ACCCACCTGC 400
   TTCAGGGGCA GAGCCTGACC CTGACCTTGG AGAGCCCCCC TGGTAGTAGC 450
   CCCTCAGTGC AATGTAGGAG TCCAAGGGGT AAAAACATAC AGGGGGGGAA 500
   GACCCTCTCC GTGTCTCAGC TGGAGCTCCA GGATAGTGGC ACCTGGACAT 550
   GCACTGTCTT GCAGAACCAG AAGAAGGTGG AGTTCAAAAT AGACATCGTG 600
   GTGCTAGCTT TCCAGAAGGC CTCCAGCATA GTCTATAAGA AAGAGGGGGA 650
   ACAGGTGGAG TTCTCCTTCC CACTCGCCTT TACAGTTGAA AAGCTGACGG 700
   GCAGTGGCGA GCTGTGGTGG CAGGCGGAGA GGGCTTCCTC CTCCAAGTCT 750
   TGGATCACCT TTGACCTGAA GAACAAGGAA GTGTCTGTAA AACGGGTTAC 800
   CCAGGACCCT AAGCTCCAGA TGGGCAAGAA GCTCCCGCTC CACCTCACCC 850
   TGCCCCAGGC CTTGCCTCAG TATGCTGGCT CTGGAAACCT CACCCTGGCC 900
   CTTGAAGCGA AAACAGGAAA GTTGCATCAG GAAGTGAACC TGGTGGTGAT 950
   GAGAGCCACT CAGCTCCAGA AAAATTTGAC CTGTGAGGTG TGGGGACCCA 1000
   CCTCCCCTAA GCTGATGCTG AGCTTGAAAC TGGAGAACAA GGAGGCAAAG 1050
   GTCTCGAAGC GGGAGAAGCC GGTGTGGGTG CTGAACCCTG AGGCGGGGAT 1100
   GTGGCAGTGT CTGCTGAGTG ACTCGGGACA GGTCCTGCTG GAATCCAACA 1150
   TCAAGGTTCT GCCCACATGG TCCACCCCGG TGCACGCGGA TCCCAAACTC 1200
   TGCTACTTGC TAGATGGAAT CCTCTTCATC TACGGAGTCA TCATCACAGC 1250
   CCTGTACCTG AGAGCAAAAT TCAGCAGGAG TGCAGAGACT GCTGCCAACC 1300
   TGCAGGACCC CAACCAGCTC TACAATGAGC TCAATCTAGG GCGAAGAGAG 1350
   GAATATGACG TCTTGGAGAA GAAGCGGGCT CGGGATCCAG AGATGGGAGG 1400
   CAAACAGCAG AGGAGGAGGA ACCCCCAGGA AGGCGTATAC AATGCACTGC 1450
   AGAAAGACAA GATGCCAGAA GCCTACAGTG AGATCGGCAC AAAAGGCGAG 1500
   AGGCGGAGAG GCAAGGGGCA CGATGGCCTT TACCAGGACA GCCACTTCCA 1550
   AGCAGTGCAG TTCGGGAACA GAAGAGAGAG AGAAGGTTCA GAACTCACAA      1600
   GGACCCTTGG GTTAAGAGCC CGCCCCAAAG GTGAAAGCAC CCAGCAGAGT      1650
   AGCCAATCCT GTGCCAGCGT CTTCAGCATC CCCACTCTGT GGAGTCCATG      1700
   GCCACCCAGT AGCAGCTCCC AGCTCTAA                              1728
(2) INFORMATION FOR SEQ ID NO:2:
   (i) SEQUENCE CHARACTERISTICS:
      (A) LENGTH: 1389 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:2:
   ATGAACCGGG GAGTCCCTTT TAGGCACTTG CTTCTGGTGC TGCAACTGGC     50
   GCTCCTCCCA GCAGCCACTC AGGGAAACAA AGTGGTGCTG GGCAAAAAAG    100
   GGGATACAGT GGAACTGACC TGTACAGCTT CCCAGAAGAA GAGCATACAA    150
   TTCCACTGGA AAAACTCCAA CCAGATAAAG ATTCTGGGAA ATCAGGGCTC    200
   CTTCTTAACT AAAGGTCCAT CCAAGCTGAA TGATCGCGCT GACTCAAGAA    250
   GAAGCCTTTG GGACCAAGGA AACTTCCCCC TGATCATCAA GAATCTTAAG    300
   ATAGAAGACT CAGATACTTA CATCTGTGAA GTGGAGGACC AGAAGGAGGA    350
   GGTGCAATTG CTAGTGTTCG GATTGACTGC CAACTCTGAC ACCCACCTGC    400
   TTCAGGGGCA GAGCCTGACC CTGACCTTGG AGAGCCCCCC TGGTAGTAGC    450
   CCCTCAGTGC AATGTAGGAG TCCAAGGGGT AAAAACATAC AGGGGGGGAA    500
   GACCCTCTCC GTGTCTCAGC TGGAGCTCCA GGATAGTGGC ACCTGGACAT    550
   GCACTGTCTT GCAGAACCAG AAGAAGGTGG AGTTCAAAAT AGACATCGTG    600
   GTGCTAGCTT TCCAGAAGGC CTCCAGCATA GTCTATAAGA AAGAGGGGGA    650
   ACAGGTGGAG TTCTCCTTCC CACTCGCCTT TACAGTTGAA AAGCTGACGG    700
   GCAGTGGCGA GCTGTGGTGG CAGGCGGAGA GGGCTTCCTC CTCCAAGTCT    750
   TGGATCACCT TTGACCTGAA GAACAAGGAA GTGTCTGTAA AACGGGTTAC    800
   CCAGGACCCT AAGCTCCAGA TGGGCAAGAA GCTCCCGCTC CACCTCACCC    850
   TGCCCCAGGC CTTGCCTCAG TATGCTGGCT CTGGAAACCT CACCCTGGCC    900
   CTTGAAGCGA AAACAGGAAA GTTGCATCAG GAAGTGAACC TGGTGGTGAT    950
   GAGAGCCACT CAGCTCCAGA AAAATTTGAC CTGTGAGGTG TGGGGACCCA   1000
   CCTCCCCTAA GCTGATGCTG AGCTTGAAAC TGGAGAACAA GGAGGCAAAG   1050
   GTCTCGAAGC GGGAGAAGCC GGTGTGGGTG CTGAACCCTG AGGCGGGGAT   1100
   GTGGCAGTGT CTGCTGAGTG ACTCGGGACA GGTCCTGCTG GAATCCAACA   1150
   TCAAGGTTCT GCCCACATGG TCCACCCCGG TGCACGCGGA TCCGCAGCTC   1200
   TGCTATATCC TGGATGCCAT CCTGTTTTTG TATGGTATTG TCCTTACCCT   1250
   GCTCTACTGT CGACTCAAGA TCCAGGTCCG AAAGGCAGAC ATAGCCAGCC   1300
   GTGAGAAATC AGATGCTGTC TACACGGGCC TGAACACCCG GAACCAGGAG   1350
   ACATATGAGA CTCTGAAACA TGAGAAACCA CCCCAATAG                1389
(2) INFORMATION FOR SEQ ID NO:3:
   (i) SEQUENCE CHARACTERISTICS:
      (A) LENGTH: 1599 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:3:
   ATGAACCGGG GAGTCCCTTT TAGGCACTTG CTTCTGGTGC TGCAACTGGC      50
   GCTCCTCCCA GCAGCCACTC AGGGAAACAA AGTGGTGCTG GGCAAAAAAG     100
   GGGATACAGT GGAACTGACC TGTACAGCTT CCCAGAAGAA GAGCATACAA     150
   TTCCACTGGA AAAACTCCAA CCAGATAAAG ATTCTGGGAA ATCAGGGCTC     200
   CTTCTTAACT AAAGGTCCAT CCAAGCTGAA TGATCGCGCT GACTCAAGAA     250
   GAAGCCTTTG GGACCAAGGA AACTTCCCCC TGATCATCAA GAATCTTAAG     300
   ATAGAAGACT CAGATACTTA CATCTGTGAA GTGGAGGACC AGAAGGAGGA     350
   GGTGCAATTG CTAGTGTTCG GATTGACTGC CAACTCTGAC ACCCACCTGC     400
   TTCAGGGGCA GAGCCTGACC CTGACCTTGG AGAGCCCCCC TGGTAGTAGC     450
   CCCTCAGTGC AATGTAGGAG TCCAAGGGGT AAAAACATAC AGGGGGGGAA     500
   GACCCTCTCC GTGTCTCAGC TGGAGCTCCA GGATAGTGGC ACCTGGACAT     550
   GCACTGTCTT GCAGAACCAG AAGAAGGTGG AGTTCAAAAT AGACATCGTG     600
   GTGCTAGCTT TCCAGAAGGC CTCCAGCATA GTCTATAAGA AAGAGGGGGA     650
   ACAGGTGGAG TTCTCCTTCC CACTCGCCTT TACAGTTGAA AAGCTGACGG     700
   GCAGTGGCGA GCTGTGGTGG CAGGCGGAGA GGGCTTCCTC CTCCAAGTCT     750
   TGGATCACCT TTGACCTGAA GAACAAGGAA GTGTCTGTAA AACGGGTTAC     800
   CCAGGACCCT AAGCTCCAGA TGGGCAAGAA GCTCCCGCTC CACCTCACCC     850
   TGCCCCAGGC CTTGCCTCAG TATGCTGGCT CTGGAAACCT CACCCTGGCC     900
   CTTGAAGCGA AAACAGGAAA GTTGCATCAG GAAGTGAACC TGGTGGTGAT      950
   GAGAGCCACT CAGCTCCAGA AAAATTTGAC CTGTGAGGTG TGGGGACCCA     1000
   CCTCCCCTAA GCTGATGCTG AGCTTGAAAC TGGAGAACAA GGAGGCAAAG     1050
   GTCTCGAAGC GGGAGAAGCC GGTGTGGGTG CTGAACCCTG AGGCGGGGAT     1100
   GTGGCAGTGT CTGCTGAGTG ACTCGGGACA GGTCCTGCTG GAATCCAACA     1150
   TCAAGGTTCT GCCCACATGG TCCACCCCGG TGCACGCGGA TCCCAAACTC     1200
   TGCTACCTGC TGGATGGAAT CCTCTTCATC TATGGTGTCA TTCTCACTGC     1250
   CTTGTTCCTG AGAGTGAAGT TCAGCAGGAG CGCAGAGCCC CCCGCGTACC     1300
   AGCAGGGCCA GAACCAGCTC TATAACGAGC TCAATCTAGG ACGAAGAGAG     1350
   GAGTACGATG TTTTGGACAA GAGACGTGGC CGGGACCCTG AGATGGGGGG     1400
   AAAGCCGAGA AGGAAGAACC CTCAGGAAGG CCTGTACAAT GAACTGCAGA     1450
   AAGATAAGAT GGCGGAGGCC TACAGTGAGA TTGGGATGAA AGGCGAGCGC     1500
   CGGAGGGGCA AGGGGCACGA TGGCCTTTAC CAGGGTCTCA GTACAGCCAC     1550
   CAAGGACACC TACGACGCCC TTCACATGCA GGCCCTGCCC CCTCGCTAA      1599
(2) INFORMATION FOR SEQ ID NO:4:
   (i) SEQUENCE CHARACTERISTICS:
      (A) LENGTH: 575 amino acids
      (B) TYPE: amino acid
      (D) TOPOLOGY: linear
   (ii) MOLECULE TYPE: protein
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 462 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Asn Arg Gly Val Pro Phe Arg His Leu Leu Leu Val Leu Gln Leu
   1               5                   10                  15
   Ala Leu Leu Pro Ala Ala Thr Gln Gly Asn Lys Val Val Leu Gly Lys
               20                  25                  30
   Lys Gly Asp Thr Val Glu Leu Thr Cys Thr Ala Ser Gln Lys Lys Ser
           35                  40                  45
   Ile Gln Phe His Trp Lys Asn Ser Asn Gln Ile Lys Ile Leu Gly Asn
       50                  55                  60
   Gln Gly Ser Phe Leu Thr Lys Gly Pro Ser Lys Leu Asn Asp Arg Ala
   65                  70                  75                  80
   Asp Ser Arg Arg Ser Leu Trp Asp Gln Gly Asn Phe Pro Leu Ile Ile
                   85                  90                  95
   Lys Asn Leu Lys Ile Glu Asp Ser Asp Thr Tyr Ile Cys Glu Val Glu
               100                 105                 110
   Asp Gln Lys Glu Glu Val Gln Leu Leu Val Phe Gly Leu Thr Ala Asn
           115                 120                 125
   Ser Asp Thr His Leu Leu Gln Gly Gln Ser Leu Thr Leu Thr Leu Glu
       130                 135                 140
   Ser Pro Pro Gly Ser Ser Pro Ser Val Gln Cys Arg Ser Pro Arg Gly
   145                 150                 155                 160
   Lys Asn Ile Gln Gly Gly Lys Thr Leu Ser Val Ser Gln Leu Glu Leu
                   165                 170                 175
   Gln Asp Ser Gly Thr Trp Thr Cys Thr Val Leu Gln Asn Gln Lys Lys
               180                 185                 190
   Val Glu Phe Lys Ile Asp Ile Val Val Leu Ala Phe Gln Lys Ala Ser
           195                 200                 205
   Ser Ile Val Tyr Lys Lys Glu Gly Glu Gln Val Glu Phe Ser Phe Pro
       210                 215                 220
   Leu Ala Phe Thr Val Glu Lys Leu Thr Gly Ser Gly Glu Leu Trp Trp
   225                 230                 235                 240
   Gln Ala Glu Arg Ala Ser Ser Ser Lys Ser Trp Ile Thr Phe Asp Leu
                   245                 250                 255
   Lys Asn Lys Glu Val Ser Val Lys Arg Val Thr Gln Asp Pro Lys Leu
               260                 265                 270
   Gln Met Gly Lys Lys Leu Pro Leu His Leu Thr Leu Pro Gln Ala Leu
           275                 280                 285
   Pro Gln Tyr Ala Gly Ser Gly Asn Leu Thr Leu Ala Leu Glu Ala Lys
       290                 295                 300
   Thr Gly Lys Leu His Gln Glu Val Asn Leu Val Val Met Arg Ala Thr
   305                 310                 315                 320
   Gln Leu Gln Lys Asn Leu Thr Cys Glu Val Trp Gly Pro Thr Ser Pro
                   325                 330                 335
   Lys Leu Met Leu Ser Leu Lys Leu Glu Asn Lys Glu Ala Lys Val Ser
               340                 345                 350
   Lys Arg Glu Lys Pro Val Trp Val Leu Asn Pro Glu Ala Gly Met Trp
           355                 360                 365
   Gln Cys Leu Leu Ser Asp Ser Gly Gln Val Leu Leu Glu Ser Asn Ile
       370                 375                 380
   Lys Val Leu Pro Thr Trp Ser Thr Pro Val His Ala Asp Pro Gln Leu
   385                 390                 395                 400
   Cys Tyr Ile Leu Asp Ala Ile Leu Phe Leu Tyr Gly Ile Val Leu Thr
                   405                 410                 415
   Leu Leu Tyr Cys Arg Leu Lys Ile Gln Val Arg Lys Ala Asp Ile Ala
               420                 425                 430
   Ser Arg Glu Lys Ser Asp Ala Val Tyr Thr Gly Leu Asn Thr Arg Asn
           435                 440                 445
   Gln Glu Thr Tyr Glu Thr Leu Lys His Glu Lys Pro Pro Gln
       450                 455                 460     462
(2) INFORMATION FOR SEQ ID NO:6:
   (i) SEQUENCE CHARACTERISTICS:
      (A) LENGTH: 532 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Asn Arg Gly Val Pro Phe Arg His Leu Leu Leu Val Leu Gln Leu
    1               5                  10                  15
   Ala Leu Leu Pro Ala Ala Thr Gln Gly Asn Lys Val Val Leu Gly Lys
               20                  25                  30
   Lys Gly Asp Thr Val Glu Leu Thr Cys Thr Ala Ser Gln Lys Lys Ser
           35                  40                  45
   Ile Gln Phe His Trp Lys Asn Ser Asn Gln Ile Lys Ile Leu Gly Asn
       50                  55                  60
   Gln Gly Ser Phe Leu Thr Lys Gly Pro Ser Lys Leu Asn Asp Arg Ala
   65                  70                  75                  80
   Asp Ser Arg Arg Ser Leu Trp Asp Gln Gly Asn Phe Pro Leu Ile Ile
                   85                  90                  95
   Lys Asn Leu Lys Ile Glu Asp Ser Asp Thr Tyr Ile Cys Glu Val Glu
               100                 105                 110
   Asp Gln Lys Glu Glu Val Gln Leu Leu Val Phe Gly Leu Thr Ala Asn
           115                 120                 125
   Ser Asp Thr His Leu Leu Gln Gly Gln Ser Leu Thr Leu Thr Leu Glu
       130                 135                 140
   Ser Pro Pro Gly Ser Ser Pro Ser Val Gln Cys Arg Ser Pro Arg Gly
   145                 150                 155                 160
   Lys Asn Ile Gln Gly Gly Lys Thr Leu Ser Val Ser Gln Leu Glu Leu
                   165                 170                 175
   Gln Asp Ser Gly Thr Trp Thr Cys Thr Val Leu Gln Asn Gln Lys Lys
               180                 185                 190
   Val Glu Phe Lys Ile Asp Ile Val Val Leu Ala Phe Gln Lys Ala Ser
           195                 200                 205
   Ser Ile Val Tyr Lys Lys Glu Gly Glu Gln Val Glu Phe Ser Phe Pro
       210                 215                 220
   Leu Ala Phe Thr Val Glu Lys Leu Thr Gly Ser Gly Glu Leu Trp Trp
   225                 230                 235                 240
   Gln Ala Glu Arg Ala Ser Ser Ser Lys Ser Trp Ile Thr Phe Asp Leu
                   245                 250                 255
   Lys Asn Lys Glu Val Ser Val Lys Arg Val Thr Gln Asp Pro Lys Leu
               260                 265                 270
   Gln Met Gly Lys Lys Leu Pro Leu His Leu Thr Leu Pro Gln Ala Leu
           275                 280                 285
   Pro Gln Tyr Ala Gly Ser Gly Asn Leu Thr Leu Ala Leu Glu Ala Lys
       290                 295                 300
   Thr Gly Lys Leu His Gln Glu Val Asn Leu Val Val Met Arg Ala Thr
   305                 310                 315                 320
   Gln Leu Gln Lys Asn Leu Thr Cys Glu Val Trp Gly Pro Thr Ser Pro
                   325                 330                 335
   Lys Leu Met Leu Ser Leu Lys Leu Glu Asn Lys Glu Ala Lys Val Ser
               340                 345                 350
   Lys Arg Glu Lys Pro Val Trp Val Leu Asn Pro Glu Ala Gly Met Trp
           355                 360                 365
   Gln Cys Leu Leu Ser Asp Ser Gly Gln Val Leu Leu Glu Ser Asn Ile
       370                 375                 380
   Lys Val Leu Pro Thr Trp Ser Thr Pro Val His Ala Asp Pro Lys Leu
   385                 390                 395                 400
   Cys Tyr Leu Leu Asp Gly Ile Leu Phe Ile Tyr Gly Val Ile Leu Thr
                   405                 410                 415
   Ala Leu Phe Leu Arg Val Lys Phe Ser Arg Ser Ala Glu Pro Pro Ala
               420                 425                 430
   Tyr Gln Gln Gly Gln Asn Gln Leu Tyr Asn Glu Leu Asn Leu Gly Arg
           435                 440                 445
   Arg Glu Glu Tyr Asp Val Leu Asp Lys Arg Arg Gly Arg Asp Pro Glu
       450                 455                 460
   Met Gly Gly Lys Pro Arg Arg Lys Asn Pro Gln Glu Gly Leu Tyr Asn
   465                 470                 475                 480
   Glu Leu Gln Lys Asp Lys Met Ala Glu Ala Tyr Ser Glu Ile Gly Met
                   485                 490                 495
   Lys Gly Glu Arg Arg Arg Gly Lys Gly His Asp Gly Leu Tyr Gln Gly
               500                 505                 510
   Leu Ser Thr Ala Thr Lys Asp Thr Tyr Asp Ala Leu His Met Gln Ala
           515                 520                 525
   Leu Pro Pro Arg
       530
(2) INFORMATION FOR SEQ ID NO:7:
   (i) SEQUENCE CHARACTERISTICS:
      (A) LENGTH: 33 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:7:
   CGCGGGGTGA CCGTGCCCTC CAGCAGCTTG GGC                    33
(2) INFORMATION FOR SEQ ID NO:8:
   (i) SEQUENCE CHARACTERISTICS:
      (A) LENGTH: 50 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:8:
   CGCGGGGATC CGTCGTCCAG AGCCCGTCCA GCTCCCCGTC CTGGGCCTCA      50
(2) INFORMATION FOR SEQ ID NO:9:
   (i) SEQUENCE CHARACTERISTICS:
      (A) LENGTH: 33 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:9:
   CGCGGGCGGC CGCGACGCCG GCCAAGACAG CAC 33
(2) INFORMATION FOR SEQ ID NO:10:
   (i) SEQUENCE CHARACTERISTICS:
      (A) LENGTH: 33 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:10:
   CGCGTTGACG AGCAGCCAGT TGGGCAGCAG CAG 33
(2) INFORMATION FOR SEQ ID NO:11:
   (i) SEQUENCE CHARACTERISTICS:
      (A) LENGTH: 15 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:11:
   CGCGGGCGGC CGCTA 15
(2) INFORMATION FOR SEQ ID NO:12:
   (i) SEQUENCE CHARACTERISTICS:
      (A) LENGTH: 42 bases
      (3) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:12:
   CGCGGGCTCG TTATAGAGCT GGTTCTGGCG CTGCTTCTTC TG 42
(2) INFORMATION FOR SEQ ID NO:13:
   (i) SEQUENCE CHARACTERISTICS:
      (A) LENGTH: 48 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:13:
   CGCGGGGAGC TCGTTATAGA GCTGGTTTGC CGCCGAATTC TTATCCCG 48
(2) INFORMATION FOR SEQ ID NO:14:
   (i) SEQUENCE CHARACTERISTICS:
      (A) LENGTH: 33 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:14:
   CGCGGGGCGG CCACGCGTCC TCGCCAGCAC ACA 33
(2) INFORMATION FOR SEQ ID NO:15:
   (i) SEQUENCE CHARACTERISTICS:
      (A) LENGTH: 36 bases
      (B) TYPE: nucleic acid
      (C) STRANDEDNESS: single
      (D) TOPOLOGY: linear
   (ii) MOLECULE TYPE: nucleic acid
      (xi) SEQUENCE DESCRIPTION: SEQ ID NO:15:
   CGCGGGACGC GTTTCAGCCG TCCTCGCCAG CACACA 36
(2) INFORMATION FOR SEQ ID NO:16:
   (i) SEQUENCE CHARACTERISTICS:
      (A) LENGTH: 33 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:16:
   CGCGGGACGC GTGACCCTGA GATGGGGGGA AAG 33
(2) INFORMATION FOR SEQ ID NO:17:
   (i) SEQUENCE CHARACTERISTICS:
      (A) LENGTH: 33 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:17:
   CGCGGGACGC GTATTGGGAT GAAAGGCGAG CGC 33
(2) INFORMATION FOR SEQ ID NO:18:
   (i) SEQUENCE CHARACTERISTICS:
      (A) LENGTH: 26 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:18:
   CCCGGATCCC AGCATGGGCA GCTCTT 26
(2) INFORMATION FOR SEQ ID NO:19:
   (i) SEQUENCE CHARACTERISTICS:
      (A) LENGTH: 42 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:19:
   CGCGGGGCGG CCGCTTTAGT TATTACTGTT GACATGGTCG TT 42
(2) INFORMATION FOR SEQ ID NO:20:
   (i) SEQUENCE CHARACTERISTICS:
      (A) LENGTH: 40 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:20:
   GCGGGGGGAT CCCACTGTCC AAGCTCCCAG CTCTTCACCG 40
(2) INFORMATION FOR SEQ ID NO:21:
   (i) SEQUENCE CHARACTERISTICS:
      (A) LENGTH: 32 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:21:
   GCGGGGGCGG CCGCCTAAAT ACGGTTCTGG TC 32
(2) INFORMATION FOR SEQ ID NO:22:
   (i) SEQUENCE CHARACTERISTICS:
      (A) LENGTH: 31 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:22:
   TCAGAAAGAG ACAACCTGAA GAAACCAACA A 31
(2) INFORMATION FOR SEQ ID NO:23:
   (i) SEQUENCE CHARACTERISTICS:
      (A) LENGTH: 31 bases
      (B) TYPE: nucleic acid
      (C) STRANDEDNESS: single
      (D) TOPOLOGY: linear
   (ii) MOLECULE TYPE: nucleic acid
   (xi) SEQUENCE DESCRIPTION: SEQ ID NO:23:
   TTGTTGGTTT CTTCAGGTTG TGTCTTTCTG A                                    31
(2) INFORMATION FOR SEQ ID NO:24:
   (i) SEQUENCE CHARACTERISTICS:
      (A) LENGTH: 171 amino acids
      (B) TYPE: amino acid
      (D) TOPOLOGY: linear
   (ii) MOLECULE TYPE: amino acid
   (xi) SEQUENCE DESCRIPTION: SEQ ID NO:24:
   Met Glu His Ser Thr Phe Leu Ser Gly Leu Val Leu Ala Thr Leu Leu
                   5                   10                  15
   Ser Gln Val Ser Pro Phe Lys Ile Pro Ile Glu Glu Leu Glu Asp Arg
               20                  25                  30
   Val Phe Val Asn Cys Asn Thr Ser Ile Thr Trp Val Glu Gly Thr Val
           35                  40                  45
   Gly Thr Leu Leu Ser Asp Ile Thr Arg Leu Asp Leu Gly Lys Arg Ile
       50                  55                  60
   Leu Asp Pro Arg Gly Ile Tyr Arg Cys Asn Gly Thr Asp Ile Tyr Lys
   65                  70                  75                  80
   Asp Lys Glu Ser Thr Val Gln Val His Tyr Arg Met Cys Gln Ser Cys
                   85                  90                  95
   Val Glu Leu Asp Pro Ala Thr Val Ala Gly Ile Ile Val Thr Asp Val
               100                 105                 110
   Ala Ile Thr Leu Leu Leu Ala Leu Gly Val Phe Cys Phe Ala Gly His
           115                 120                 125
   Glu Thr Gly Arg Leu Ser Gly Ala Ala Asp Thr Gln Ala Leu Leu Arg
       130                 135                 140
   Asn Asp Gln Val Tyr Gln Pro Leu Arg Asp Arg Asp Asp Ala Gln Tyr
   145                 150                 155                 160
   Ser His Leu Gly Gly Asn Trp Ala Arg Asn Lys
                   165                 170
(2) INFORMATION FOR SEQ ID NO:25:
   (i) SEQUENCE CHARACTERISTICS:
      (A) LENGTH: 182 amino acids
      (B) TYPE: amino acid
      (D) TOPOLOGY: linear
   (ii) MOLECULE TYPE: amino acid
   (xi) SEQUENCE DESCRIPTION: SEQ ID NO:25:
   Met Glu Gln Gly Lys Gly Leu Ala Val Leu Ile Leu Ala Ile Ile Leu
                   5                   10                  15
   Leu Gln Gly Thr Leu Ala Gln Ser Ile Lys Gly Asn His Leu Val Lys
               20                  25                  30
   Val Tyr Asp Tyr Gln Glu Asp Gly Ser Val Leu Leu Thr Cys Asp Ala
           35                  40                  45
   Glu Ala Lys Asn Ile Thr Trp Phe Lys Asp Gly Lys Met Ile Gly Phe
       50                  55                  60
   Leu Thr Glu Asp Lys Lys Lys Trp Asn Leu Gly Ser Asn Ala Lys Asp
   65                  70                  75                  80
   Pro Arg Gly Met Tyr Gln Cys Lys Gly Ser Gln Asn Lys Ser Lys Pro
                   85                  90                  95
   Leu Gln Val Tyr Tyr Arg Met Cys Gln Asn Cys Ile Glu Leu Asn Ala
               100                 105                 110
   Ala Thr Ile Ser Gly Phe Leu Phe Ala Glu Ile Val Ser Ile Phe Val
           115                 120                 125
   Leu Ala Val Gly Val Tyr Phe Ile Ala Gly Gln Asp Gly Val Arg Gln
       130                 135                 140
   Ser Arg Ala Ser Asp Lys Gln Thr Leu Leu Pro Asn Asp Gln Leu Tyr
   145                 150                 155                 160
   Gln Pro Leu Lys Asp Arg Glu Asp Asp Gln Tyr Ser His Leu Gln Gly
                   165                 170                 175
   Asn Gln Leu Arg Arg Asn
               180
(2) INFORMATION FOR SEQ ID NO:26:
   (i) SEQUENCE CHARACTERISTICS:
      (A) LENGTH: 220 amino acids
      (B) TYPE: amino acid
      (D) TOPOLOGY: linear
   (ii) MOLECULE TYPE: amino acids
   (xi) SEQUENCE DESCRIPTION: SEQ ID NO:26:
   Met Pro Gly Gly Leu Glu Ala Leu Arg Ala Leu Pro Leu Leu Leu Phe
                   5                   10                  15
   Leu Ser Tyr Ala Cys Leu Gly Pro Gly Cys Gln Ala Leu Arg Val Glu
               20                  25                  30
   Gly Gly Pro Pro Ser Leu Thr Val Asn Leu Gly Glu Glu Ala Arg Leu
           35                  40                  45
   Thr Cys Glu Asn Asn Gly Arg Asn Pro Asn Ile Thr Trp Trp Phe Ser
       50                  55                  60
   Leu Gln Ser Asn Ile Thr Trp Pro Pro Val Pro Leu Gly Pro Gly Gln
   65                  70                  75                  80
   Gly Thr Thr Gly Gln Leu Phe Phe Pro Glu Val Asn Lys Asn Thr Gly
                   85                  90                  95
   Ala Cys Thr Gly Cys Gln Val Ile Glu Asn Asn Ile Leu Lys Arg Ser
               100                 105                 110
   Cys Gly Thr Tyr Leu Arg Val Arg Asn Pro Val Pro Arg Pro Phe Leu
           115                 120                 125
   Asp Met Gly Glu Gly Thr Lys Asn Arg Ile Ile Thr Ala Glu Gly Ile
       130                 135                 140
   Ile Leu Leu Phe Cys Ala Val Val Pro Gly Thr Leu Leu Leu Phe Arg
   145                 150                 155                 160
   Lys Arg Trp Gln Asn Glu Lys Phe Gly Val Asp Met Pro Asp Asp Tyr
                   165                 170                 175
   Glu Asp Glu Asn Leu Tyr Glu Gly Leu Asn Leu Asp Asp Cys Ser Met
               180                 185                 190
   Tyr Glu Asp Ile Ser Arg Gly Leu Gln Gly Thr Tyr Gln Asp Val Gly
           195                 200                 205
   Asn Leu His Ile Gly Asp Ala Gln Leu Glu Lys Pro
       210                 215                 220
(2) INFORMATION FOR SEQ ID NO:27:
   (i) SEQUENCE CHARACTERISTICS:
      (A) LENGTH: 228 amino acids
      (B) TYPE: amino acid
      (D) TOPOLOGY: linear
   (ii) MOLECULE TYPE: amino acid
   (xi) SEQUENCE DESCRIPTION: SEQ ID NO:27:
   Met Ala Thr Leu Val Leu Ser Ser Met Pro Cys His Trp Leu Leu Phe
                   5                   10                  15
   Leu Leu Leu Leu Phe Ser Gly Glu Pro Val Pro Ala Met Thr Ser Ser
               20                  25                  30
   Asp Leu Pro Leu Asn Phe Gln Gly Ser Pro Cys Ser Gln Ile Trp Gln
           35                  40                  45
   His Pro Arg Phe Ala Ala Lys Lys Arg Ser Ser Met Val Lys Phe His
       50                  55                  60
   Cys Tyr Thr Asn His Ser Gly Ala Leu Thr Trp Phe Arg Lys Arg Gly
   65                  70                  75                  80
   Ser Gln Gln Pro Gln Glu Leu Val Ser Glu Glu Gly Arg Ile Val Gln
                   85                  90                  95
   Thr Gln Asn Gly Ser Val Tyr Thr Leu Thr Ile Gln Asn Ile Gln Tyr
               100                 105                 110
   Glu Asp Asn Gly Ile Tyr Phe Cys Lys Gln Lys Cys Asp Ser Ala Asn
           115                 120                 125
   His Asn Val Thr Asp Ser Cys Gly Thr Glu Leu Leu Val Leu Gly Phe
       130                 135                 140
   Ser Thr Leu Asp Gln Leu Lys Arg Arg Asn Thr Leu Lys Asp Gly Ile
   145                 150                 155                 160
   Ile Leu Ile Gln Thr Leu Leu Ile Ile Leu Phe Ile Ile Val Pro Ile
                   165                 170                 175
   Phe Leu Leu Leu Asp Lys Asp Asp Gly Lys Ala Gly Met Glu Glu Asp
               180                 185                 190
   His Thr Tyr Glu Gly Leu Asn Ile Asp Gln Thr Ala Thr Tyr Glu Asp
           195                 200                 205
   Ile Val Thr Leu Arg Thr Gly Glu Val Lys Trp Ser Val Gly Glu His
       210                 215                     220
   Pro Gly Gln Glu
   225
(2) INFORMATION FOR SEQ ID NO:28:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: protein
   SEQUENCE DESCRIPTION/ SEQ ID NO:28
(2) INFORMATION FOR SEQ ID NO:29:
   (i) SEQUENCE CHARACTERISTICS:
      (A) LENGTH: 19 amino acids
      (3) TYPE: amino acid
      (C) STRANDEDNESS: not relevant
      (D) TOPOLOGY: Linear
   (ii) MOLECULE TYPE: protein
   (i) SEQUENCE DESCRIPTION: SEQ ID NO:30:
(2) INFORMATION FOR SEQ ID NO:30:
   (i) SEQUENCE CHARACTERISTICS:
      (A) LENGTH: 12 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION : SEQ ID NO:30:
(2) INFORMATION FOR SEQ ID NO:31:
   (i) SEQUENCE CHARACTERISTICS:
      (A) LENGTH: 19 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:31:
   Pro Thr Trp Ser Thr Pro Val His Ala Asp Pro Gln Leu Cys Tyr Ile
   1               5                   10                  15
   Leu Asp Ala
(2) INFORMATION FOR SEQ ID NO:32:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:32:
   Gln Ser Phe Gly Leu Leu Asp Pro Lys Leu Cys Tyr Leu Leu Asp Gly
   1               5                   10                  15
(2) INFORMATION FOR SEQ ID NO:33:
   (i) SEQUENCE CHARACTERISTICS:
      (A) LENGTH: 16 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:33:
   Phe Ser Pro Pro Gly Ala Asp Pro Lys Leu Cys Tyr Leu Leu Asp Gly
   1               5                   10                  15
(2) INFORMATION FOR SEQ ID NO:34:
   (i) SEQUENCE CHARACTERISTICS:
      (A) LENGTH: 142 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:34:
   Gln Ser Phe Gly Leu Leu Asp Pro Lys Leu Cys Tyr Leu Leu Asp Gly
   1               5                   10                  15
   Ile Leu Phe Ile Tyr Gly Val Ile Leu Thr Ala Leu Phe Leu Arg Val
               20                  25                  30
   Lys Phe Ser Arg Ser Ala Glu Pro Pro Ala Tyr Gln Gln Gly Gln Asn
           35                  40                  45
   Gln Leu Tyr Asn Glu Leu Asn Leu Gly Arg Arg Glu Glu Tyr Asp Val
       50                  55                  60
   Leu Asp Lys Arg Arg Gly Arg Asp Pro Glu Met Gly Gly Lys Pro Arg
   65                  70                  75                  80
   Arg Lys Asn Pro Gln Glu Gly Leu Tyr Asn Glu Leu Gln Lys Asp Lys
                   85                  90                  95
   Met Ala Glu Ala Tyr Ser Glu Ile Gly Met Lys Gly Glu Arg Arg Arg
               100                 105                 110
   Gly Lys Gly His Asp Gly Leu Tyr Gln Gly Leu Ser Thr Ala Thr Lys
           115                 120                 125
   Asp Thr Tyr Asp Ala Leu His Met Gln Ala Leu Pro Pro Arg
       130                 135                 140
(2) INFORMATION FOR SEQ ID NO:35:
   (i) SEQUENCE CHARACTERISTICS:
      (A) LENGTH: 35 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:35:
   Arg Val Lys Phe Ser Arg Ser Ala Glu Pro Pro Ala Tyr Gln Gln Gly
   1               5                   10                  15
   Gln Asn Gln Leu Tyr Asn Glu Leu Asn Leu Gly Arg Arg Glu Glu Tyr
               20                  25                  30
   Asp Val Leu
           35
(2) INFORMATION FOR SEQ ID NO:36:
   (i) SEQUENCE CHARACTERISTICS:
      (A) LENGTH: 32 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:36:
   Lys Lys Leu Val Lys Lys Phe Arg Gln Lys Lys Gln Arg Gln Asn Gln
   1               5                   10                  15
   Leu Tyr Asn Glu Leu Asn Leu Gly Arg Arg Glu Glu Tyr Asp Val Leu
               20                  25                  30
(2) INFORMATION FOR SEQ :D NO:37:
   (i) SEQUENCE CHARACTERISTICS:
      (A) LENGTH: 35 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:37:
(2) INFORMATION FOR SEQ :D NO:38:
   (i) SEQUENCE CHARACTERISTICS:
      (A) LENGTH: 35 amino acids
      (B) TYPE: amino acid
      (C) STRANDEDNESS: not relevant
      (D) TOPOLOGY: linear
   (ii) MOLECULE TYPE: protein
   (xi) SEQUENCE DESCRIPTION: SEQ ID NO:38: